# EUROPEAN PATENT SPECIFICATION

(11) **EP 2 254 880 B1**
(45) Date of publication and mention of the grant of the patent: **17.08.2016**
(21) Application number: 09713314.4
(22) Date of filing: 23.02.2009
(51) Int. Cl.: C07D 313/00, C07D 315/00, A61K 31/365

(54) **Macrocyclic prodrug compounds useful as therapeutics**
Als Therapeutika geeignete makrocyclische Prodrugverbindungen
Composés de promédicaments macrocycliques utilisés comme agents thérapeutiques

(30) Priority: 21.02.2008 US 30446 P
(43) Date of publication of application: 01.12.2010
(73) Proprietor: Nexgenix Pharmaceuticals, New York, NY 10019 (US); Université de Strasbourg, 67070 Strasbourg (FR)
(72) Inventor: HECK, James, New York, NY 10019 (US); WINSSINGER, Nicolas, F-67000 Strasbourg (FR); CHABALA, John C., New York, NY 10019 (US); BARLUENGA, Sofia, F-67000 Strasbourg (FR); CHEN, Ruihong, New York, NY 10019 (US); RUBENSTEIN, Allan, New York, NY 10019 (US); YU, Jin-Chen, New York, NY 10019 (US)
(74) Representative: Vossius & Partner Patentanwälte Rechtsanwälte mbB
(86) International application number: PCT/US2009/034878
(87) International publication number: WO 2009/105755

(56) References cited:
- EP-A1- 0 460 950
- EP-A1- 0 823 429
- WO-A1-2004/024142
- JP-A- 9 202 781
- JP-A- 10 265 381
- JP-A- 2003 113 183
- US-A1- 2005 182 129
- FRANZ BRACHER ET AL.: 'The total synthesis of both enantiomers of the macrocyclic lactone zearalane.' EUR.J.ORG.CHEM. 2001, pages 4701 - 4704, XP008141388

## Description

### FIELD OF THE INVENTION

The present invention relates to prodrugs of novel derivatives, analogs and intermediates of the natural products radicicol and pochonins, and to their syntheses. The present invention further relates to use of these compounds in the treatment of various diseases including an autoimmune disease, an inflammatory disease, a neurological or neurodegenerative disease, cancer, a cardiovascular disease, allergy, asthma, a hormone-related disease, and tumors or symptoms resulting from neurofibromatosis.

### BACKGROUND OF THE INVENTION

In the mid-1950's, it was discovered that phosphorylation can reversibly alter the function of enzymes by means of protein kinases which catalyze phosphorylation, or by protein phosphatases which are involved in the dephosphorylation step. These reactions play an essential role in regulating many cellular processes, especially signaling transduction pathways. In the late 1970's, the Rous sarcoma virus (v-Src)'s transforming factor was discovered to be a protein kinase, and also tumor-promoting phorbol esters were found to be potent activators of protein kinase C, revealing the first known connection between disease and abnormal protein phosphorylation. Since then transduction mechanistic defects have been found to cause numerous oncogenic processes and to have a role in diabetes, inflammatory disorders, and cardiovascular diseases. (T. Hunter, Cell, 100:113-127 (2000); P. Cohen, Nat. Rev. Drug Discov., 1:309 (2002)). Thus selective kinase and phosphatase inhibitors have emerged as important drug targets, and inhibition of kinase phosphorylation activity is one of the most promising strategies for chemotherapy. Three kinase inhibitor drugs are already approved: Gleevec, which inhibits Abl, and Iressa and Tarceva, which both inhibit EGFR.

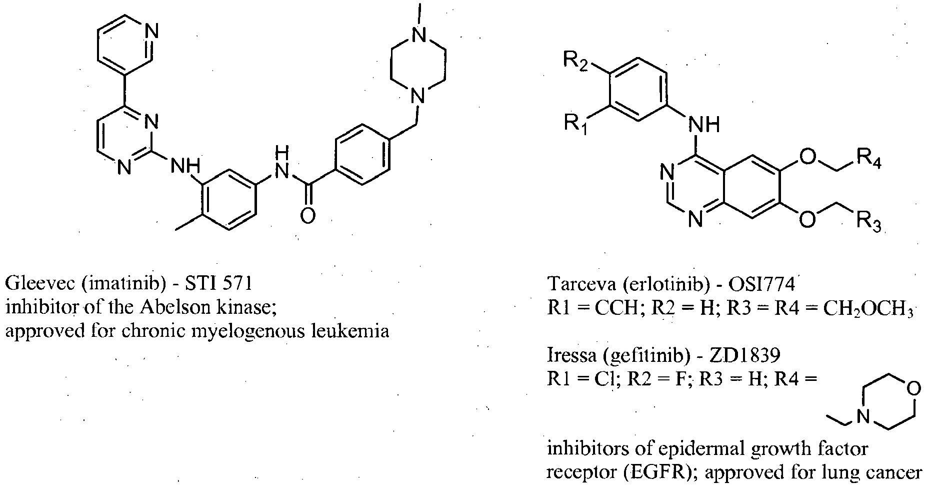

Modulation of protein activity by kinase-mediated phosphorylation or phosphatase-mediated dephosphorylation of a serine, threonine or tyrosine residue is at the center of most signal transduction mechanisms. (T. Hunter, Cell, 100:113 (2000)). Small molecule inhibitors such as 6-dimethylaminopurine and staurosporine were instrumental in elucidating the importance of such phosphorylation mechanisms and shed light on the biological function of kinases. Kinases bind to ATP with a Kₘ of 0.1-10 µM, and transfer the γ-phosphate group selectively to a specific residue of a given protein. The core domain of kinases, consisting of the ATP-binding site with the residues involved in phosphotransfer reaction, are highly conserved throughout the kinome. (G. Manning et al., Science, 298:1912 (2002)). This led to the speculation that inhibitors targeting this highly conserved ATP-binding pocket would not only have to compete with ATP present at high concentration (mM) but would necessarily lack selectivity. The discovery that modified purines such as (R)-roscovitine were potent and fairly selective inhibitors (L. Meijer and E. Raymond, Acc. Chem. Res., 36:417 (2003)) refuted that notion and inspired the synthesis of combinatorial libraries around the purine scaffold (Y. T. Chang et al., Chem. Biol., 6:361 (1999); S. Ding et al., J. Am. Chem. Soc., 124:1594 (2002)), yielding important leads. (N. S. Gray et al., Science, 281:533 (1998); M. Knockaert et al., Chem. Biol., 7:411 (2000)).

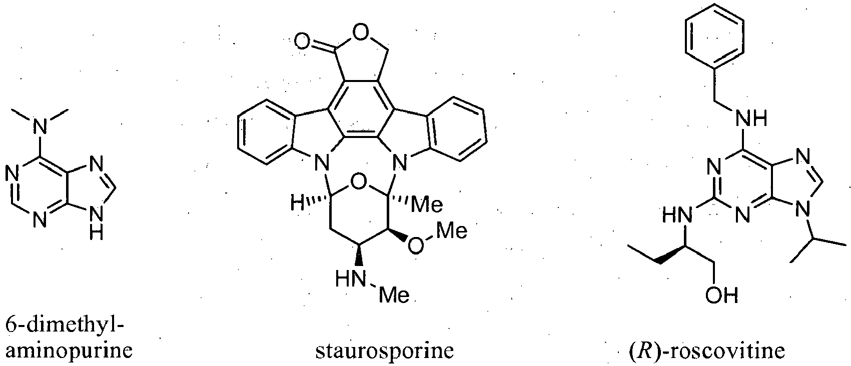

Macrocyclic resorcylic acid lactones have also been investigated in this respect. The archetypes of this class of compounds are radicicol and the related pochonins, which are a structurally related group of secondary metabolites isolated from cultures of the clavicipitaceous hyphomycete *Pochonia* genus, such as *Pochonia chlamydosporia* var. catenulate strain P0297. See, e.g., V. Hellwig et al., J. Natural Prod., 66(6):829-837 (2003). Halohydrin and oxime derivatives of radicicol were prepared and evaluated for their v-src tyrosine kinase inhibitory, antiproliferative, and antitumor *in vitro* activity (T. Agatsuma et al., Bioorg. & Med. Chem., 10(11):3445-3454 (2002).

Like kinases, heat shock proteins (HSPs) interact with ATP and are important targets for controlling disease, however they have a different mechanistic effect. Immediately after exposure to a stress such as heat, hypoxia or acidosis, cells in most tissues rapidly escalate production rate of the HSPs. It is now believed that heat HSPs are molecular chaperones, i.e., they prevent improper associations and assist in the correct folding of other cellular proteins collectively termed clients and substrates. HSP's are also found in association with tumors and other pathophysiological conditions. In fact, chaperone proteins facilitate the survival of tumor cells in stressful environments by facilitating tolerance of alterations inside the cell. HSPs are ubiquitous, highly conserved among the species, and usually classified by molecular weight to the following major families: HSP100, HSP90, HSP70, HSP60 and small HSPs. These families have structural and functional differences, but work cooperatively at different stages of protein folding. HSP90 has attracted particular attention due to its association with many types of signaling molecules such as v-Src and Raf that play a critical role in malignant transformation and metastasis development. Thus, HSP90 inhibitors are desired for designing chemotherapies, and also for elucidating the interplay in complex signaling networks.

Heat Shock Protein 90's (Hsp90s) are ubiquitous chaperone proteins that maintain the proper conformation of many "client" proteins (see Kamal et. al. Trends Mol. Med. 2004, 10, 283-290; Dymock et. al. Expert Opin. Ther. Patents 2004, 14, 837-847; Isaacs et. al. Cancer Cell, 2003, 3, 213; Maloney et. al. Expert Opin. Biol. Ther. 2002, 2, 3-24 and Richter et. al. J. Cell. Physiol. 2001, 188, 281-290), and are involved in folding, activation and assembly of a wide range of proteins, including key proteins involved in signal transduction, cell cycle control and transcriptional regulation. Researchers have reported that HSP90 chaperone proteins are associated with important signaling proteins, such as steroid hormone receptors and protein kinases, including, e.g., Raf-1, EGFR, v-Src family kinases, Cdk4, and ErbB-2 (Buchner, TIBS, 1999, 24, 136-141; Stepanova et. al., Genes Dev. 1996, 10, 1491-502; Dai et. al., J. Biol. Chem. 1996, 271, 22030-4). Studies further indicate that certain co-chaperones, e.g., Hsp70, p60/Hop/Sti1, Hip, Bag1, HSP40/Hdj2/Hsj1, immunophilins, p23, and p50, may assist HSP90 in its function (see for example Caplan, Trends in Cell Biol, 1999, 9, 262-268). Inhibition of Hsp90 causes these client proteins to adopt aberrant conformations, and these abnormally folded proteins are rapidly eliminated by the cell via ubiquitinylation and proteasome degradation. Interestingly, the list of Hsp90 client proteins includes a series of notorious oncogenes. Four of them are clinically validated cancer targets: HER-2/neu (Herceptin® (trastuzumab)), Bcr-Abl (Gleevec® (imatinib mesylate)), the estrogen receptor (tamoxifen), and the androgen receptor (Casodex® (bicalutamide)), while the others play a critical role in the development of cancer. Some of the most sensitive Hsp90 clients are involved in growth signalling (Raf-1, Akt, cdk4, Src, Bcr-Abl, etc). In contrast, few tumor suppressor genes, if any, seem to be clients of Hsp90 (for lists of client proteins see Pratt et. al. Exp. Biol. Med. 2003, 228, 111-133; Workman et. al. Cancer Lett. 2004, 206, 149-157 and Zhang et. al. J. Mol. Med. 2004, 82, 488-499.), and consequently, inhibition of Hsp90 has an overall anti-proliferative effect. In addition, some client proteins are involved in other fundamental processes of tumorigenesis, namely apoptosis evasion (e.g. Apaf-1, RIP, Akt), immortality (e.g. hTert), angiogenesis (e.g. VEGFR, Flt-3, FAK, HIF-1), and metastasis (c-Met).

However medicinal HSP inhibitors must be selective because HSPs also play a constructive role. Under non-stressed conditions, HSP90 is one of the most abundant proteins present in the eukaryotic cells, representing between 1-2% of the total cellular protein content and increasing only about two-fold when cells are stressed. Upon binding with the native client HSP90 is an essential housekeeper, e.g., for folding of nascent polypeptides, transporting proteins across membranes, and for normal protein turnover. Moreover, HSP90 plays a crucial role in post-translational regulation of signaling molecules, leading to their activation. HSP90 rarely functions alone but instead works with chaperone HSP70, with co-chaperones (HSP40, CDC37/p50, AHA1, p23), and with accessory proteins.

The numerous client proteins of HSP90 play a crucial role in growth control, cell survival and development processes, and those clients are known to include receptor tyrosine kinases, serine/threonine kinases, steroid hormone receptors, transcription factors and telomerase. Oncogenic mutants of clients are also clients themselves but have higher requirements for HSP90 function, for instance the mutant v-SRC tyrosine kinase requires more protein-folding capability from HSP90's cooperative assembly of proteins (Y. Xu et al., Proc. Natl. Acad. Sci. U.S.A., 96:109 (1999); .H. Oppermann et al., Ibid., 78:1067 (1981); L. Whitesell et al., Ibid., 91:8324 (1994). Likewise, mutations of the tumor-suppressor protein p53 lead to the most common molecular genetic defect found in human cancers, and most p53 mutants show extended interactions with HSP90 (probably because of aberrant conformations), preventing their usual ubiquitylation and subsequent degradation by the proteasome (M.V. Blagosklonny et al., Ibid., 93:8379 (1996). However despite its ubiquitous participation, HSP90's clients are mostly pro-growth signaling proteins, and its chaperoning function is subverted during oncogenesis, leading to development of malignant transformation and the maintenance of transformed phenotypes.

In addition to anti-cancer and antitumorgenic activity, HSP90 inhibitors have also been implicated in a wide variety of other utilities, including use as anti-inflammation agents, anti-infectious disease agents, agents for treating autoimmunity, agents for treating ischemia, and agents useful in promoting nerve regeneration (See, e.g., Rosen et al., WO 02/09696; PCT/US01/23640; Degranco et al., WO 99/51223; PCT/US99/07242; Gold, U.S. Pat. No. 6,210,974 B1). There are reports in the literature that fibrogenetic disorders including but not limited to scleroderma, polymyositis, systemic lupus, rheumatoid arthritis, liver cirrhosis, keloid formation, interstitial nephritis, and pulmonary fibrosis may be treatable. (Strehlow, WO 02/02123; PCT/US01/20578).

Ansamycins and other HSP90 inhibitors thus hold great promise for the treatment and/or prevention of many types of disorders. However, many of the natural-product derived Hsp90 inhibitors exhibit pharmaceutical deficiencies; their relative insolubility makes them difficult to formulate and administer, and they are not easily synthesized and currently must, at least in part, be generated through fermentation. Further, the dose limiting toxicity of ansamyins is hepatic. For example, the semi-synthetic inhibitor 17-allylamino,17-desmethoxy-geldanamycin (17-AAG), currently in phase 11 clinical trials, is expensive to manufacture, difficult to formulate (the NCI clinical protocol consists of injecting a DMSO solution of 17-AAG) and at present administered only parenterally. Although the 17-dimethylaminoethylamino analog (17-DMAG) is more soluble, it exhibits all of the side effects of 17-AAG as well as gastrointestinal hemorrhaging in preclinical toxicity studies (Glaze et. al. Proc. Am. Assoc. Cancer. Res. 2003, 44, 162-162 and Eiseman et. al. Cancer Chemother. Pharmacol. 2005, 55, 21-32). Radicicol (RC), another natural product Hsp90 inhibitor, is poorly water-soluble and is inactive in tumor xenograft models. Semi-synthetic oxime derivatives of radicicol provide better solubility and substantially improved the pharmacological profile in murine models, but are still limited to intravenous administration (Ikuina et. al. J. Med. Chem. 2003, 46, 2534-2541. Furthermore, radicicol and its oximes contain an oxirane ring which has been viewed as a liability for stability and toxicity, prompting the synthesis of cycloproparadicicol: Yang et. al. J. Am. Chem. Soc. 2004, 126, 7881 and 2003, 125, 9602-9603.) Despite the potential of ansamycins, alternative HSP90 inhibitors are therefore needed.

Fully synthetic, orally active inhibitors of Hsp90 have been sought in order to provide more flexible dosing schedule options, and to possibly avoid the side-effects of the natural product inhibitors. Chiosis et al. described the design and synthesis of purine analogs that mimic geldanamycin and other ansamycins in their ability to bind the ATP binding pocket of, and thus inhibit, HSP90. See International Patent Application PCT/US01/46303 (WO 02/36075; Chemistry & Biology 8:289-299 (2001). The specific compounds that Chiosis et al. described included a trimethoxybenzyl entity substituted at positions 3, 4, and 5. Using gel-binding assays, these were shown to bind HSP90 approximately 20-fold less avidly than 17-AAG.

More recently, other novel non-natural product Hsp90 inhibitors have been reported (e.g. PU3 and CCT018159; see Chiosis et. al. Bioorg. Med. Chem. Lett. 2002, 10, 3555-3564; Vilenchik et. al. Chem. Biol. 2004, 11, 787-797; Chiosis et. al. WO 0236075, 2002; Drysdale et. al. WO 03/055860 A1, 2003; Wright et. al. Chem. Biol. 2004, 11, 775-785; Dymock et. al. Bioorg. Med. Chem. Lett. 2004, 14, 325-328; Dymock et. al. J. Med. Chem. 2005, 48, 4212-4215. Structure of Hsp90 in complex with PU3 pdb code 1UY6, and with PU24FC1: pdb code 1UYF and Clevenger et. al. Org. Lett. 2004, 6, 4459-4462). The structures of these inhibitors were designed using the crystal structures of Hsp90 in complex with ATP, geldanamycin, or radicicol. The 8-benzyladenines such as PU3 were designed to adopt the same C-shaped conformation as geldanamycin (Chiosis et. al. Current Cancer Drug Targets, 2003, 3, 371-376) with the adenine ring pointing to the adenine-binding site (hinge region), and the trimethoxybenzene ring emulating the H-bond accepting nature of the quinone ring of geldanamycin. (The benzene ring of PU3 was not designed to have exactly the same orientation as the quinone ring of geldanamycin. Rather, the trimethoxybenzene moiety was designed to point in the same general direction and form a hydrogen bond with Lys112, an amino acid which forms a hydrogen bond with the quinone ring of geldanamycin.) The recently obtained crystal structure of Hsp90 in complex with PU3 confirmed that the purine ring occupies the position normally occupied by ADP/ATP, but the benzene ring points in a direction opposite to the predicted one, to form a r-stacking interaction with Phe138. Nevertheless, PU3 inhibits Hsp90 (HER-2 degradation assay, HER-2 IC₅₀=40 µM) and afforded a valuable starting point for further optimization. Structure-activity studies based on PU3 led to the more active PU24FC1 (HER-2 IC₅₀=1.7 µM) which was subsequently also co-crystallized with Hsp90. When PU24FC1 was formulated in DMSO/EtOH/phosphate-buffered saline 1:1:1 and administered intraperitoneally to mice bearing MCF-7 xenograft tumors, it induced at 100-300 mg/kg down-regulation of HER-2 and Raf-1, a pharmacodynamic response consistent with Hsp90 inhibition, and at 200 mg/kg it significantly repressed tumor growth. Very high doses (500-1000 mg/kg) of PU24FC1 were required to observe a similar pharmacodynamic response upon oral administration, and no 8-benzyladenine has been reported to inhibit tumor growth by the oral route. In our hands, PU24FC1 proved to be too insoluble to be effectively formulated and delivered orally. So far, despite extensive SAR studies to improve potency and pharmaceutics properties, Hsp90 inhibitors have not demonstrated activity in animal models of human cancer (xenografts) when administered orally.

The discovery of the 8-benzyladenines led to the design of 8-sulfanyladerlines (Kasibhatla et. al. WO 3037860, 2003 and Llauger et. al. J. Med. Chem. 2005, 48, 2892-2905), exemplified by 8-(2-iodo-5-methoxy-phenylsulfanyl)-9-pent-4-ynyl-9H-purin-6-ylamine, which exhibited excellent potency in several cell-based assays, but was poorly soluble in water and did not have sufficient oral bioavailability in clinically acceptable formulations.

When HSP90 is inhibited, its clients are degraded, i.e., the unfolded protein is ubiquitinated, followed by proteasome-mediated hydrolysis. Most of the inhibitors reported so far bind to the N-terminal domain *(vide infra*)*,* but some are reported to interact with the C-terminal domain; HSP90 has binding sites for ATP in both locations. The function of HSP90's C terminus is not entirely clear, but compounds interacting in this domain clearly impair HSP90 function and have anti-cancer effects. Some resorcylic acid lactones have been found to inhibit HSP90, thus natural products radicicol and geldanamycin (P. Delmotte and J. Delmotte-Plaquee, Nature (London), 171:344 (1953); and C. DeBoer et al., J Antibiot (Tokyo), 23:442 (1970), respectively) were shown to suppress the transformed phenotype of cell expressing activated Src (H.J. Kwon et al., Cancer Research, 52:6926 (1992); Y. Uehara et al., Virology, 164:294 (1988)). Related compounds such as herbimycin have been reported to have similar effects (S. Omura et al., J Antibiot (Tokyo), 32:255 (1979).

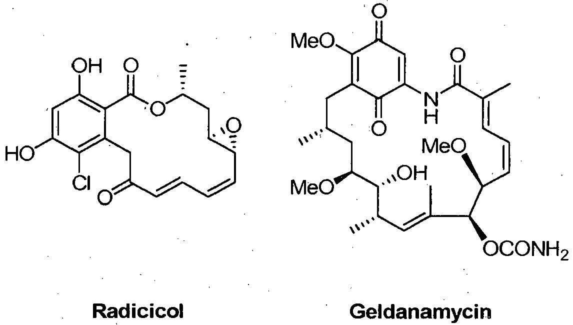

Other resorcylic acid lactones (RALs) studied in this respect include 17-allylamino-17-demethoxygeldanamycin (17AAG) (D.B. Solit et al., Clin. Cancer Res., 8:986 (2002); L.R. Kelland et al., J. Natl. Cancer Inst., 91:1940 (1999)); 17DMAG (J.L. Eiseman et al., Cancer Chemother. Pharmacol, 55:21-32 (2005)); IPI-504 (J. Ge et al., J. Med. Chem., 49:4606 (2006); oxime derivatives such as KF25706 (S. Soga, et al., Cancer Res., 59:2931 (1999)) and KF55823 (S. Soga et al., Cancer Chemotherapy and Pharmacology, 48:435 (2001)); and Danishefsky *et al.'s* cycloproparadicicol (A. Rivkin *et al., Ibid.,* 44:2838 (2005)). Structurally related variants include chimeric inhbitors having radicicol's carboxyresorcinol and the geldanamycin's benzoquinone (R.C. Clevenger and B.S. Blagg, Org. Lett., 6:4459 (2004); G. Shen and B.S. Blagg, Ibid., 7:2157 (2004); G. Shen et al., J. Org. Chem., 71:7618 (2006)).

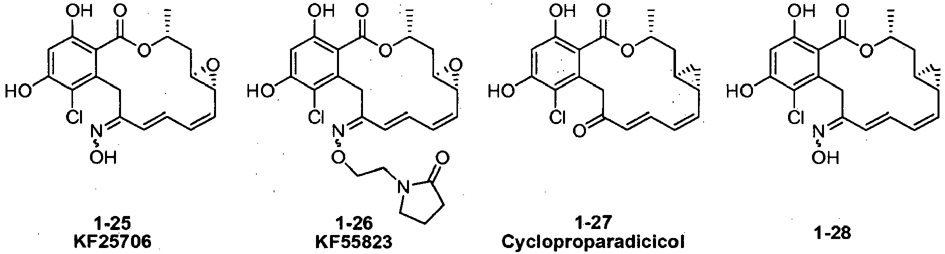

### Radicicol-based HSP90 inhibitors

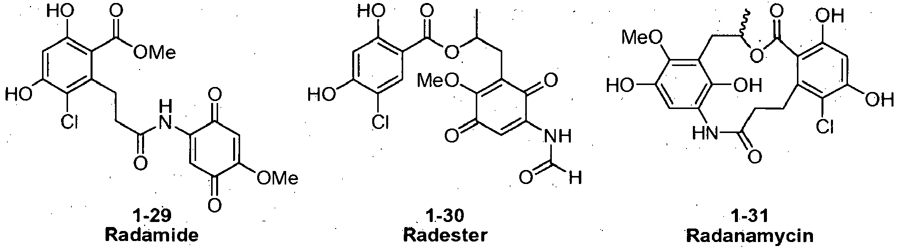

### Chimeric inhibitors of HSP90

Purines such as PU3 have been studied in an effort to design small molecules that fit HSP90's ATP binding site (G. Chiosis, et al., Chem Biol 8, 289-299 (2001); G. Chiosis, et al., Bioorg. Med. Chem., 10:3555 (2002); L. LLauger, et a/., J. Med. Chem.. 48:2892 (2005); H. He et al., Ibid., 49:381 (2006); M.A. Biamonte et al., Ibid., 49:817 (2006)).

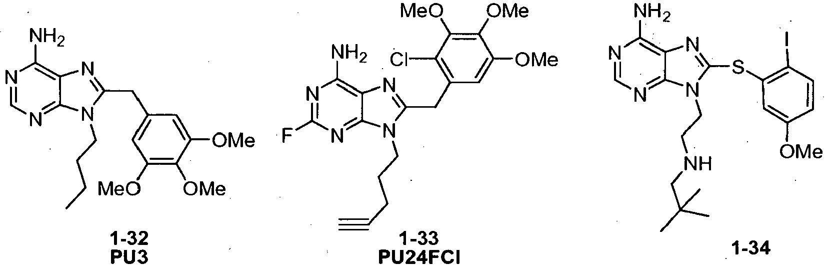

### Purine-based designed HSP90 inhibitors

Pyrazoles **(1**-**35)** (M.G. Rowlands et al., Anal. Biochem., 327:176 (2004); B.W. Dymock et al., J Med. Chem., 48:4212 (2005)) and benzothiazolothio-purines (**1**-**36**) (L. Zhang. et al., J. Med. Chem., 49:5352 (2006) have been reported recently also as small-molecule inhibitors of these enzymes.

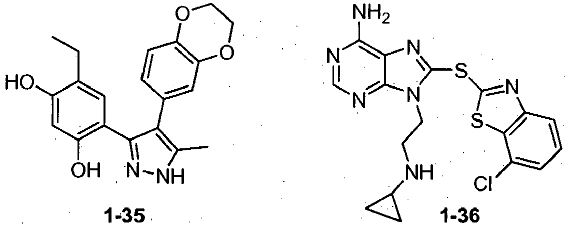

### Other classes of HSP90 inhibitors

Radicicol has been of particular interest. A 14-member macrolide, and also known as monorden, radicicol is a potent, highly competitive and highly selective ligand for HSP90's ATP-binding pocket. HSP90 is an ATPase rather than a kinase, and its ATP-binding pocket has a Bergerat fold (A. Bergerat et al.,, Nature, 386:414 (1997); R. Dutta and M. Inouye, Trends Biochem. Sci., 25:24 (2000)) which is distinct from kinases' ATP-binding pockets. (S. M. Roe et al., J. Med. Chem., 42:260 (1999)). Considerable interest in radicicol's medicinal applications have followed the initial findings. (See U.S. Patent No. 6,946,456; and U.S. Patent Application Publication Nos. 2003-0211469, 2004-0102458, 2005-0074457, 2005-0261263, 2005-0267087, 2006-0073151, 2006-0251574, 2006-0269618, 2007-0004674, and 2007-0010432).

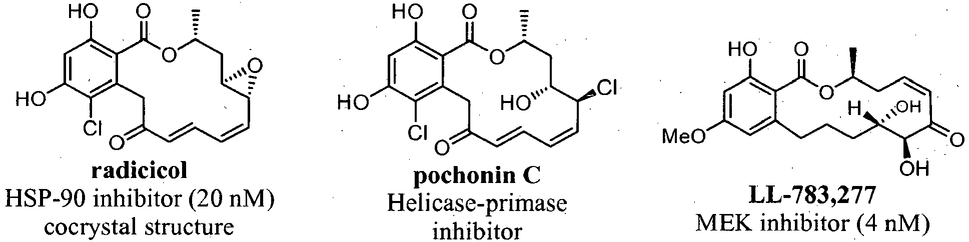

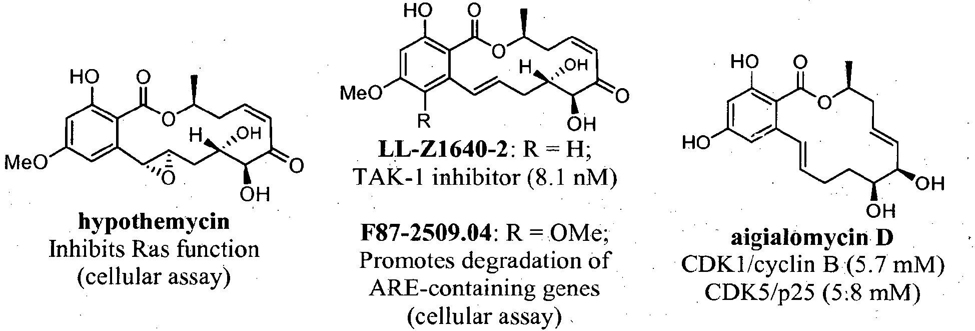

Strikingly, some resorcylic macrolides that are close analogs of radicicol are known to inhibit kinases but *not* HSP90. Indeed, LL-Z1640-2 was found to be a potent and selective inhibitor of TAK1 kinase for which radicicol and other resorcylides were not active. (J. Ninomiya-Tsuji et al., J. Biol. Chem., 278:18485 (2003); P. Rawlins et al., Int. J. Immunopharma., 21:799 (1999); K. Takehana et al., Biochem. Biophys. Res. Comm., 257:19 (1999); A. Zhao et al., J. Antibiotics, 52:1086 (1999)). Closely related LL-783,227, where one of the olefins has been reduced, is a potent inhibitor of MEK kinase. (A. Zhao et al., J. Antibiotics 52:1086 (1999)). Compound F87-2509.04 was found to induce degradation of mRNA containing AU-rich elements (ARE) (T. Kastelic et al., Cytokine, 8:751 (1996)) and hypothemycin was found to inhibit the Ras-mediated cellular signaling. (H. Tanaka et al., Jap. J. Cancer Res., 90:1139 (1999)). We have recently shown that aigialomycin D is a CDK inhibitor. (S. Barluenga et al., Angew. Chem., Int. Ed., 46(24):3951 (2006)).

Other close analogs of radicicol do inhibit HSP90. Pochonin D is a potent inhibitor of HSP90. (E. Moulin et al., J. Am. Chem. Soc., 127(19):6999 (2005)). And pochonin A has been reported to be a 90 nM inhibitor of HSP90. Pochonin C was found to be an inhibitor of herpes' helicase-primase, which is an ATPase rather than a kinase. (V. Hellwig et al., J. Nat. Prod., 66:829 (2003)). Although radicicol and pochonin C are structurally very similar, they have very different conformations in solution, and different biological activities. (S. Barluenga et al., Chem. Eur. J., 11:4935 (2005). Thus it appears the "floppiness" of the macrocyclic may play an essential role in inhibitory differences among resorcylic acid macrolides, and in any case makes those effects difficult to predict by theoretical methods.

Some resorcylic acid macrolides had been known as kinase or phosphatase inhibitors (U.S. Patent Nos. 5,674,892; 5,728,726; 5,731,343; and 5,795,910), or to inhibit other enzymes (U.S. Patent No. 5,710,174 inhibiting FXIIIa catalysis of fibrin cross-linking). Resorcylic acid macrolides were also employed for other medical indications (U.S. Patent Nos. 3,453,367; 3,965,275; 4,035,504; 4,670,249; 4,778,821; 4,902,711; and 6,635,671).

Radicicol and the pochonins are natural products; intermediates for synthesizing some of their analogues of them may be obtained by fermentation, however relying only upon those natural products or their fermentation derivatives severely limits the range of compounds. Thus a number of novel resorcylic acid macrolides have been synthesized. Many of these are zearalane and related compounds in which the macrocyclic ring contains no carbon-carbon double bond other than between carbons of the phenyl ring (U.S. Patent serial nos. 3,373,038; 3,586,701; 3,621,036; 3,631,179; 3,687,982; 3,704,249; 3,751,431; 3,764,614; 3,810,918; 3,836,544; 3,852,307; 3,860,616; 3,901,921; 3,901,922; 3,903,115; 3,957,825; 4,042,602; 4,751,239; 4,849,447; and 2005-0256183). Syntheses have also been reported for resorcylic acid macrolides characterized by one double bond between ring carbons outside the phenyl ring. (U.S. Patent serial nos. 3,196,019; 3,551,454; 3,758,511; 3,887,583; 3,925,423; 3,954,805; and 4,088,658). Most of those are 14-member macrocycles, but syntheses have also been reported for the 12-member macrocycle analogs. (U.S. Patent serial nos. 5,710,174; 6,617,348; and 2004-0063778. and PCT publication no. WO 02/48135)

Syntheses have also been reported for radicicol-related compounds having two non-aromatic double bonds and either a halide or a 1,2-oxo group (i.e., an epoxide) on the macrocyclic ring. (U.S. Patent serial nos. 4,228,079; 5,597,846; 5,650,430; 5,977,165; 7,115,651; and Japanese patent document nos. JP 6-279279A, JP 6-298764A, JP 9-202781A, JP 10-265381A2; and JP 2000-236984). Syntheses of oximes of radicicol-related compounds are disclosed in U.S. Patent serial nos. 5,977,165; 6,239,168; 6,316,491; 6,635,662; 2001-0027208; 2004-0053990; Japanese patent document no. JP 2003-113183A2; and PCT publication no. WO 99/55689 Synthesis of cyclopropa-analogs of radicicol is disclosed in U.S. Patent No. 7,115,651 and PCT Publication No. WO 05/061481. Syntheses of some other resorcylic acid macrolide analogs are disclosed in U.S. patent publication no. 2006-0247448 and in PCT publication no. WO 02/48135. Radicicol as well as Pochonins A and C have also been synthesized. (S. Barluenga et al., Angew. Chemie, 43(26):3467-3470 (2004); S. Barluenga et al., Chemistry - A European Journal, 11(17):4935-4952 (August 19, 2005); E. Moulin et al., et al., Organic Letters, 7(25):5637-5639 (December 8, 2005).

EP 0 460 950A1 to Sankyo Co relates to radicicol derivatives, their preparation and anti-tumor activity.

JP 10 26 5381-1 discloses a radisicol for use in preventing vascular restenosis, in particular after percutaneous coronary angioplasty.

Despite the progress described above, chemical biologists continue to suffer from a limited ability to knock out specific kinase activity in order to deconvolute the role of specific kinases within complex signaling networks. Small molecules that can permeate cells have promise for solving this problem. And it has become increasingly apparent that the biological function of kinases is often regulated by their conformation, which is in turn dictated by their phosphorylation level and by intra- and inter-molecular associations. Small molecule inhibitors also have the potential to discriminate between different conformations of a given kinase, thus small molecules offer a means to dissect the respective functions of those conformation. Unfortunately the portfolio of known kinase inhibitors cannot yet support the full range of work to be done in parsing the roles of the various members of the kinome. This is not a merely academic pursuit, because the rationality of drug design will continue to suffer until kinase mechanisms and their selectivity is understood.

Thus there is an ongoing need for kinase inhibitors and HSP90 inhibitors that not only have improved potency and selectivity, but also have improved solubility and bioavailability.

### SUMMARY OF THE INVENTION

The present application discloses compounds having structural formula I, II, III, IV and V, or their pharmaceutically acceptable salts, solvates, and/or esters; pharmaceutical compositions comprising the compounds or therir pharmaceutically acceptable salts, solvates, and/or esters; and the use of the compounds or therir pharmaceutically acceptable salts, solvates, and/or esters for the treatment of kinase-mediated or HSP90-mediated disorders.

The present application discloses a compound of formula I or a pharmaceutically acceptable salt, solvate, and/or ester thereof: 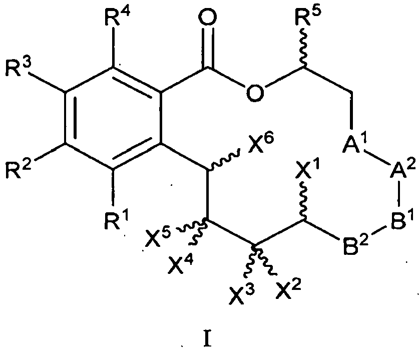 wherein:
R¹, R², R³, and R⁴ are each independently hydrogen, halogen, nitro, cyano, alkyl, alkenyl, alkynyl, arylalkyl, aryl, heteroalkyl, alkylheteroaryl, heterocyclyl, heteroaryl, OR, NR₂, SR, S(O)R, S(O)₂R, -SO₂N(R)₂, -N(R)SO₂R, -N(CO)R,-N(CO)NR₂, -N(CO)OR, -O(CO)R, -(CO)R, -(CO)OR, -(CO)NR₂, -O(CO)OR, -O(CO)NR₂, or a structural formula selected from the group consisting of 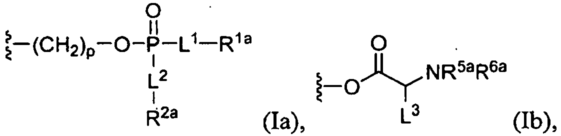 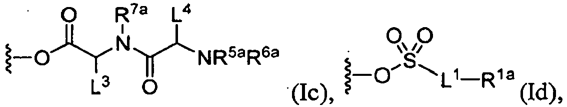 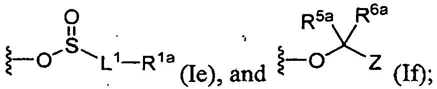 provided that at least one of R¹, R², R³, and R⁴ have a structural formula selected from the group consisting of (Ia), (Ib), (Ic), (Id), (Ie), and (If);
L¹ and L² are each independently a covalent bond, -O-, or -NR^{3a}-;
p is 0, 1, or 2;
R^{1a} and R^{2a} are each independently hydrogen, alkyl, heteroalkyl, heteroaryl, heterocyclyl, alkenyl, alkynyl, arylalkyl, heteroarylalkyl, heterocyclylalkyl, -alkylene-C(O)-O-R^{4a}, or -alkylene-O-C(O)-O-R^{4a}; and
R^{3a} and R^{4a} are each independently hydrogen, alkyl, heteroalkyl, cyclylalkyl, heterocyclyl, aryl, heteroaryl, alkenyl, alkynyl, arylalkyl, heterocyclylalkyl, or heteroarylalkyl;
L³ and L⁴ are each independently hydrogen, halogen, nitro, cyano, alkyl, alkenyl, alkynyl, arylalkyl, aryl, heteroalkyl, heterocyclyl, heteroaryl, heterocyclylalkyl, heteroarylalkyl, OR, NR₂, or SR;
R^{5a}, R^{6a}, and R^{7a} are each independently hydrogen, alkyl, alkenyl, alkynyl, alkylaryl, arylalkyl, aryl, heteroalkyl, alkylheteroaryl, heterocyclyl, or heteroaryl;
R⁵ is hydrogen, halogen, nitro, cyano, alkyl, alkenyl, alkynyl, arylalkyl, aryl, heteroalkyl, alkylheteroaryl, heterocyclyl, heteroaryl, OR, NR₂, SR, S(O)R, S(O)₂R,-SO₂N(R)₂, -N(R)SO₂R, -N(CO)R, -N(CO)NR₂, -N(CO)OR, -O(CO)R, -(CO)R,-(CO)OR, -(CO)NR₂, -O(CO)OR, or -O(CO)NR₂;
Z has a structural formula selected from the group consisting of (Ia), (Ib), (Ic), (Id), and (Ie);
A¹ and A² together are -CH₂-CH₂-, =CH=CH-, -CH(OH)-CH(OH)-, -CH(OH)-CH(halogen)-, -CH(halogen)-CH(OH)-, 1,2-cyclopropadiyl, or 1,2-oxirane;
B¹ and B² together are -CH₂-CH₂- or B₁ and B₂ together represent a covalent bond;
X¹ is hydrogen, halogen, OR, NR₂, NH-OR, SR, S(O)R, S(O)₂R, -N-O-(CH₂)ₙ-CO₂-R; or X¹ together with X² or X³ represents a covalent bond;
X² and X³ are both hydrogen, or one of X² and X³ is hydrogen and the other together with X¹ represents a covalent bond;
X⁴ and X⁵ together are =O, =S, =N-OR, =N-O-(CH₂)ₙCOOR, =N-O-(CH₂)ₙCONR₂, =N-NR₂, =N-N-SOR or =N-N-SO₂R; or one of X⁴ and X⁵ is hydrogen and the other is OH, OR, O(CO)R, O(CO)OR, O(CO)NR₂, -(CH₂)ₙ-O(CO)OR, -(CH₂)ₙ-O(CO)NR₂; or one of X₄ and X⁵ together with X⁶ represents a covalent bond and the other of X⁴ and X⁵ is OH, OR, O(CO)R, O(CO)OR, or O(CO)NR₂;
X⁶ is hydrogen or X⁶ together with one of X⁴ and X⁵ represents a covalent bond;and
each R is independently hydrogen, alkyl, acyl, aryl, alkaryl, arylalkyl, heteroalkyl, heteroaryl, heterocyclyl, a protecting group; or when two R groups are bonded to the same nitrogen, the two R groups taken together with the nitrogen form a 5-8 membered heterocyclic or heteroaryl ring; and
n is 1, 2 or 3.

The present application discloses a compound of formula II or a pharmaceutically acceptable salt, solvate, and/or ester thereof: 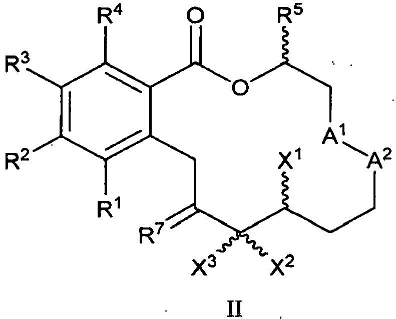 wherein, R⁷ is =O, =S, =N-OR, =N-O-(CH₂)ₙCOOR, =N-O-(CH₂)ₙCONR₂, =N-NR₂, =N-N-SOR or =N-N-SO₂R.

The present application discloses a compound of formula III or a pharmaceutically acceptable salt, solvate, and/or ester thereof: 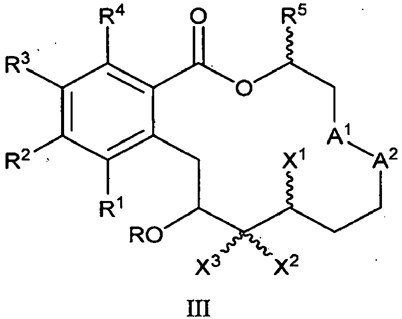 wherein R is hydrogen, alkyl, arylalkyl, acyl or a protecting group.

In one embodiment, the present invention provides a compound of formula IV or a pharmaceutically acceptable salt, solvate, and/or ester thereof: 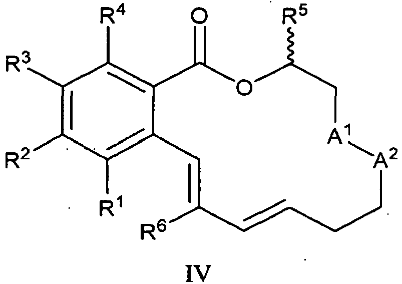 wherein R⁶ is hydrogen, OR, or NR₂.

The present application discloses a compound of formula V or a pharmaceutically acceptable salt, solvate, and/or ester thereof: 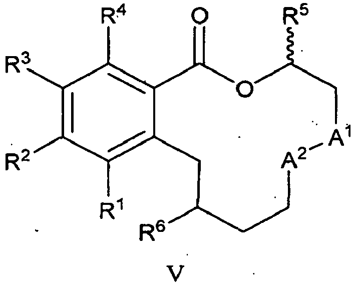 wherein R⁶ is (CH₂)ₙC(O)OR, or -(CH₂)ₙC(O)NR₂; and n is 1, 2 or 3.

Furthermore, a pharmaceutical composition comprising a compound of formula I, II, III, IV or V or its pharmaceutically acceptable salt, solvate, and/or ester thereof in combination with a pharmaceutically acceptable carrier is disclosed.

The present application discloses a method of treating, preventing or ameliorating tumors or symptoms resulting from neurofibromatosis in a subject suffering from neurofibromatosis type 2 (NF2) or a condition associated with the loss of NF2 function or neurofibromatosis type 1 (NF1) or a condition associated with the loss of NF1 function comprising administering to said subject a therapeutically effective amount of at least one compound of formula I, II, III, IV or V, or a pharmaceutically acceptable tautomer, salt, solvate, ester, and/or prodrug thereof.

The present application discloses the use of a compound of formula I, or a pharmaceutically acceptable tautomer, salt, solvate, ester, and/or prodrug thereof, for the manufacture of a medicament for the treatment, prevention, or amelioration of tumors or symptoms resulting from neurofibromatosis in a subject suffering from neurofibromatosis type 2 (NF2) or a condition associated with the loss of NF2 function or neurofibromatosis type 1 (NF1) or a condition associated with the loss of NF1 function.

The present application discloses a method of treating, preventing or ameliorating a neurodegenerative disease in a patient comprising administering to said patient a therapeutically effective amount of at least one compound of formula I, II, III, IV, or V, or a pharmaceutically acceptable tautomer, salt, solvate, ester, and/or prodrug thereof.

The present application discloses the use of a compound of formula I, II, III, IV, or V, or a pharmaceutically acceptable tautomer, salt, solvate, ester, and/or prodrug thereof, for the manufacture of a medicament for the treatment, prevention, or amelioration of a neurodegenerative disease,

Furthermore,, the present application discloses a method of inhibiting or reducing the growth or number of NF2-deficient tumor cells or NF1-deficient tumor cells comprising contacting said NF2-deficient tumor cells or NF1-deficient tumor cells with at least one compound of formula I, II, III, IV or V, or a pharmaceutically acceptable tautomer, salt, solvate, ester, and/or prodrug thereof.

Moreover, a pharmaceutical composition comprising an effective HSP 90-inhibiting amount of a compound of formula I, II, III, IV or V in combination with a pharmaceutically acceptable carrier is disclosed. In some embodiments, the carrier is suitable for oral, parenteral, inhalation, topical or intradermal administration.

In still other embodiments, the pharmaceutical compositions comprising the compounds of formula I, II, III, IV or V comprises particles that are less than about 2 microns average particle size. In stil other embodiments, the composition is incorporated into a biodegradable or non-biodegradable polymer.

In one embodiment, the composition comprises an additive selected from an antioxidant, a buffer, a bacteriostat, a liquid carrier, a solute, a suspending agent, a thickening agent, a flavoring agent, a gelatin, glycerin, a binder, a lubricant, an inert diluent, a preservative, a surface active agent, a dispersing agent, a biodegradable polymer, or any combination thereof.

In another embodiment, a method of treating a patient with a disease comprising administering to the patient with the disease an effective amount of a compound of formula I, II, III, IV or V, wherein the disease is an autoimmune disease, an inflammatory disease, a neurological or neurodegenerative disease, cancer, a cardiovascular disease, allergy, asthma, or a hormone-related disease is disclosed.

In one embodiment, a method of treating a patient with cancer is disclosed comprising administering to the patient having the cancer an effective cancer-treating amount of a compound of formula I, II, III, IV or V, wherein the cancer may be a solid tumor, a blood borne tumor, breast cancer, cancer of the ovary, cancer of the cervix, prostate cancer, cancer of the testis, cancer of the urinary tract, cancer of the esophagus, cancer of the larynx, glioblastoma, stomach cancer, skin cancer, keratoacanthoma, lung cancer, epidermoid carcinoma, large cell carcinoma, small cell carcinoma, lung adenocarcinoma, bone cancer, colon cancer, adenoma, cancer of the pancreas, adenocarcinoma, thyroid cancer, follicular carcinoma, undifferentiated carcinoma, papillary carcinoma, seminoma, melanoma, sarcoma, bladder carcinoma, liver carcinoma and biliary passages, kidney carcinoma, myeloid disorders, lymphoid disorders, Hodgkin's, hairy cells, buccal cavity cancer, pharynx cancer, lip cancer, tongue cancer, mouth cancer, cancer of the pharynx, cancer of the small intestine, colon-rectum cancer, cancer of the large intestine, cancer of the rectum, brain cancer and cancer of the central nervous system, or leukemia.

In another embodiment, the application dislcoses a method of treating a patient with a disease associated with undesirable neovascularization comprising administering to the patient with the undersirable neovascularization an effective amount of a compound of formula I, II, III, IV or V.

The disease associated with undesirable neovasculariation comprises ocular neovascular disease, diabetic retinopathy, retinopathy of prematurity, corneal graft rejection, neovascular glaucoma and retrolental fibroplasias, epidemic keratoconjunctivitis, Vitamin A deficiency, contact lens overwear, atopic keratitis, superior limbic keratitis, pterygium keratitis sicca, Sjögren's syndrome, acne rosacea, phylectenulosis, syphilis, *Mycobacteria* infections, lipid degeneration, chemical bums, bacterial ulcers, fungal ulcers, *Herpes simplex* infections, *Herpes zoster* infections, protozoan infections, Kaposi's sarcoma, Mooren's ulcer, Terrien's marginal degeneration, marginal keratolysis, trauma, rheumatoid arthritis, systemic lupus, polyarteritis, Wegener's sarcoidosis, Scleritis, Steven-Johnson disease, pemphigoid, radial keratotomy, or corneal graph rejection, sickle cell anemia, sarcoid, pseudoxanthoma elasticum, Paget's disease, vein occlusion, artery occlusion, carotid obstructive disease, chronic uveitis/vitritis, Lyme's disease, systemic lupus erythematosis, Eales' disease, Bechet's disease, infections causing a retinitis or choroiditis, presumed ocular histoplasmosis, Best's disease, myopia, optic pits, Stargart's disease, pars planitis, chronic retinal detachment, hyperviscosity syndromes, toxoplasmosis, or post-laser complications.

In still another embodiment, a method of treating a patient with an inflammatory disease is disclosed comprising administering to the patient with the inflammatory disease an effective amount of a compound of formula I, II, III, IV or V.

The inflammatory disease may be excessive or abnormal stimulation of endothelial cells, atherosclerosis, vascular malfunctions, abnormal wound healing, inflammatory and immune disorders, Bechet's disease, gout or gouty arthritis, abnormal angiogenesis accompanying rheumatoid arthritis, skin diseases, psoriasis, diabetic retinopathy, retinopathy of prematurity, retrolental fibroplasia, macular degeneration, corneal graft rejection, neovascular glaucoma or Osler Weber syndrome.

In particular, the present invention provides the subject-matter of claims 1 to 18 attached hereto.

### BRIEF DESCRIPTION OF THE DRAWINGS

Figures 1A and 1B show the *In vitro* hydrolysis of phosphate prodrugs 1a and 1b to their parent compounds in liver homogenate and intestine homogenate.

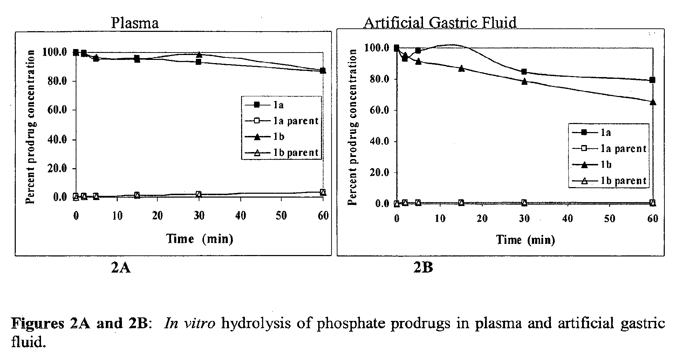

Figures 2A and 2B show the *In vitro* hydrolysis of phosphate prodrugs 1a and 1b to their parent compounds in plasma and artificial gastric fluid.

### DETAILED DESCRIPTION OF THE INVENTION

Provided are novel compounds based on the resorcylic acid lactones that are useful as inhibitors of kinases and HSP90. Also provided are compositions comprising the compounds and processes for the preparation of the compounds. Use of the compounds for the inhibition of kinases and HSP-90, and a method for the treatment of kinase-mediated or HSP90-mediated diseases comprising administering an effective kinase-inhibiting amount or an effective HSP90-inhibiting amount of a compound of formula II to a patient with a kinase-mediated or HSP90-mediated disease, are provided.

### Compounds

In one embodiment, the present invention provides a compound of formula II, or a pharmaceutically acceptable salt, solvate, or ester thereof: 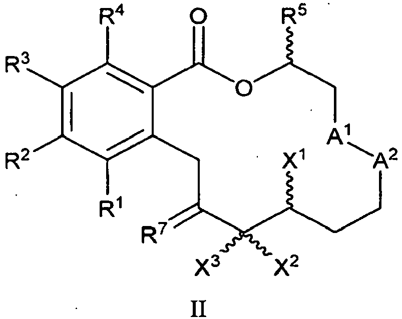 wherein:
R¹, R², R³, and R⁴ are each independently hydrogen, halogen, nitro, cyano, alkyl, alkenyl, alkynyl, arylalkyl, aryl, heteroalkyl, alkylheteroaryl, heterocyclyl, heteroaryl, OR, NR₂, SR, S(O)R, S(O)₂R, -SO₂N(R)₂, -N(R)SO₂R, -N(CO)R,-N(CO)NR₂, -N(CO)OR, -O(CO)R, -(CO)R, -(CO)OR, -(CO)NR₂, -O(CO)OR, -O(CO)NR₂, or a structural formula selected from the group consisting of 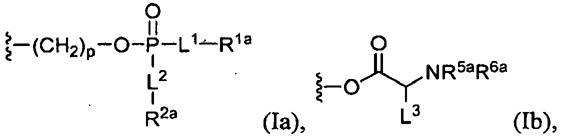 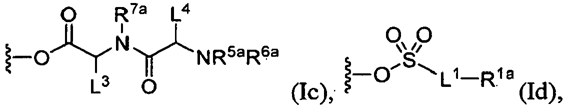 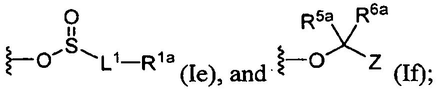 provided that at least one of R¹, R², R³, and R⁴ have a structural formula selected from the group consisting of (Ia), (Ib), (Ic), (Id), (Ie), and (If), wherein each R can be the same or different;
L¹ and L² are each independently a covalent bond, -0-, or NR^{3a}-;
p is 0, 1, or 2;
R^{1a} and R^{2a} are each independently hydrogen, alkyl, heteroalkyl, heteroaryl, heterocyclyl, alkenyl, alkynyl, arylalkyl, heteroarylalkyl, heterocyclylalkyl, -alkylene-C(O)-O-R^{4a}, or -alkylene-O-C(O)-O-R^{4a}; and
R^{3a} and R^{4a} are each independently hydrogen, alkyl, heteroalkyl, cyclylalkyl, heterocyclyl, aryl, heteroaryl, alkenyl, alkynyl, arylalkyl, heterocyclylalkyl, or heteroarylalkyl;
L³ and L⁴ are each independently hydrogen, halogen, nitro, cyano, alkyl, alkenyl, alkynyl, arylalkyl, aryl, heteroalkyl, heterocyclyl, heteroaryl, heterocyclylalkyl, heteroarylalkyl, OR, NR₂, or SR, wherein each R can be the same or different;
R^{5a}, R^{6a}, and R^{7a} are each independently hydrogen, alkyl, alkenyl, alkynyl, alkylaryl, arylalkyl, aryl, heteroalkyl, alkylheteroaryl, heterocyclyl, or heteroaryl;
R⁵ is hydrogen, halogen, nitro, cyano, alkyl, alkenyl, alkynyl, arylalkyl, aryl, heteroalkyl, alkylheteroaryl, heterocyclyl, heteroaryl, OR, NR₂, SR, S(O)R, S(O)₂R,-SO₂N(R)₂, -N(R)SO₂R, -N(CO)R, -N(CO)NR₂, -N(CO)OR, -O(CO)R, -(CO)R,-(CO)OR, -(CO)NR₂, -O(CO)OR, or -O(CO)NR₂, wherein each R can be the same or different;
Z has a structural formula selected from the group consisting of (Ia), (Ib), (Ic), (Id), and (Ie);
A¹ and A² together are -CH₂-CH₂-, -CH=CH-, -CH(OH)-CH(OH)-, -CH(OH)-CH(halogen)-, -CH(halogen)-CH(OH)-, 1,2-cyclopropadiyl, or 1,2-oxirane;
X¹ is hydrogen, halogen, OR, NR₂, NH-OR, SR, S(O)R, S(O)₂R, -N-O-(CH₂)ₙ-CO₂-R; or X¹ together with X² or X³ represents a covalent bond, wherein each R can be the same or different;
X² and X³ are both hydrogen, or one of X² and X³ is hydrogen and the other together with X¹ represents a covalent bond, wherein, R⁷ is =O, =S, =N-OR, =NO-(CH₂)ₙCOOR, =N-O-(CH₂)ₙCONR₂, =N-NR₂, =N-N-SOR or =N-N-SO₂R; and
n is 1, 2 or 3.

In one embodiment of the compound of formula II, R¹ is H, halogen or heterocyclyl.

In one embodiment of the compound of formula II, R⁵ is hydrogen, alkyl, aryl, heteroaryl or arylalkyl.

In one embodiment of the compound of formula II, A¹ and A² together are -CH=CH-.

In one embodiment of the compound of formula II, A¹ and A² together are -CH(OH)-CH(OH)-, -CH(OH)-CH(halogen)- or -CH(halogen)-CH(OH)-.

In one embodiment of the compound of formula II, A¹ and A² together are 1,2-oxirane.

In one embodiment of the compound of formula II, R¹ is H, Cl or heterocyclyl; R⁵ is hydrogen, alkyl, , aryl or arylalkyl; A¹ and A² together are -CH=CH- or -C(OH)-C(OH)-; X¹ is hydrogen, halogen or NH-OR; and R⁷ is =O, =S, =N-OR, =N-O-(CH₂)ₙCOOR, =N-O-(CH₂)ₙCONR₂, =N-NR₂, =N-N-SOR, =N-N-SO₂R. Preferably, R⁷ is =O. It is also preferable that R⁷ is =N-OR, =N-O-(CH₂)ₙCOOR, or =N-O-(CH₂)ₙCONR₂.

In one embodiment of the compound of formula II, R¹ is H, Cl or heterocyclyl; R⁵ is hydrogen, alkyl, , aryl or arylalkyl; A¹ and A² together are 1,2-oxirane; X¹ is hydrogen, halogen or NH-OR; and R⁷ is =O, =S, =N-OR, =N-O-(CH₂)ₙCOOR, =N-O-(CH₂)ₙCONR₂, =N-NR₂, =N-N-SOR, =N-N-SO₂R.

In one embodiment of the compound of formula II, R¹ is H, Cl or heterocyclyl; R⁵ is hydrogen, alkyl, lower alkyl, aryl or arylalkyl; A¹ and A² together are -CH=CH- or -C(OH)-C(OH)-; X¹ together with X² represent a bond; and R⁷ is =O, =S, =N-OR, =N-O-(CH₂)ₙCOOR, =N-O-(CH₂)ₙCONR₂, =N-NR₂, =N-N-SOR, =N-N-SO₂R. Preferable, R¹ is H or Cl; R⁵ is hydrogen, methyl, propyl, isopropyl or phenyl; and R⁷ is =N-OR, =NO-(CH₂)ₙCOOR, or =N-O-(CH₂)ₙCONR₂. It is also preferable that R¹ is Cl and R⁵ is hydrogen. It is also preferable that n is 1. It is also preferable that R⁵ is hydrogen and R⁷ is =N-O-(CH₂)ₙCOOR, or =N-O-(CH₂)ₙCONR₂. It is also preferable that R⁵ is hydrogen and R⁷ is =N-OR.

In one embodiment of the compound of formula II, R¹ is H, Cl or heterocyclyl; R⁵ is hydrogen, alkyl, lower alkyl, aryl or arylalkyl; A¹ and A² together are 1,2-oxirane; X¹ together with X² represent a bond; and R⁷ is =O, =S, =N-OR, =N-O-(CH₂)ₙCOOR, =N-O-(CH₂)ₙCONR₂, =N-NR₂, =N-N-SOR, =N-N-SO₂R. Preferably, R⁷ is =O. It is also preferable that R⁷ is =N-OR, =N-O-(CH₂)ₙCOOR, or =N-O-(CH₂)ₙCONR₂.

In certain embodiments of the present invention, R² and R⁴ in any of the above formula structures are independently OR, or a structural formula selected from the group consisting of (Ia), (Ib), (Ic), (Id), (Ie), and (If); R is independently alkyl, acyl, aryl, alkaryl, arylalkyl, heteroalkyl, heteroaryl, heterocyclyl, a protecting group; provided that at least one of R² and R⁴ is a structural formula selected from the group consisting of (Ia), (Ib), (Ic), (Id), (Ie), and (If).

In certain embodiments, the present invention provides compounds having a structural formula selected from the group consisting of 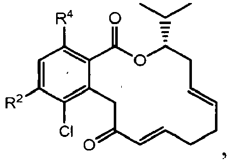 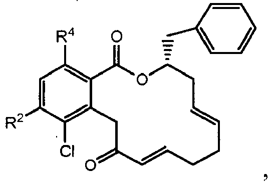 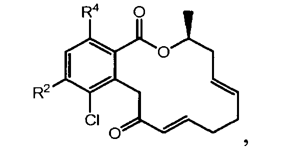 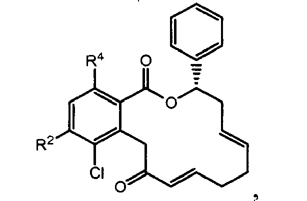 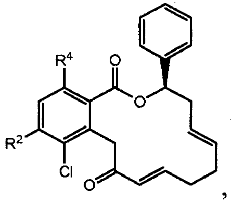 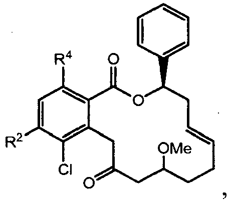 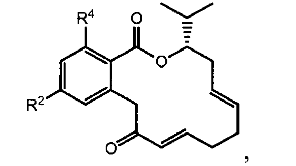 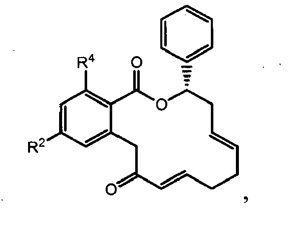 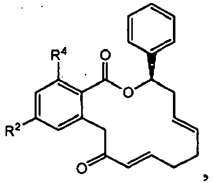 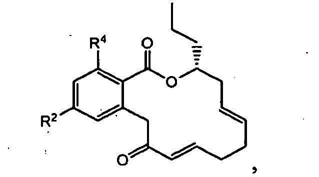 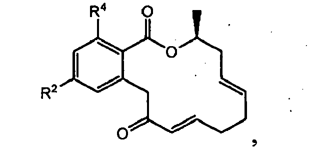 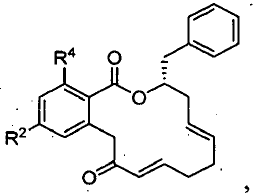 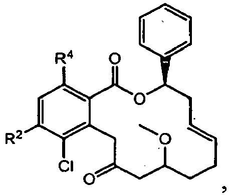 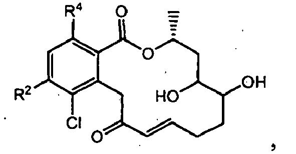 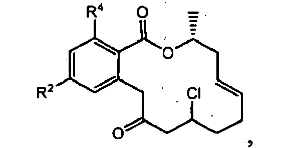 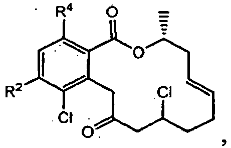 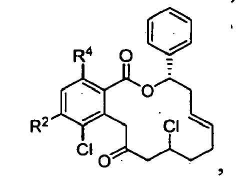 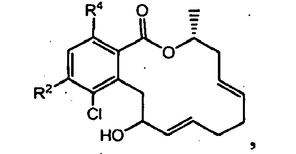 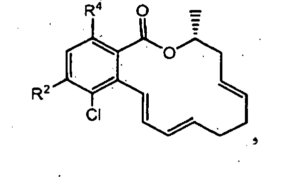 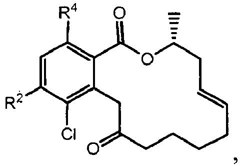 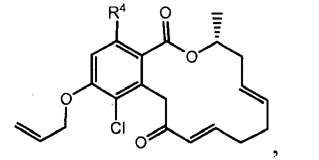 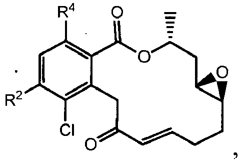 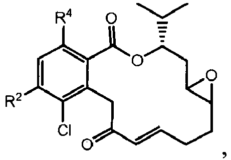 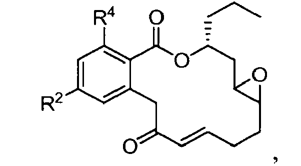 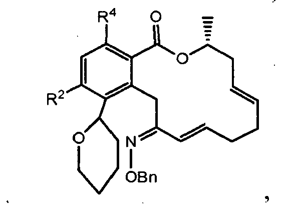 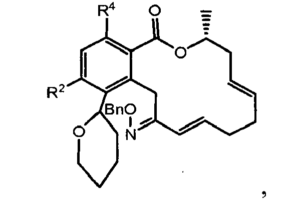 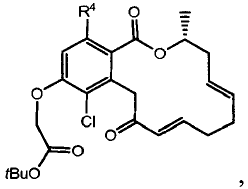 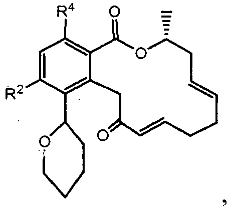 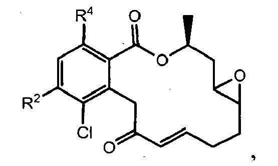 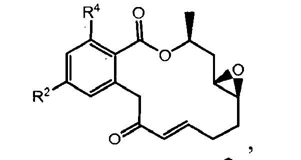 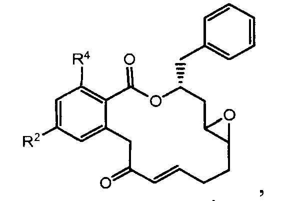 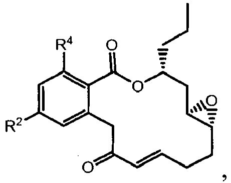 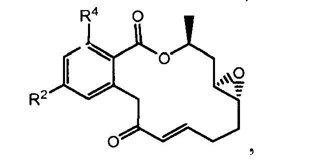 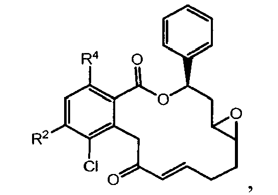 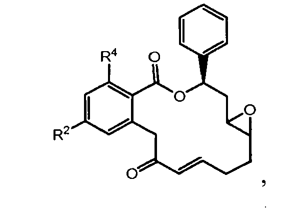 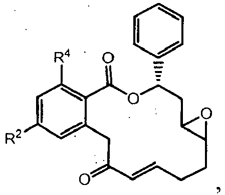 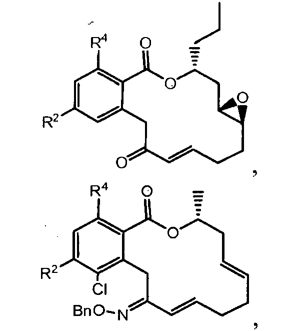 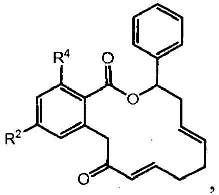 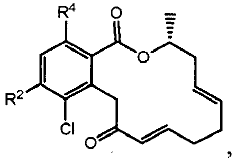 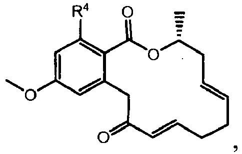 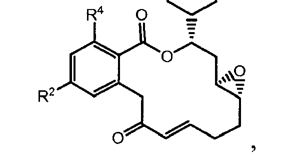 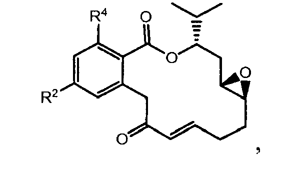 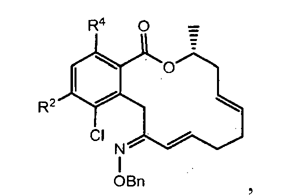 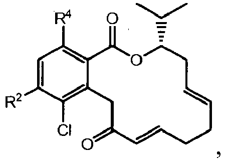 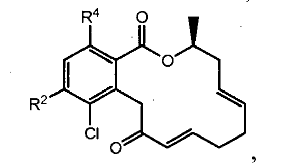 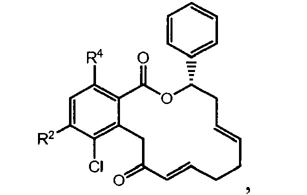 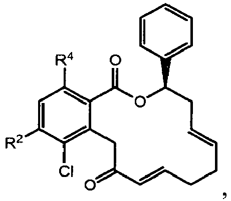 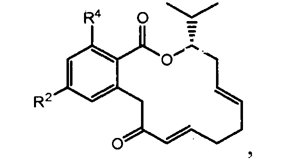 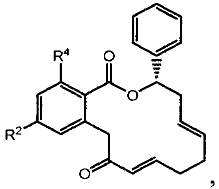 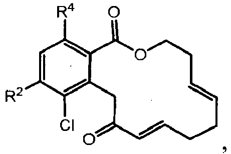 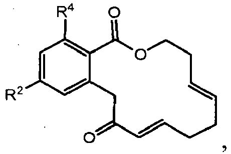 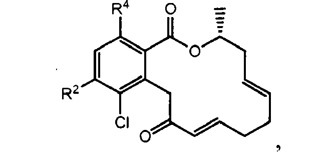 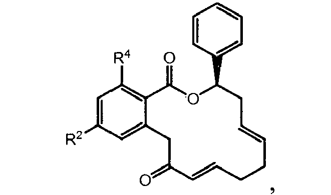 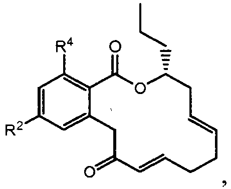 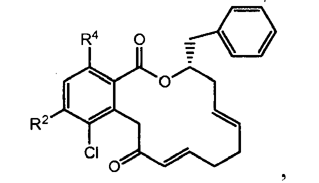 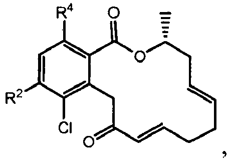 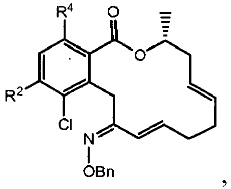 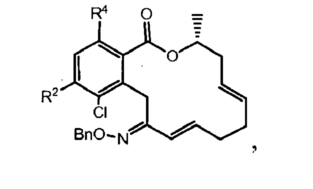 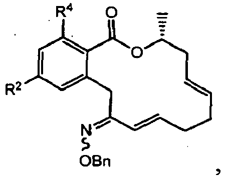 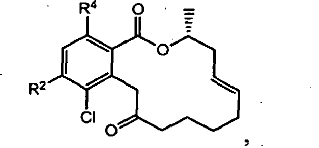 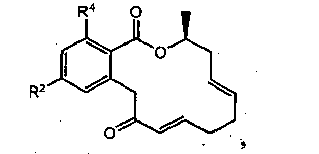 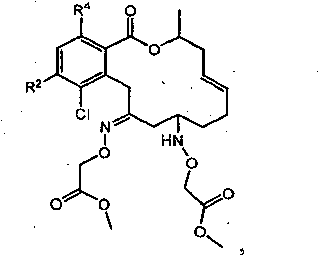 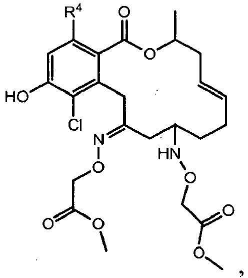 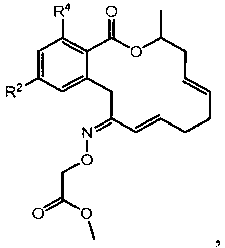 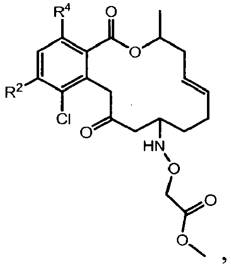 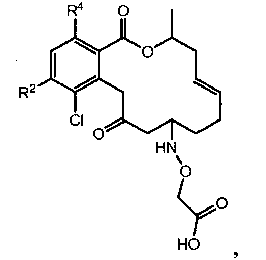 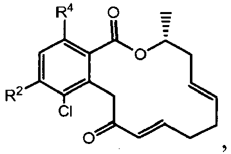 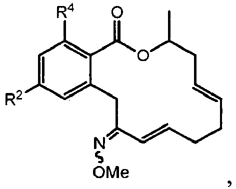 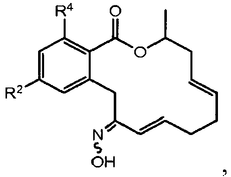 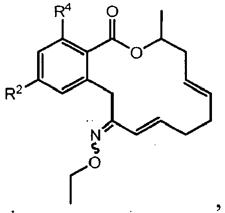 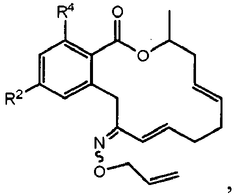 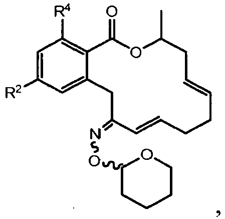 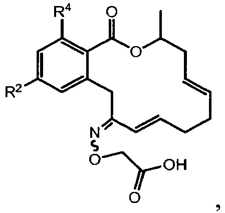 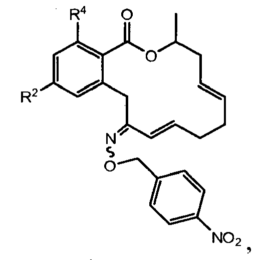 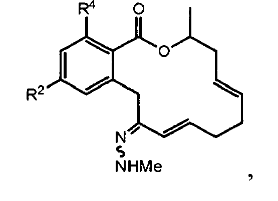 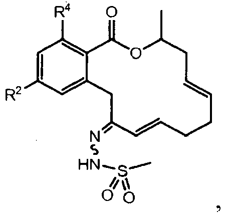 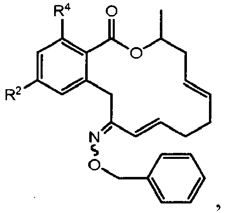 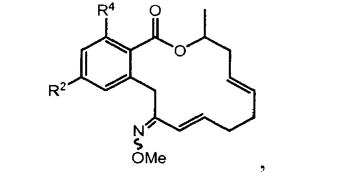 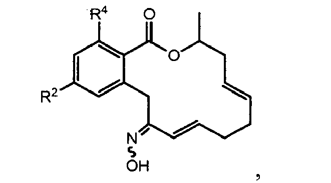 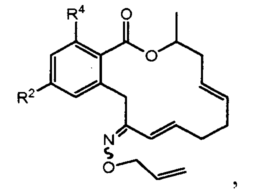 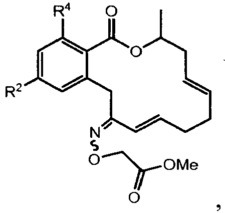 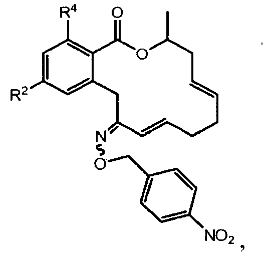 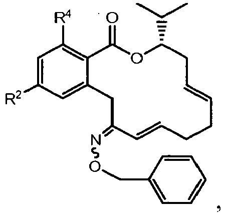 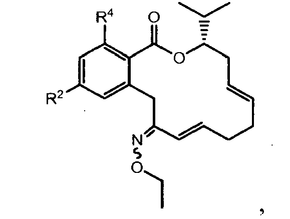 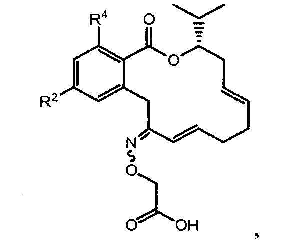 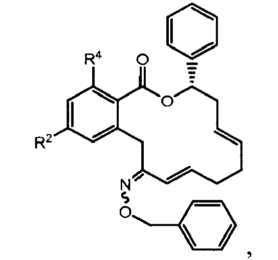 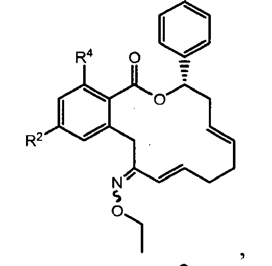 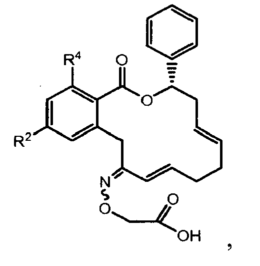 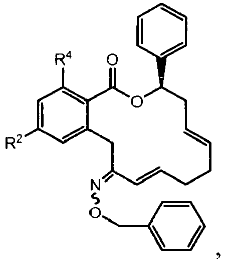 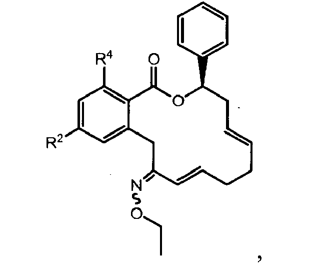 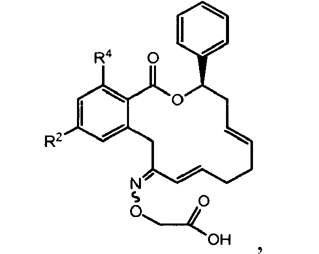 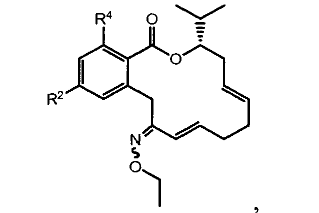 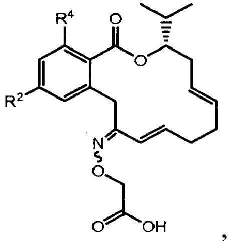 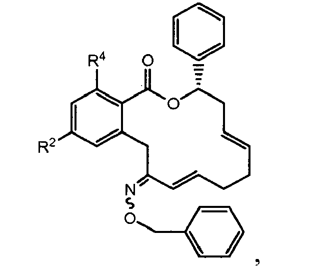 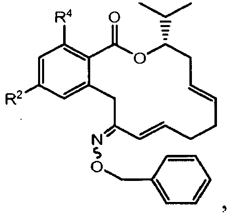 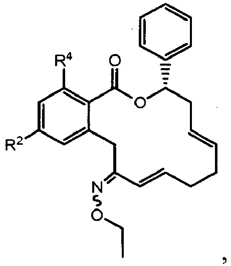 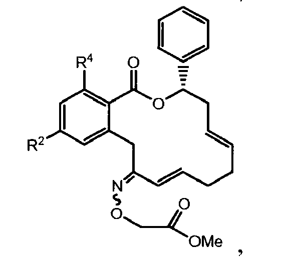 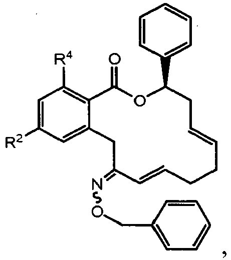 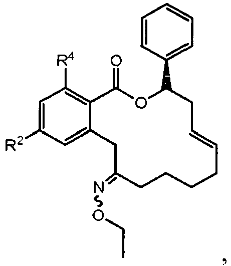 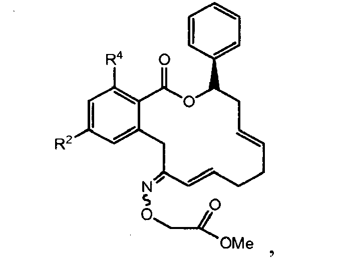 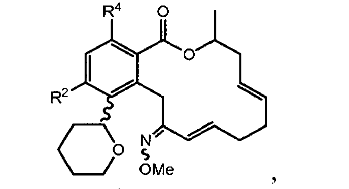 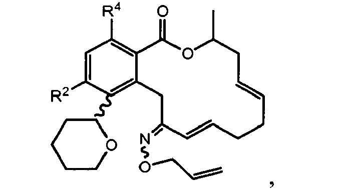 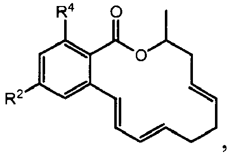 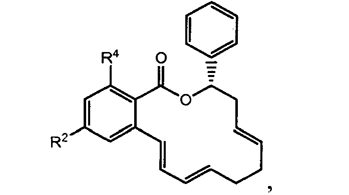 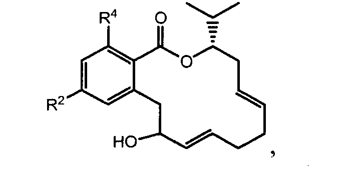 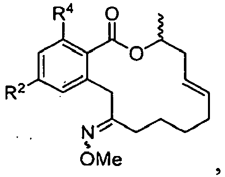 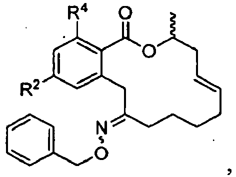 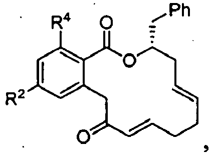 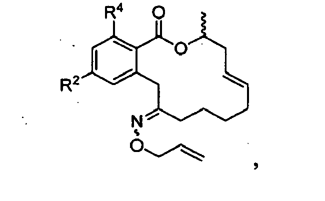 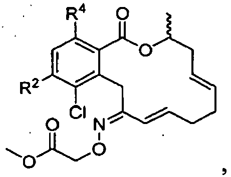 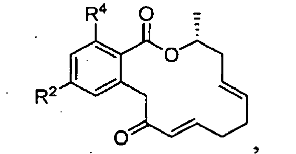 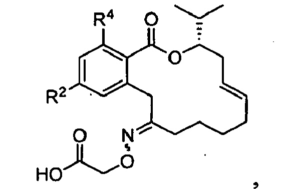 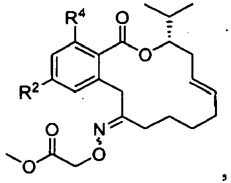 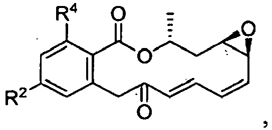 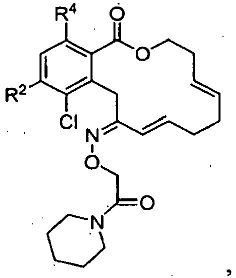 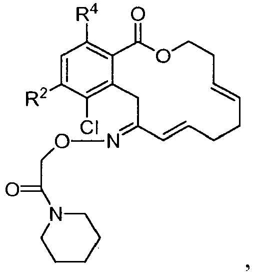 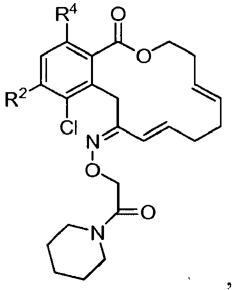 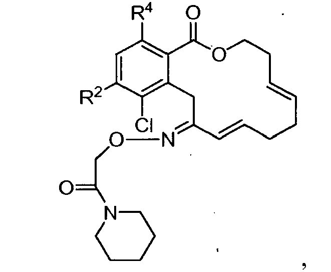 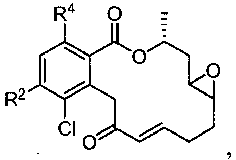 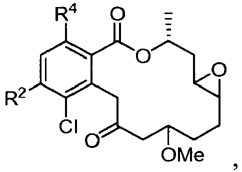 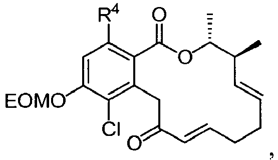 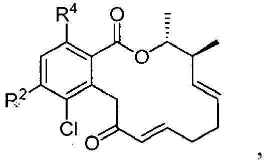 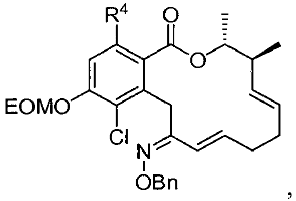 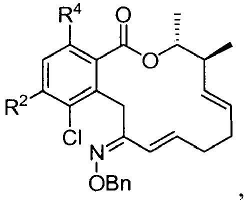 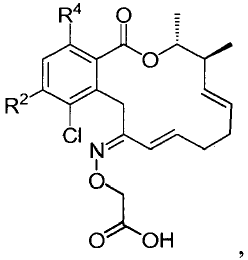 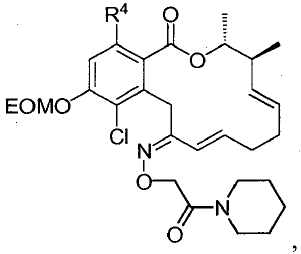 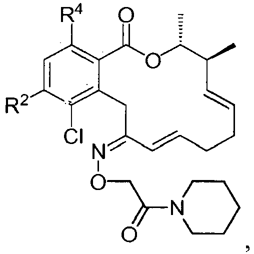 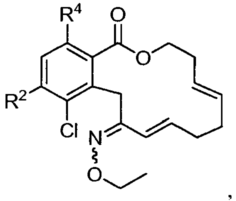 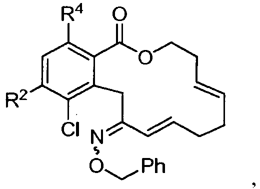 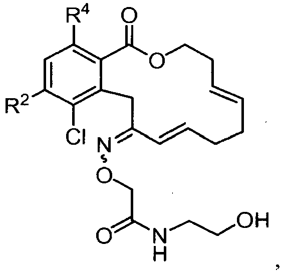 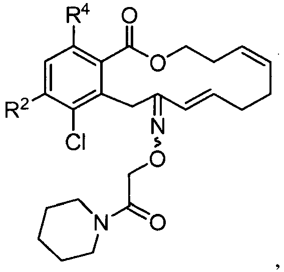 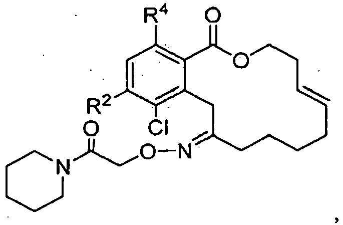 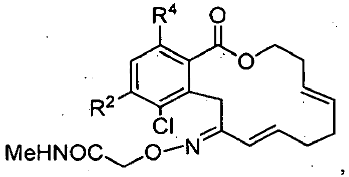 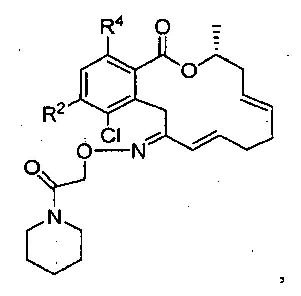 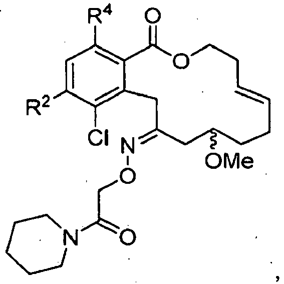 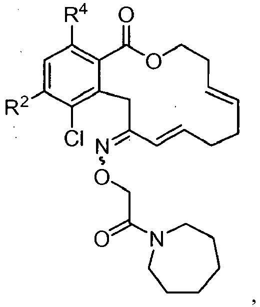 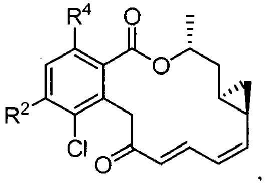 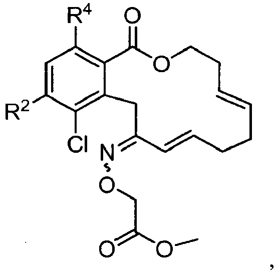 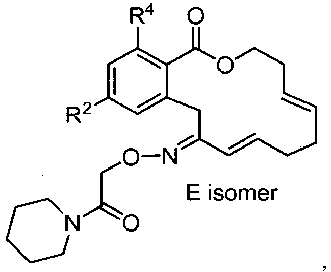 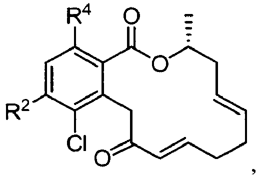 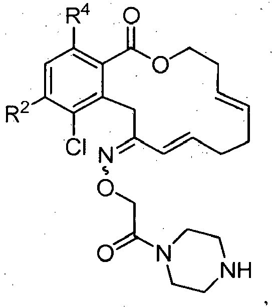 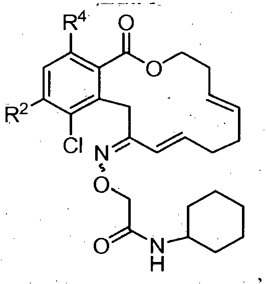 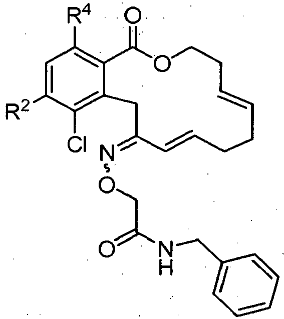 and 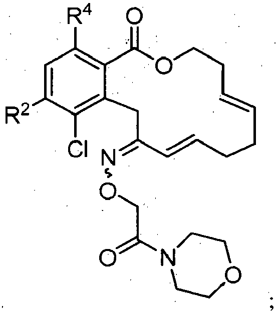 or a pharmaceutically acceptable salt, solvate, or ester thereof.

In one embodiment, the present invention provides compounds wherein one of R² and R⁴ has structural formula (Ia), at least one of L¹ and L² is -O-, and p is 0 or 1. Preferably, L¹ and L² are both -O-.

In one embodiment, the present invention provides compounds wherein one of R² and R⁴ has structural formula (Ib), and R^{5a} and R^{6a} are independently hydrogen or lower alkyl.

In one embodiment, the present invention provides compounds wherein one of R² and R⁴ has structural formula (Ic), and R^{5a}, R^{6a}, and R^{7a} are independently hydrogen or lower alkyl.

In one embodiment, the present invention provides compounds wherein one of R² and R⁴ has structural formula (Id), and L¹ is -O-.

In one embodiment, the present invention provides compounds wherein one of R² and R⁴ has structural formula (Ie), and L¹ is -O-.

In one embodiment, the present invention provides compounds wherein one of R² and R⁴ has structural formula (If), and R^{5a} and R^{6a} are independently hydrogen or lower alkyl.

In some specific embodiments, the present invention provides compounds having a structural formula selected from the group consisting of 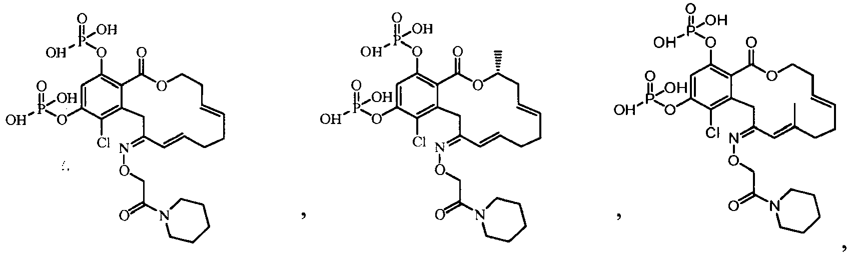 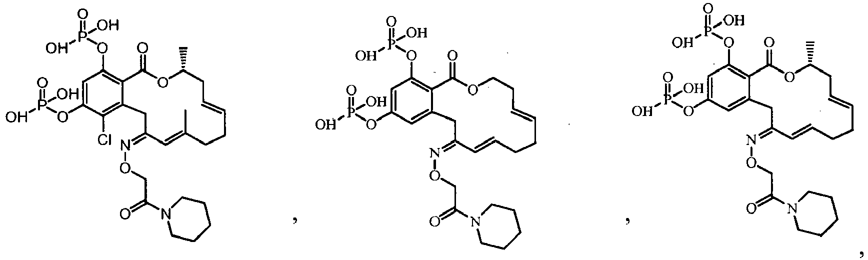 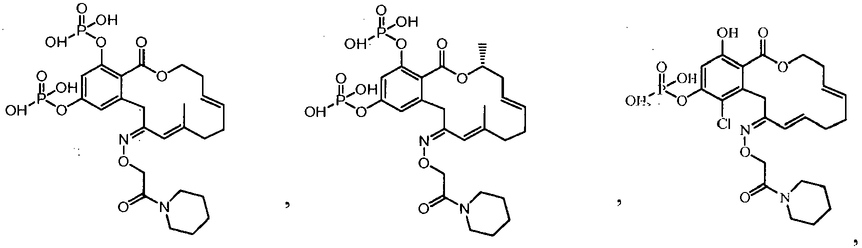 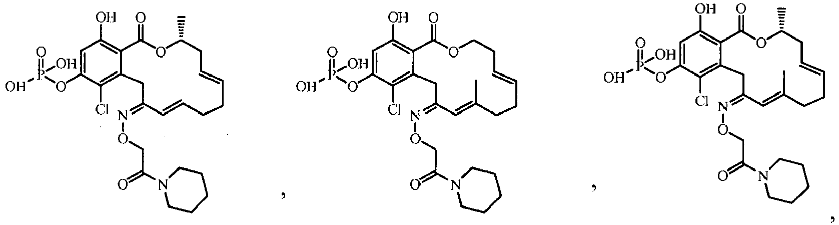 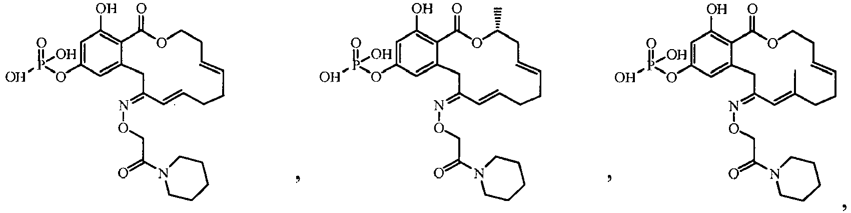 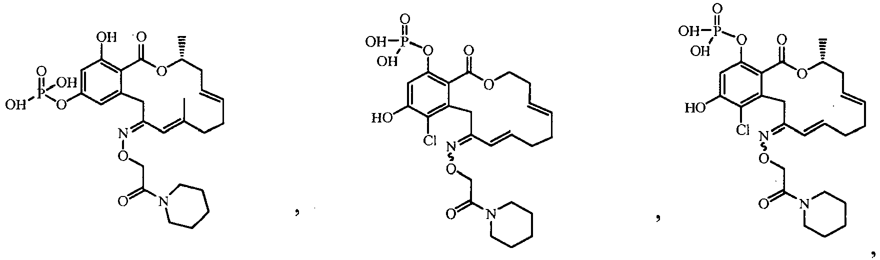 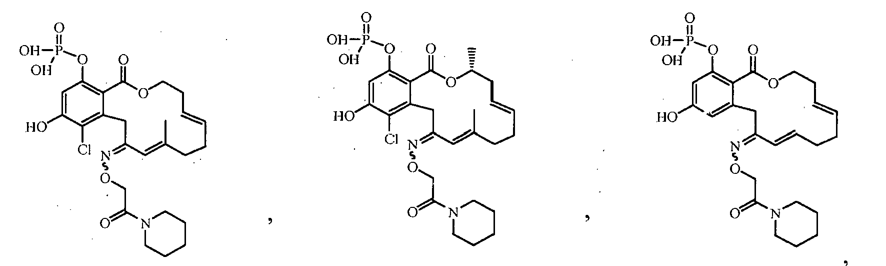 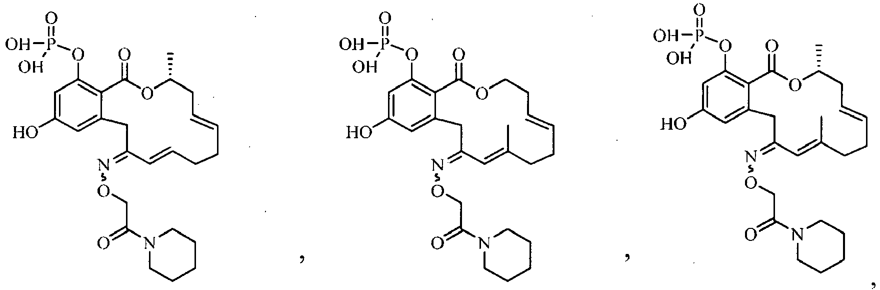 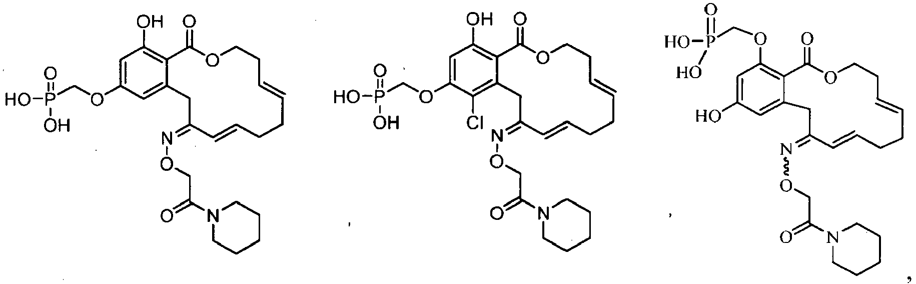 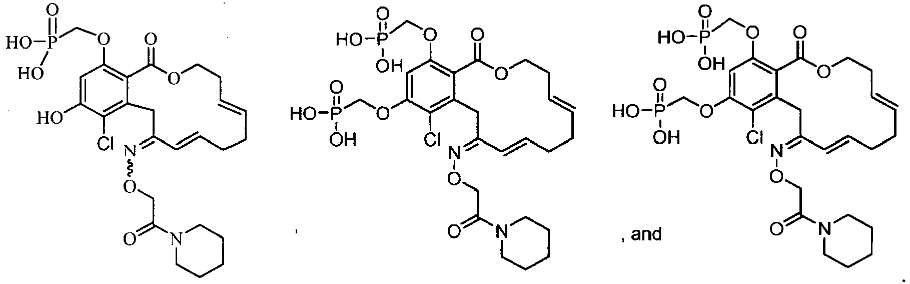 or a pharmaceutically acceptable salt, solvent, or ester thereof.

### Additional Specific Compounds

In one aspect, the present invention also provides specific compounds having a structural formula selected from the group consisting of 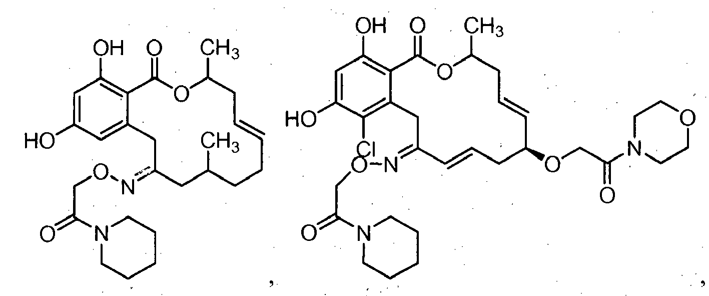 and 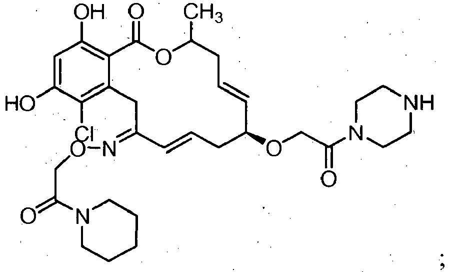 or a pharmaceutically acceptable salt, solvent, or ester thereof.

These specific compounds can be formulated in the pharmaceutical compositions described herein and be used for treating various diseases as described herein.

### Stereoisomerism and Polymorphism

Compounds of the present invention having a chiral center may exist in and be isolated in optically active and racemic forms. The present invention encompasses any racemic, optically-active, diastereomeric, polymorphic, or stereoisomeric form, or mixtures thereof, of a compound of the invention, which possess the useful properties described herein.

In one embodiment, the compounds are prepared in optically active form by asymmetric synthesis using the processes described herein or synthetic transformations known to those skilled in the art.

Examples of methods to obtain optically active materials are known in the art, and include at least the following.
i) physical separation of crystals--a technique whereby macroscopic crystals of the individual enantiomers are manually separated. This technique can be used if crystals of the separate enantiomers exist, i.e., the material is a conglomerate, and the crystals are visually distinct;
ii) simultaneous crystallization--a technique whereby the individual enantiomers are separately crystallized from a solution of the racemate, possible only if the latter is a conglomerate in the solid state;
iii) enzymatic resolutions--a technique whereby partial or complete separation of a racemate by virtue of differing rates of reaction for the enantiomers with an enzyme;
iv) enzymatic asymmetric synthesis--a synthetic technique whereby at least one step of the synthesis uses an enzymatic reaction to obtain an enatiomerically pure or enriched synthetic precursor of the desired enantiomer;
v) chemical asymmetric synthesis--a synthetic technique whereby the desired enantiomer is synthesized from an achiral precursor under conditions that produce asymmetry (i.e., chirality) in the product, which may be achieved using chrial catalysts or chiral auxiliaries;
vi) diastereomer separations--a technique whereby a racemic compound is reacted with an enantiomerically pure reagent (the chiral auxiliary) that converts the individual enantiomers to diastereomers. The resulting diastereomers are then separated by chromatography or crystallization by virtue of their now more distinct structural differences and the chiral auxiliary later removed to obtain the desired enantiomer;
vii) first- and second-order asymmetric transformations--a technique whereby diastereomers from the racemate equilibrate to yield a preponderance in solution of the diastereomer from the desired enantiomer or where preferential crystallization of the diastereomer from the desired enantiomer perturbs the equilibrium such that eventually in principle all the material is converted to the crystalline diastereomer from the desired enantiomer. The desired enantiomer is then released from the diastereomer;
viii) kinetic resolutions--this technique refers to the achievement of partial or complete resolution of a racemate (or of a further resolution of a partially resolved compound) by virtue of unequal reaction rates of the enantiomers with a chiral, non-racemic reagent or catalyst under kinetic conditions;
ix) enantiospecific synthesis from non-racemic precursors--a synthetic technique whereby the desired enantiomer is obtained from non-chiral starting materials and where the stereochemical integrity is not or is only minimally compromised over the course of the synthesis;
x) chiral liquid chromatography--a technique whereby the enantiomers of a racemate are separated in a liquid mobile phase by virtue of their differing interactions with a stationary phase. The stationary phase can be made of chiral material or the mobile phase can contain an additional chiral material to provoke the differing interactions;
xi) chiral gas chromatography--a technique whereby the racemate is volatilized and enantiomers are separated by virtue of their differing interactions in the gaseous mobile phase with a column containing a fixed non-racemic chiral adsorbent phase;
xii) extraction with chiral solvents--a technique whereby the enantiomers are separated by virtue of preferential dissolution of one enantiomer into a particular chiral solvent; or
xiii) transport across chiral membranes--a technique whereby a racemate is placed in contact with a thin membrane barrier. The barrier typically separates two miscible fluids, one containing the racemate, and a driving force such as concentration or pressure differential causes preferential transport across the membrane barrier. Separation occurs as a result of the non-racemic chiral nature of the membrane which allows only one enantiomer of the racemate to pass through.

### Definitions

Whenever a term in the specification is identified as a range (i.e. C₁₋₄ alkyl), the range independently refers to each element of the range. As a non-limiting example, C₁₋₄ alkyl means, independently, C₁, C₂, C₃ or C₄ alkyl. Similarly, when one or more substituents are referred to as being "independently selected from" a group, this means that each substituent can be any element of that group, and any combination of these groups can be separated from the group. For example, if R¹ and R² can be independently selected from X, Y and Z, this separately includes the groups R¹ is X and R² is X; R¹ is X and R² is Y; R¹ is X and R² is Z; R¹ is Y and R² is X; R¹ is Y and R² is Y; R¹ is Y and R² is Z; R¹ is Z and R² is X; R¹ is Z and R² is Y; and R¹ is Z and R² is Z.

The term "alkyl" as used herein, unless otherwise specified, refers to a saturated straight, branched, or cyclic, primary, secondary, or tertiary hydrocarbon, including but not limited to groups with C₁ to C₁₀. The term "alkyl" also includes "lower alkyl".

The term "lower alkyl" refers to a saturated straight, branched, or cyclic, primary, secondary, or tertiary hydrocarbon, including groups with C₁ to C₄, and if appropriate a cyclic alkyl group (for example cyclopropyl).

Illustrative examples of alkyl groups are methyl, ethyl, propyl, isopropyl, cyclopropyl, butyl, secbutyl, isobutyl, *tert*butyl, cyclobutyl, 1-methylbutyl, 1,1-dimethylpropyl, pentyl, cyclopentyl, isopentyl, neopentyl, cyclopentyl, hexyl, isohexyl, and cyclohexyl. Unless otherwise specified, the alkyl group can be unsubstituted or substituted with one or more moieties selected from the group consisting of alkyl, halo, haloalkyl, hydroxyl, carboxyl, acyl, acyloxy, amino, amido, carboxyl derivatives, alkylamino, dialkylamino, arylamino, alkoxy, aryloxy, nitro, cyano, thiol, imine, sulfonic acid, sulfate, sulfonyl, sulfanyl, sulfinyl, sulfamonyl, ester, carboxylic acid, amide, phosphonyl, phosphinyl, phosphoryl, phosphine, thioester, thioether, acid halide, anhydride, oxime, hydrozine, carbamate, phosphonic acid, phosphate, phosphonate, or any other viable functional group that does not inhibit the pharmacological activity of this compound, either unprotected, or protected as necessary, as known to those skilled in the art, for example, as taught in Greene, et al., Protective Groups in Organic Synthesis, John Wiley and Sons, Second Edition, 1991.

The term "halo" or "halogen", as used herein, includes chloro, bromo, iodo, and fluoro.

The term "chiral" as used herein includes a compound that has the property that it is not superimposable on its mirror image.

The term "alkylthio" refers to a straight or branched chain alkylsulfide of the number of carbons specified, such as for example, C₁₋₄alkylthio, ethylthio, -S-alkyl, -S-alkenyl, -S-alkynyl, etc.

The terms "alkylamino"or "arylamino" refer to an amino group that has one or two alkyl or aryl substituents, respectively. Unless otherwise specifically stated in this application, when alkyl is a suitable moiety, then it is a lower alkyl, whether substituted or unsubstituted.

The term "alkylsulfonyl" means a straight or branched alkylsulfone of the number of carbon atoms specified, as for example, C₁₋₆alkylsulfonyl or methylsulfonyl.

The term "alkoxycarbonyl" refers to a straight or branched chain ester of a carboxylic acid derivative of the number of carbon atoms specified, such as for example, a methoxycarbonyl, MeOCO-.

As used herein, the term "nitro" means -NO₂; the term "sulfhydryl means -SH; and the term "sulfonyl" means -SO₂.

The terms "alkenyl" and "alkynyl" refer to alkyl moieties, including both substituted and unsubstituted forms wherein at least one saturated C-C bond is replaced by a double or triple bond. Thus, C₂₋₆ alkenyl may be vinyl, allyl, 1-propenyl, 2-propenyl, 1-butenyl, 2-butenyl, 3-butenyl, 1-pentenyl, 2-pentenyl, 3-pentenyl, 4-pentenyl, 1-hexenyl, 2-hexenyl, 3-hexenyl, 4-hexenyl, or 5-hexenyl. Similarly, C₂₋₆ alkynyl may be ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, or 5-hexynyl.

The term "alkylene" includes a saturated, straight chain, divalent alkyl radical of the formula -(CH₂)ₙ-, wherein "n" may be any whole integer from 1 to 10.

"Alkyl", "alkoxy", "alkenyl", "alkynyl", etc., includes both straight chain and branched groups. However, reference to an individual radical such as "propyl" embraces only that straight-chain radical, whereas a branched chain isomer such as "isopropyl" is specifically termed such.

The term "aryl" as used herein and unless otherwise specified refers to any stable monocyclic, bicyclic, or tricyclic carbon ring of up to 8 members in each ring, wherein at least one ring is aromatic as defined by the Huckel 4n+2 rule, and especially phenyl, biphenyl, or naphthyl. The term includes both substituted and unsubstituted moieties. The aryl group can be substituted with any described moiety, including but not limited to one or more moieties selected from the group consisting of halogen (fluoro, chloro, bromo or iodo), hydroxyl, amino, azido, alkylamino, arylamino, alkoxy, aryloxy, nitro, cyano, sulfonic acid, sulfate, phosphonic acid, phosphate, or phosphonate, either protected or unprotected as necessary, as known to those skilled in the art, for example, as taught in Greene et al., Protective Groups in Organic Synthesis, John Wiley & Sons, 3rd Ed., 1999.

The term "alkaryl" or "alkylaryl" refers to an alkyl group with an aryl substituent or an alkyl group linked to the molecule through an aryl group as defined herein. The term "aralkyl" or "arylalkyl" refers to an aryl group substituted with an alkyl substituent or linked to the molecule through an alkyl group as defined above.

The term "cycloalkyl" includes a ring of C₃₋₈, including but not limited to cyclopropyl, cyclobutyl, cyclopentyl, cyclohexyl, cycloheptyl and cyclooctyl.

The term "alkoxy" means a straight or branched chain alkyl group having an attached oxygen radical, the alkyl group having the number of carbons specified or any number within this range. For example, a "-O-alkyl", C₁₋₄ alkoxy, methoxy, etc.

The term "acyl" or "O-linked ester" includes a group of the formula C(O)R', wherein R' is an straight, branched, or cyclic alkyl (including lower alkyl), carboxylate residue of an amino acid, aryl including phenyl, heteroaryl, alkaryl, aralkyl including benzyl, alkoxyalkyl including methoxymethyl, aryloxyalkyl such as phenoxymethyl; or substituted alkyl (including lower alkyl), aryl including phenyl optionally substituted with chloro, bromo, fluoro, iodo, C₁ to C₄ alkyl or C₁ to C₄ alkoxy, sulfonate esters such as alkyl or aralkyl sulphonyl including methanesulfonyl, the mono, di or triphosphate ester, trityl or monomethoxy-trityl, substituted benzyl, alkaryl, aralkyl including benzyl, alkoxyalkyl including methoxymethyl, aryloxyalkyl such as phenoxymethyl. Aryl groups in the esters optimally comprise a phenyl group. In nonlimiting embodiments, acyl groups include acetyl, trifluoroacetyl, methylacetyl, cyclopropylacetyl, cyclopropyl- carboxy, propionyl, butyryl, isobutyryl, hexanoyl, heptanoyloctanoyl, neo-heptanoyl, phenylacetyl, 2-acetoxy-2-phenylacetyl, diphenylacetyl, α-methoxy-α-trifluoromethyl-phenylacetyl, bromoacetyl, 2-nitro-benzeneacetyl, 4-chloro-benzeneacetyl, 2-chloro-2,2-diphenylacetyl, 2-chloro-2-phenylacetyl, trimethylacetyl, chlorodifluoroacetyl, perfluoroacetyl, fluoroacetyl, bromodifluoroacetyl, methoxyacetyl, 2-thiopheneacetyl, chlorosulfonylacetyl, 3-methoxyphenylacetyl, phenoxyacetyl, tert-butylacetyl, trichloroacetyl, monochloro-acetyl, dichloroacetyl, 7H-dodecafluoro-heptanoyl, perfluoro-heptanoyl, 7H-dodeca-fluoroheptanoyl, 7-chlorododecafluoro-heptanoyl, 7-chloro-dodecafluoro-heptanoyl, 7H-dodecafluoroheptanoyl, 7H-dodeca-fluoroheptanoyl, nona-fluoro-3,6-dioxa-heptanoyl, nonafluoro-3,6-dioxaheptanoyl, perfluoroheptanoyl, methoxybenzoyl, methyl 3-amino-5-phenylthiophene-2-carboxyl, 3,6-dichloro-2-methoxy-benzoyl, 4-(1,1,2,2-tetrafluoro-ethoxy)-benzoyl, 2-bromo-propionyl, omega-aminocapryl, decanoyl, n-pentadecanoyl, stearyl, 3-cyclopentyl-propionyl, 1-benzene-carboxyl, O-acetylmandelyl, pivaloyl acetyl, 1-adamantane-carboxyl, cyclohexane-carboxyl, 2,6-pyridinedicarboxyl, cyclopropane-carboxyl, cyclobutane-carboxyl, perfluorocyclohexyl carboxyl, 4-methylbenzoyl, chloromethyl isoxazolyl carbonyl, perfluorocyclohexyl carboxyl, crotonyl, 1-methyl-1H-indazole-3-carbonyl, 2-propenyl, isovaleryl, 1-pyrrolidinecarbonyl, 4-phenylbenzoyl.

The term "acylamino" includes a group having a structure of "-N(R')-C(=O)-R'", wherein each R' is independently as defined above.

The term "carbonyl" includes a group of the structure "-C(=O)-X-R'" or "X-C(=O)-R'", where X is O, S, or a bond, and each R is independently as defined above.

The term "heteratom" includes an atom other than carbon or hydrogen in the structure of a heterocyclic compound, nonlimiting examples of which are nitrogen, oxygen, sulfur, phosphorus or boron.

The term "heterocycle", "heterocyclyl", or "heterocyclic" as used herein includes non-aromatic ring systems having four to fourteen members, preferably five to ten, in which one or more ring carbons, preferably one to four, are each replaced by a heteroatom. Examples of heterocyclic rings include 3-1-H-benzimidazol-2-one, (1-substituted)-2-oxo-benzimidazol-3-yl, 2-tetrahydro-furanyl, 3-tetrahydrofuranyl, 2-tetrahydropyranyl, 3-tetrahydropyranyl, 4-tetra-hydropyranyl, [1,3]-dioxalanyl, [1,3]-dithiolanyl, [1,3]-dioxanyl, 2-tetra-hydro-thiophenyl, 3-tetrahydrothiophenyl, 2-morpholinyl, 3-morpholinyl, 4-morpholinyl, 2-thiomorpholinyl, 3-thiomorpholinyl, 4-thiomorpholinyl, 1-pyrrolidinyl, 2-pyrrolidinyl, 3-pyrrolidinyl, 1-piperazinyl, 2-piperazinyl, 1-piperidinyl, 2-piperidinyl, 3-piperidinyl, 4-piperidinyl, 4-thiazolidinyl, diazolonyl, N-substituted diazolonyl, 1-phthalimidinyl, benzoxanyl, benzopyrrolidinyl, benzopiperidinyl, benzoxolanyl, benzothiolanyl, and benzothianyl. Also included within the scope of the term "heterocyclyl" or "heterocyclic", as it is used herein, is a group in which a non-aromatic heteroatom-containing ring is fused to one or more aromatic or non-aromatic rings, such as in an indolinyl, chromanyl, phenanthridinyl, or tetrahydroquinolinyl, where the radical or point of attachment is on the non-aromatic heteroatom-containing ring. The term "heterocycle", "heterocyclyl", or "heterocyclic" whether saturated or partially unsaturated, also refers to rings that are optionally substituted.

The term "heteroaryl", used alone or as part of a larger moiety as in "heteroaralkyl" or "heteroarylalkoxy", refers to heteroaromatic ring groups having five to fourteen members. Examples of heteroaryl rings include 2-furanyl, 3-furanyl, 3-furazanyl, N-imidazolyl, 2-imidazolyl, 4-imidazolyl, 5-imidazolyl, 3-isoxazolyl, 4-isoxazolyl, 5-isoxazolyl, 2-oxadiazolyl, 5-oxadiazolyl, 2-oxazolyl, 4-oxazolyl, 5-oxazolyl, 1-pyrrolyl, 2-pyrrolyl, 3-pyrrolyl, 1-pyrazolyl, 2-pyrazolyl, 3-pyrazolyl, 2-pyridyl, 3-pyridyl, 4-pyridyl, 2-pyrimidyl, 4-pyrimidyl, 5-pyrimidyl, 3-pyridazinyl, 2-thiazolyl, 4-thiazolyl, 5-thiazolyl, 5-tetrazolyl, 2-triazolyl, 5-triazolyl, 2-thienyl, 3-thienyl, carbazolyl, benzimidazolyl, benzothienyl, benzofuranyl, indolyl, quinolinyl, benzotriazolyl, benzothiazolyl, benzooxazolyl, benzimidazolyl, isoquinolinyl, indazolyl, isoindolyl, acridinyl, and benzoisoxazolyl. Also included within the scope of the term "heteroaryl", as it is used herein, is a group in which a heteroatomic ring is fused to one or more aromatic or nonaromatic rings where the radical or point of attachment is on the heteroaromatic ring. Examples include tetrahydroquinolinyl, tetrahydroisoquino-linyl, and pyrido [3,4-d]pyrimidinyl. The term "heteroaryl" also refers to rings that are optionally substituted. The term "heteroaryl" may be used interchangeably with the term "heteroaryl ring" or the term "heteroaromatic".

The term "amino" as used herein unless otherwise specified, includes a moiety represented by the structure "-NR₂", and includes primary, secondary and tertiary amines optionally substituted by alkyl, aryl, heterocyclyl, and/or sulfonyl groups. Thus R₂ may represent two hydrogen atoms, two alkyl moieties, or one hydrogen and one alkyl moiety.

The term "amido" as used herein includes an amino-substituted carbonyl, while the term "amidino" means a group having the structure "-C(=NH)-NH₂".

The term "quaternary amine" as used herein includes quaternary ammonium salts that have a positively charged nitrogen. They are formed by the reaction between a basic nitrogen in the compound of interest and an appropriate quaternizing agent such as, for example, methyliodide or benzyliodide. Appropriate counterions accompanying a quaternary amine include acetate, trifluoroacetate, chloro, bromo and iodo ions.

It should be understood that the above-mentioned functional groups, such as alkyl, alkenyl, alkynyl, heteroalkyl, aryl, heteroaryl, arylalkyl, alkylaryl, etc, include the substituted form of those funcitonal groups, i.e., substituted alkyl, substituted alkenyl, substituted alkynyl, substituted heteroalkyl, substituted aryl, substituted heteroaryl, substituted arylalkyl, substituted alkylaryl, etc,. The term "substituted" includes multiple degrees of substitution by one or more named substituents such as, for example, halo, hydroxyl, thio, alkyl, alkenyl, alkynyl, nitro, cyano, azido, amino, carboxamido, etc. Where multiple substituent possibilities exist, the compound can be substituted by one or more of the disclosed or claimed substituent groups, independently from one another, and taken singly or plurally.

The term "protected" as used herein and unless otherwise defined refers to a group that is added to an oxygen, nitrogen, or phosphorus atom to prevent its further reaction or for other purposes. A wide variety of oxygen and nitrogen protecting groups are known to those skilled in the art of organic synthesis.

The term "protecting group" as used herein refers to a group that may be attached to a reactive group, including heteroatoms such as oxygen or nitrogen, to prevent the the reactive group from participating in a reaction. Any protecting groups taught in Greene, et al., Protective Groups in Organic Synthesis, John Wiley and Sons, Second Edition, 1991 may be used. Examples of suitable protecting groups include but are not limited to alkoxyalkyl groups such as ethoxymethyl and methoxymethyl; silyl protecting groups, such tert-butyldimethyl silyl (TBS), phenyldimethylsilyl, trimethylsilyl (TMS), 2-trimethylsilylethoxymethyl (SEM) and 2-trimethylsilylethyl; and benzyl and substituted benzyl.

It should be understood that the various possible stereoisomers of the groups mentioned above and herein are within the meaning of the individual terms and examples, unless otherwise specified. As an illustrative example, "1-methyl-butyl" exists in both (R) and the (S) form, thus, both (R)-1-methyl-butyl and (S)-1-methyl-butyl is covered by the term "1-methyl-butyl", unless otherwise specified.

"Salt thereof" means any acid and/or base addition salt of a compound of the present invention. The term "salt thereof" includes but is not limited to pharmaceutically acceptable salt thereof.

"Solvate thereof" means a compound of the present invention formed by solvation (the combination of solvent molecules with molecules or ions of the solute), or an aggregate that consists of a solute ion or molecule with one or more solvent molecules. One example of solvent is hydrate. The term "solvate thereof" includes but is not limited to pharmaceutically acceptable solvate thereof.

"Ester thereof" means any ester of a compound of the present invention in which any of the -COOH functions of the molecule is replaced by a -COOR function, in which the R moiety of the ester is any carbon-containing group which forms a stable ester moiety, including but not limited to alkyl, alkenyl, alkynyl, cycloalkyl, cycloalkylalkyl, aryl, arylalkyl, heterocyclyl, heterocyclylalkyl and substituted derivatives thereof. The term "ester thereof" includes but is not limited to pharmaceutically acceptable ester thereof.

"Pharmaceutically acceptable" means a salt, solvate, and/or ester of a compound of the present invention which is, within the scope of sound medical judgment, suitable for use in contact with the tissues of humans and lower animals without undue toxicity, irritation, allergic response, and the like, commensurate with a reasonable benefit/risk ratio, generally water or oil-soluble or dispersible, and effective for their intended use.

Where applicable and compatible with the chemical properties of the compound of the present invention, "pharmaceutically acceptable salt" includes pharmaceutically-acceptable acid addition salts and pharmaceutically-acceptable base addition salts. Lists of suitable salts are found in, e.g., S. M. Birge et al., J. Pharm. Sci., 1977, 66, pp. 1-19.

In cases where compounds are sufficiently basic or acidic to form stable nontoxic acid or base salts, administration of the compounds as salts may be appropriate. The term pharmaceutically acceptable salts or complexes refers to salts or complexes that retain the desired biological activity of the compounds of the present invention and exhibit minimal undesired toxicological effects.

Nonlimiting examples of such salts are (a) acid addition salts formed with inorganic acids such as sulfate, nitrate, bicarbonate, and carbonate salts (for example, hydrochloric acid, hydrobromic acid, sulfuric acid, phosphoric acid, nitric acid, and the like), and salts formed with organic acids including tosylate, methanesulfonate, acetate, citrate, malonate, tartarate, succinate, benzoate, ascorbate, α-ketoglutarate, and α-glycerophosphate salts, such as acetic acid, oxalic acid, tartaric acid, succinic acid, malic acid, ascorbic acid, benzoic acid, tannic acid, pamoic acid, alginic acid, polyglutamic acid, naphthalenesulfonic acid, naphthalenedisulfonic acid, and polygalcturonic acid; (b) base addition salts formed with metal cations such as zinc, calcium, bismuth, barium, magnesium, aluminum, copper, cobalt, nickel, cadmium, sodium, potassium, lithium and the like, or with a cation formed from ammonia, N,N-dibenzylethylenediamine, D-glucosamine, tetraethylammonium, or ethylenediamine; or (c) combinations of (a) and (b); e.g., a zinc tannate salt or the like. Also included in this definition are pharmaceutically acceptable quaternary salts known by those skilled in the art, which specifically include the quaternary ammonium salt of the formula - NR⁺A⁻, wherein R is as defined above and A is a counterion, including chloride, bromide, iodide, -O-alkyl, toluenesulfonate, methylsulfonate, sulfonate, phosphate, or carboxylate (such as benzoate, succinate, acetate, glycolate, maleate, malate, citrate, tartrate, ascorbate, benzoate, cinnamoate, mandeloate, benzyloate, and diphenylacetate).

Pharmaceutically acceptable salts may be obtained using standard procedures well known in the art, for example by reacting a sufficiently basic compound such as an amine with a suitable acid affording a physiologically acceptable anion.

"Prodrug", as used herein, refer to a compound that is metabolized, for example hydrolyzed or oxidized, in the host to form the active compound. Typical examples of prodrugs include compounds that have biologically labile protecting groups on a functional moiety of the active compound. Prodrugs include compounds that can be oxidized, reduced, aminated, deaminated, hydroxylated, dehydroxylated, hydrolyzed, dehydrolyzed, alkylated, dealkylated, acylated, deacylated, phosphorylated, dephosphorylated to produce the active compound.

The term "patient" includes human and veterinary subjects.

The term "biological sample", as used herein, includes, without limitation, cell cultures or extracts thereof; preparations of an enzyme suitable for *in vitro* assay; biopsied material obtained from a mammal or extracts thereof; and blood, saliva, urine, feces, semen, tears, or other body fluids or extracts thereof.

The term "cancer" includes, but is not limited to, solid tumors and blood borne tumors and include, but is not limited to, the following cancers: breast, ovary, cervix, prostate, testis, genitourinary tract, esophagus, larynx, glioblastoma, stomach, skin, keratoacanthoma, lung, epidermoid carcinoma, large cell carcinoma, small cell carcinoma, lung adenocarcinoma, bone, colon, adenoma, pancreas, adenocarcinoma, thyroid, follicular carcinoma, undifferentiated carcinoma, papillary carcinoma, seminoma, melanoma, sarcoma, bladder carcinoma, liver carcinoma and biliary passages, kidney carcinoma, myeloid disorders, lymphoid disorders, Hodgkin's, hairy cells, buccal cavity and pharynx (oral), lip, tongue, mouth, pharynx, small intestine, colon-rectum, large intestine, rectum, brain and central nervous system, and leukemia. The term "cancer" includes primary cancer, cancers secondary to treatment, and metastatic cancers.

The term "pharmaceutically acceptable carrier" refers to a non-toxic carrier, adjuvant, or vehicle that may be administered to a patient, together with a compound of this invention, and which does not destroy the pharmacological activity thereof.

The terms "GSK-3-mediated disease, or "GSK-3-mediated condition", as used herein, mean any disease or other deleterious condition or state in which GSK-3 is known to play a role. Such diseases or conditions include, without limitation, diabetes, Alzheimer's disease, Huntington's Disease, Parkinson's Disease, AIDS-associated dementia, amyotrophic lateral sclerosis (AML), multiple sclerosis (MS), schizophrenia, cardiomycete hypertrophy, reperfusion/ischemia, and baldness.

The terms "CDK-2-mediated disease" or CDK-2-mediated condition", as used herein, mean any disease or other deleterious condition in which CDK-2 is known to play a role. The terms "CDK-2-mediated disease" or "CDK-2-mediated condition" also mean those diseases or conditions that are alleviated by treatment with a CDK-2 inhibitor. Such conditions include, without limitation, cancer, Alzheimer's disease, restenosis, angiogenesis, glomerulonephritis, cytomegalovirus, HIV, herpes, psoriasis, atherosclerosis, alopecia, and autoimmune diseases such as rheumatoid arthritis, such as are described for example in Fischer, P. M. and Lane, D. P., Current Medicinal Chemistry, 7, 1213-1245 (2000); Mani, S., Wang, C., Wu, K., Francis, R. and Pestell, R., Exp. Opin. Invest. Drugs, 9, 1849 (2000); Fry, D. W. and Garrett, M. D., Current Opinion in Oncologic, Endocrine & Metabolic Investigational Drugs, 2, 40-59 (2000).

The terms "ERK-mediated disease" or "ERK-mediated condition", as used herein mean any disease or other deleterious condition in which ERK may play a role. The terms "ERK-2-mediated disease" or "ERK-2-mediated condition" also mean those diseases or conditions that are alleviated by treatment with a ERK-2 inhibitor. Such conditions include, without limitation, cancer, stroke, diabetes, hepatomegaly, cardiovascular disease including cardiomegaly, Alzheimer's disease, cystic fibrosis, viral disease, autoimmune diseases, atherosclerosis, restenosis, psoriasis, allergic disorders including asthma, inflammation, neurological disorders and hormone-related diseases. ERK-2 protein kinase and its implication in various diseases has been described for example in Bokemeyer et al. 1996, Kidney Int. 49, 1187; Anderson et al., 1990, Nature 343, 651; Crews et al., 1992, Science 258, 478; Bjorbaek et al., 1995, J. Biol. Chem. 270, 18848; Rouse et al., 1994, Cell 78, 1027; Raingeaud et al., 1996, Mol. Cell Biol. 16, 1247; Raingeaud et al. 1996; Chen et al., 1993 Proc. Natl. Acad. Sci. USA 90, 10952; Oliver et al., 1995, Proc. Soc. Exp. Biol. Med. 210, 162; Moodie et al., 1993, Science 260, 1658; Frey and Mulder, 1997, Cancer Res. 57, 628; Sivaraman et al., 1997, J Clin. Invest. 99, 1478; Whelchel et al., 1997, Am. J. Respir. Cell Mol. Biol. 16, 589.

The terms "AKT-mediated disease" or "AKT-mediated condition", as used herein, mean any disease or other deleterious condition in which AKT is known to play a role. The terms "AKT-mediated disease" or "AKT-mediated condition" also mean those diseases or conditions that are alleviated by treatment with a AKT inhibitor. AKT-mediated diseases or conditions include, but are not limited to, proliferative disorders, cancer, and neurodegenerative disorders. The association of AKT, also known as protein kinase B, with various diseases has been described for example in Khwaja, A., Nature, pp. 33-34, 1990; Zang, Q. Y., et al, Oncogene, 19 2000; Kazuhiko, N., et al, The Journal of Neuroscience, 20 2000.

The terms "Src-mediated disease" or "Src-mediated condition", as used herein mean any disease or other deleterious condition in which Src is known to play a role. The terms "Src-mediated disease" or "Src-mediated condition" also mean those diseases or conditions that are alleviated by treatment with a Src inhibitor. Such conditions include, without limitation, hypercalcemia, osteoporosis, osteoarthritis, cancer, symptomatic treatment of bone metastasis, and Paget's disease. Src protein kinase and its implication in various diseases has been described for example in Soriano, Cell, 69, 551 (1992); Soriano et al., Cell, 64, 693 (1991); Takayanagi, J. Clin. Invest., 104, 137 (1999); Boschelli, Drugs of the Future 2000, 25(7), 717, (2000); Talamonti, J. Clin. Invest., 91, 53 (1993); Lutz, Biochem. Biophys. Res. 243, 503 (1998); Rosen, J. Biol. Chem., 261, 13754 (1986); Bolen, Proc. Natl. Acad. Sci. USA, 84, 2251 (1987); Masaki, Hepatology, 27, 1257 (1998); Biscardi, Adv. Cancer Res., 76, 61 (1999); Lynch, Leukemia, 7, 1416 (1993); Wiener, Clin. Cancer Res., 5, 2164 (1999); Staley, Cell Growth Diff., 8, 269 (1997).

The terms "Lck-mediated disease" or "Lck-mediated condition", as used herein, mean any disease state or other deleterious condition in which Lck is known to play a role. The terms "Lck-mediated disease" or "Lck-mediated condition" also mean those diseases or conditions that are alleviated by treatment with an Lck inhibitor. Lck-mediated diseases or conditions include, but are not limited to, autoimmune diseases such as transplant rejection, allergies, rheumatoid arthritis, and leukemia. The association of Lck with various diseases has been described for example in Molina et al., Nature, 357, 161 (1992).

The terms "Abl-mediated disease" or "Abl-mediated condition", as used herein, mean any disease state or other deleterious condition in which Abl is known to play a role. The terms "Abl-mediated disease" or "Abl-mediated condition" also mean those diseases or conditions that are alleviated by treatment with an Abl inhibitor. Abl-mediated diseases or conditions include, but are not limited to, leukemias, particularly chronic myeloid leukemia. The association of Abl with various diseases has been described for example in Druker, et al., N. Engl. J. Med.2001, 344, 1038-1042.

The terms "cKit-mediated disease" or "cKit-mediated condition", as used herein, mean any disease state or other deleterious condition in which cKit is known to play a role. The terms "cKit-mediated disease" or "cKit-mediated condition" also mean those diseases or conditions that are alleviated by treatment with an cKit inhibitor. cKit-mediated diseases or conditions include, but are not limited to, mastocytosis/mast cell leukemia, gastrointestinal stromal tumor, sinonasal natural killer/T-cell lymphoma, seminoma/dysgerminoma, throid carcinoma, samll-cell lung carcinoma, malignant melanoma, adenoid cystic carcinoma, ovarian carcinoma, acute myelogenious leukemia, anaplastic large-cell lymphoma, angiosarcoma, endometrial carcinom, pediatric T-cell ALL/lymphoma, breast carcinoma and prostate carcinoma. The association of cKit with various diseases has been described for example in Heinrich, et al., J. Clinical Oncology 2002, 20, 1692-1703.

The terms "Flt3-mediated disease" or "Flt3-mediated condition", as used herein, mean any disease state or other deleterious condition in which Flt3 is known to play a role. The terms "Flt3-mediated disease" or "Flt3-mediated condition" also mean those diseases or conditions that are alleviated by treatment with an Flt3 inhibitor. Flt3-mediated diseases or conditions include, but are not limited to, acute myelogenous leukemia, mixed lineage leukemia and acute lymphocytic leukemia. The association of Flt3 with various diseases has been described for example in Sternberg and Licht, Curr. Opin Hematol. 2004, 12, 7-13.

The terms "KDR-mediated disease" or "KDR-mediated condition", as used herein, mean any disease state or other deleterious condition in which KDR is known to play a role. The terms "KDR-mediated disease" or "KDR-mediated condition" also mean those diseases or conditions that are alleviated by treatment with an KDR inhibitor. KDR-mediated diseases or conditions include, but are not limited to, carcinoma of the lung, breast, gastrointestinal tract, kidney, bladder, ovary and endometrium, intracranial tumors including glioblatoma multiforme, sporadic capillary hemangioblastoma, hematological malignancies, including T cell lymphoma, acute lymphoblastic leukemia, Burkitt's lymphoma and promyelocytic leukemia, age-related macular degeneration, herpetic ocular disease, rheumatoid arthritis, cerebral ischemia and endometriosis. The association of KDR with various diseases has been described for example in Ferrara, Endocrine Reviews 2004, 25, 581-611.

The term "HSP90-mediated disease" or "HSP90-mediated condition" refers to a condition in which HSP90 is known to pay a role. The conditions include but are not limited to inflammatory disorders, abnormal cellular proliferation, autoimmune disorders, ischemia, fibrogenetic disorders including but not limited to scleroderma, polymyositis, systemic lupus, rheumatoid arthritis, liver cirrhosis, keloid formation, interstitial nephritis, and pulmonary fibrosis. (Strehlow, WO 02/02123; PCT/US01/20578).

### Treatment

The compounds described herein, are particularly useful for the treatment or prevention of a disorder mediated by kinases or mediated by HSP90. In one embodiment, the compounds described herein, are useful for the treatment or prevention of a proliferative disorder, including cancer metastasis. In another embodiment, the compounds described herein, are useful for the treatment or prevention of an inflammatory disorder associated by kinases or HSP90.

Tumors in NF2 and NF1 patients are unique in that they are slow growing tumors. Both NF2 and NF1 tumors have mutations or loss of heterozygosity in tumor suppressor genes although the tumor suppressor genes are different- *NF2* or *NF1* genes, respectively. The inventors of the present invention have discovered that inhibitors of HSP90 potently blocked the proliferation of NF2-deficient and NF1-deficient tumor cells and also delay the growth of NF2-deficient and NF1-deficient tumors in mice. Merlin regulates the abundance and turnover of multiple cell surface receptors and interacts with multiple pathways. Many of these proteins are client proteins of HSP90. For instance, ErbB2 and other receptor tyrosine kinases, AKT, and Raf are well-established client proteins of HSP90. In addition, aberrant activation of the PI3K/AKT pathway has been found in human schwannomas from NF2 patients (as compared to normal nerves), in human NF2-deficient tumor xenografts (e.g. meningiomas and mesotheliomas), and in mouse Nf2-deficient Schwann cell tumors (reference is made to a PCT/US2007/70366 entitled "Treatment of Neurofibromatosis with Inhibitors of a Signal Transduction Pathway," filed on June 4, 2007, which is incorporated in its entirety by reference). Neurofibromin is a negative regulator of Ras. Raf, the direct downstream effector of Ras, is a well-known client protein of HSP90. Neurofibromin has also been shown to regulate AKT. Therefore, a compound that inhibits HSP90 will likely be able to reduce the amount of AKT and other HSP90 client proteins such as ErbB2, IGF-IR, and Raf in the NF2-deficient cells or NF1-deficient cells. Reduction of the amount or activity of these proteins may be useful to reduce or stabilize the proliferation of NF2-deficient cells or NF1-deficient cells or to cause apoptosis of NF2-deficient cells or NF1-deficient cells. A compound that inhibits the activity of HSP90 is a compound that directly binds to the HSP90 protein or modifies the HSP90 protein post-translationally or regulate the transcription of HSP proteins such as HSP70 and HSP27 (Zaarur et al., 2006, Cancer Res. 66(3):1783-1791). For instance, HSP90 can be acetylated and inactivated by histone deacetylase (HDAC) inhibitors (Kovacs et al., 2005, Mol. Cell 18(5):601-607; Fuino et al., 2003, Mol. Cancer Ther. 2:971-984; Aoyagi and Archer, 2005, Trends Cell. Biol. 15(11):565-567). For this reason, HSP90 inhibitory compounds are determined to be useful for the treatment of NF2-deficient tumors and NF1-deficient tumors which may not respond well to traditional chemotherapy and other cancer therapies which target fast growing and heterogeneous cancer cells.

### NF2- Associated Tumors

Patients with neurofibromatosis type-2 (NF2) have NF2-deficient tumors. This genetic characteristic, *i*.*e*., inactivation of the *NF2* gene, differentiates tumors found in *NF2* patients from genetically heterogeneous tumors such as breast and colon cancer tumors. For instance, NF2 patients have NF2-deficient meningiomas whereas some non-NF2-deficient meningiomas may contain mutations in many different oncogenes or tumor suppressor genes.

As used herein, "NF2-deficient tumors" refer to tumors which contain a non-functioning *NF2* gene. A non-functioning *NF2* gene can be the result of a one or more insertion or deletion mutations within the *NF2* gene, for instance, missense or nonsense mutations, mutations in the promoter or enhancer or introns that lead to no/low expression of the *NF2* gene, or the deletion of the entire *NF2* gene. NF2-deficient tumors are found in mesotheliomas and in patients with NF2, and include schwannomas, meningiomas, and other tumors associated with the nervous system. NF2-deficient tumors are also found in all patients with sporadic schwannomas and in 50%-70% of patients with meningiomas.

The presence of NF2-deficient bilateral vestibular schwannomas, *i*.*e*., Schwann cell tumors, is a hallmark of NF2. The compounds of the present invention can be used to inhibit the growth and/or kill NF2-deficient schwannoma cells, including those associated with vestibular schwannomas, spinal cord and other peripheral nerve schwannomas and sporadic schwannomas.

### Merlin and signaling proteins and pathways

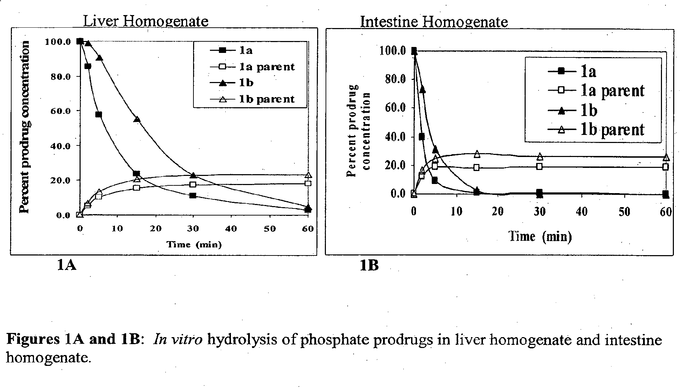

As used herein, "NF2-deficient tumors" refer to tumors which contain a non-functioning *NF2* gene. Merlin interacts with or regulates, but is not limited to, proteins and pathways such as Paxillin/Integrin-β1/ErbB2, EGFR, Patched/Smoothened, HRS, CD44, E-Cadherin, Fat, EBP50/NHE-RF/PDGFR, Wingless, Notch, Rac-PAK, PI3K-AKT, Ras-RafMek-Erk2, Hippo pathways, and downstream proteins thereof. Figure 1 is a schematic of involvement of Merlin with multiple cell surface proteins and signaling pathways. Targeting multiple proteins or pathways may be necessary for treating NF2.

### NF1-Associated Tumors

Patients with neurofibromatosis type-1 (NF1) have NF1-deficient tumors. This genetic characteristic, *i.e.,* inactivation of the *NF1* gene, differentiates tumors found in *NF1* patients from genetically heterogeneous tumors such as breast and colon cancer tumors. For instance, tumors found in NF1 patients all have *NF1* gene mutations whereas patients with other cancers have mutations in different genes or overexpression of different genes.

As used herein, "NF1-deficient tumors" refer to tumors which contain a non-functioning *NF1* gene. A non-functioning *NF1* gene can be the result of one or more insertion or deletion mutations within the *NF1* gene, for instance, missense or nonsense mutations, mutations in the promoter or enhancer or introns that lead to no/low expression of the *NF1* gene, or the deletion of the entire *NF1* gene. NF1-deficient tumors are found in patients with NF1, and include dermal, subdermal, plexiform neurofibromas, and MPNST and other tumors associated with the nervous system. NF1-deficiency also predisposes individuals to a rare form of leukemia, JMML.

NF1 diagnosis is confirmed by fulfilling NIH clinical criteria or by finding an *NF1* mutation with mutational analysis. The methods of the present invention can be used to inhibit the growth and/or kill NF1-deficient tumors, including dermal, subdermal, plexiform neurofibromas, MPNST, gliomas, astrocytomas, pheochromocytomas and JMML.

The present invention provides the use of the compounds of formula II, as HSP90 inhibitors for use in the treatment, prevention, or amelioration of tumors or symptoms resulted from neurofibromatosis. Also provided are compositions comprising the compounds and processes for the preparation of the compounds.

The present invention includes one or more compounds of formula II as described above for use in inhibiting the growth of NF2-deficient and/or NF1-deficient tumor cells by contacting, *i.e.,* treating, the NF2-deficient or NF1-deficient tumor cells with radicicol and its derivatives. The present invention also includes one or more compounds of formula II as described above for use in decreasing proliferation of NF2-deficient or NF1-deficient tumor cells by contacting the cells with radicicol and its derivatives.

In one embodiment, inhibition of the growth of NF2 or NF1 cells is determined by comparing a sample of NF2-deficient or NF1-deficient tumor cells treated with compounds of the present invention to a control, such as a sample of untreated NF2-deficient or NF1-deficient tumor cells or a sample of cells treated with a known inert compound. Prior to contacting cells with compounds of the present invention, both samples of NF2-deficient cells or NF1-deficient cells (treated and control) should consist of approximately the same number of cells and be of the same cell type, e.g., NF2-deficient schwannomas or NF1-deficient MPNST cells. NF2-deficient tumor cells or NF1-deficient tumor cells treated with radicicol and its derivatives may decrease in number by at least about 10%, at least about 20%, at least about 30%, at least about 40%, at least about 50%, at least about 60%, at least about 70%, at least about 80%, at least about 90%, or about 100% following compound treatment compared to the control.

In one embodiment of the invention, the pharmaceutical composition is applied directly to the site of a NF2-deficient tumor or NF1-deficient tumor. For instance, the HSP90 inhibitor can be applied by local treatment which encompasses both topical treatment and intralesional or intradermal treatment at the site of the tumor. Therefore, the inhibitor can be injected into, topically applied onto or near a NF2-deficient tumor or NF1-deficient tumor. In one embodiment of the invention, the inhibitor is applied intralesionally to NF2-deficient tumors or NF1-deficient tumors by methods known in the art.

The present invention includes the compounds for use in treating, preventing or ameliorating tumors or symptoms resulting from neurofibromatosis in a subject comprising administering to said subject with neurofibromatosis type 2 (NF2) or a condition associated with the loss of NF2 function or with neurofibromatosis type 1 (NF1) or a condition associated with the loss of NF1 function a therapeutically effective amount of at least one composition comprising at least one compound of the present invention, which inhibits or slows growth of one or more NF2-deficient tumors or NF1-deficient tumors, reduces the number of said tumors or inhibits and/or reduces associated symptoms as compared to no treatment with the composition as a control level to determine treatment utility. This is particularly directed to the administration of the at least one composition comprising a compound of the present invention resulting in a decrease in size and/or number of one or more NF2-deficient tumors or of one or more NF1-deficient tumors.

This comprises administering a compound of the present invention which inhibits or reduces the function of HSP90 complex. More specifically, the compound of the present invention binds and inhibits the HSP90 protein, modifies HSP90 protein posttranslationally, and/or increases HSP70 or other HSP proteins from their normal levels in the NF2-deficient or NF1-deficient tumors. The administration of a compound of the present invention results in the upregulation or increase in HSP70 in said subject. Further, this comprises administering a compound of the present invention that degrades or reduces one or more client proteins of HSP90 and the phosphorylated forms of the client proteins. Additionally, this comprises administering a compound of the present invention that inhibits or reduces activity or phosphorylation of signaling pathway proteins associated with one or more client proteins of HSP90. The one or more client proteins are selected from the group consisting of ErbB2, AKT, and Raf. The HSP90 inhibitor inhibits or reduces activity or phosphorylation of the signaling pathways proteins, such as PI3K, mTOR, GSK3, 4E-BP1, Bad, FKHR, HSP90, S6K, S6, Mek, and Erk1/2.

This treats, prevents or ameliorates tumors or symptoms resulting from neurofibromatosis type 2 (NF2) or condition(s) associated with the loss of NF2 function. More specifically the one or more NF2-deficient tumors comprise vestibular schwannomas, and more specifically comprise a unilateral vestibular schwannoma or a bilateral vestibular schwannoma. Additionally, the one or more NF2-deficient tumors that are treated comprise spinal cord schwannomas, sporadic schwannomas, peripheral nerve schwannomas, schwannoma, meningioma, mesothelioma, ependymoma, glioma and astrocytoma.

This comprises the administration of a compound of the present invention to obtain results in an improvement in at least one of the subject's hearing, balance and vision; increase in muscle mass, reduction in tumor burden in the subject, which the latter is identified using a MRI or a CAT scan.

This treats, prevents or ameliorates tumors or symptoms resulting from neurofibromatosis type 1 (NF1) or condition(s) associated with the loss of NF1 function. More specifically the one or more NF1-deficient tumors comprise a dermal and plexiform neurofibromas, optic pathway astrocytomas, optic neuromas, optic gliomas, cerebral astrocytomas, cerebral gliomas, ependymomas, pheochromocytomas and ganglioneuromas, rhabdomyosarcomas, neurofibrosarcomas, malignant peripheral nerve sheath tumors ("MPNST"), malignant schwannomas, and JMML.

The present invention also includes the use of the compounds for inhibiting or reducing the growth or number of NF2-deficient tumor cells or NF1-deficient tumor cells comprising contacting said NF2-deficient tumor cells or NF1-deficient tumor cells with at least one composition comprising at least one compound of the present invention which inhibits or slows growth and/or reduces the number of one or more NF2-deficient tumors or NF1-deficient tumors. This method comprises contacting said NF2-deficient tumor cells or NF1-deficient tumor cells with said compound occurs *in vitro* or *ex vivo.* The NF2-deficient tumor cells are Nf2-deficient mouse Schwann cells and said NF1-deficient tumor cells are Nf1-deficient mouse Schwann cells. Or the NF2-deficient tumor cells are NF2-deficient human schwannoma cells and said NF1-deficient tumor cells are NF1-deficient human Schwann cells. The NF2-deficient tumor cells are selected from the group consisting of NF2-deficient schwannoma cell line cells, NF2-deficient meningioma cell line cells and NF2-deficient mesothelioma cell line cells. The NF2-deficient tumor cells are selected from the group consisting of HEI193 cells, SF1335 cells, BAR cells and RAV cells. The NF1-deficient tumor cells are selected from the group consisting of human MPNST cells, primary neurofibroma cells derived from NF1 patients, mouse *Nƒ1;p53*-deficient MPNST cell lines established from *cisNƒ1;p53* mice, and *Nƒ1-*/*-* mouse cells, such as Schwann cells, mouse embryonic cells, and leukemia cells. More specifically, the NF1-deficient tumor cells are selected from the group consisting of ST88-14, 88-3, 90-8, and sNF96.2. The NF2-deficient tumor cells or NF1-deficient tumor cells that are contacted with said HSP90 inhibitor can occur *in vivo.* The NF2-deficient tumor cells or said NF 1-deficient tumor cells are from a human, canine, rat or mouse.

This comprises contacting the NF2-deficient tumor cells or the NF1-deficient tumor cells with a compound of the present invention that results in an inhibition of HSP90 function. This further comprises contacting the NF2-deficient tumor cells or said NF1-deficient tumor cells with a compound of the present invention that results in an upregulation of HSP70. Further the contact of the NF2-deficient tumor cells or the NF1-deficient tumor cells with a compound of the present invention results in degradation of ErbB2 and/or phosphorylated ErbB2, in degradation of Akt and/or phosphorylated Akt or in degradation of Raf and/or phosphorylated Raf. More specifically, the contact of the NF2-deficient tumor cells or said NF1-deficient tumor cells with a compound of the present invention results in a reduction in phosphorylation of proteins downstream of the ErbB2, Akt or Raf signaling pathway. The degradation or upregulation of the proteins or reduction in phosphorylated proteins is detected using an antibody.

In another embodiment, the present invention includes a method for screening a test compound for treatment of NF-2 or NF-1 comprising treating or contacting NF-2-deficient cells or NF-1-deficient cells with said test compound, wherein a degradation of one or more client proteins of HSP90 or a decrease in activity of signaling pathways associated with one or more client proteins of HSP90 or an increase in HSP70 is indicative of an efficacious treatment of NF-2 or NF-1. The method further comprises assessing inhibition of HSP90 function. The inhibition of HSP90 function results in an upregulation of HSP70. The one or more client proteins of HSP90 are selected from the group consisting of ErbB2, AKT, and Raf. Additionally, the signaling pathways are associated with one or more client proteins of HSP90 are ErbB2 pathway, AKT pathway or Raf pathway which contain at least one protein selected from group consisting of ErbB2, AKT, Raf, mTOR, GSK3, 4E-BP1, Bad, FKHR, S6K, S6, Mek, and Erk1/2. Specifically, the one or more client proteins is AKT, the AKT is degraded by said test compound, resulting in reduced phosphorylation of AKT. The treatment results in reduced phosphorylation of S6, GSK3, FKHR, Mek, or Erk1/2. The reduced phosphorylation is detected using an antibody.

The method of the present invention further comprises measuring NF-2-deficient cells or NF-1-deficient cells following treatment with the test compound, wherein a decrease in the number of NF2-deficient cells or NF-1-deficient cells following treatment with the test compound or a decrease in proliferation of NF2-deficient cells or NF-1-deficient cells following treatment with the test compound is indicative of an efficacious treatment. Additionally, the method further comprises comparing the NF2-deficient cells or NF-1-deficient cells following treatment to untreated NF2 deficient cells or NF-1-deficient cells, wherein a decrease in one or more client proteins of the HSP90 following treatment with the test compound compared to untreated NF2-deficient cells or NF-1-deficient cells is indicative of an efficacious treatment. Additionally, the method further comprising comparing the NF2-deficient cells or NF-1-deficient cells following treatment to untreated NF2 deficient cells or NF-1-deficient cells, wherein a decrease in number of NF2 deficient cells or NF-1-deficient cells following treatment with the test compound or a decrease in proliferation of NF2-deficient cells or NF-1-deficient cells following treatment with the test compound compared to untreated NF2-deficient cells or NF-1-deficient cells is indicative of an efficacious treatment. The treatment of the NF2-deficient cells or NF1-deficient cells with said test compound occurs *in vitro* or *ex vivo.* The NF2-deficient tumor cells are Nf2-deficient mouse Schwann cells and the NF1-deficient tumor cells are Nf1-deficient mouse Schwann cells. Also the NF2-deficient tumor cells are NF2-deficient human schwannoma cells and said NF1-deficient tumor cells are NF1-deficient human Schwann cells.

The NF2-deficient tumor cells are selected from the group consisting of NF2-deficient schwannoma cell line cells, NF2-deficient meningioma cell line cells and NF2-deficient mesothelioma cell line cells. The NF2-deficient tumor cells are selected from the group consisting of HEI193 cells, SF1335 cells, BAR cells and RAV cells. The NF1-deficient tumor cells are selected from the group consisting of human MPNST cells, primary neurofibroma cells derived from NF1 patients, mouse *Nƒ1;p53*-deficient MPNST cell lines established from *cisNƒ1;p53* mice, and *Nƒ1-l-* mouse cells, such as Schwann cells, mouse embryonic cells, and leukemia cells. More specifically, the NF1-deficient tumor cells are selected from the group consisting of ST88-14, 88-3, 90-8, and sNF96.2. The NF2-deficient tumor cells or NF1-deficient tumor cells that are contacted with said HSP90 inhibitor can occur *in vivo.* The NF2-deficient tumor cells or said NF1-deficient tumor cells are from a human, canine, rat or mouse.

An aspect of the invention relates to compounds and compositions that are useful for treating cancer.

Another aspect of the invention relates to the treatment of the following cancers: breast, ovary, cervix, prostate, testis, genitourinary tract, esophagus, larynx, glioblastoma, stomach, skin, keratoacanthoma, lung, epidermoid carcinoma, large cell carcinoma, small cell carcinoma, lung adenocarcinoma, bone, colon, adenoma, pancreas, adenocarcinoma, thyroid, follicular carcinoma, undifferentiated carcinoma, papillary carcinoma, seminoma, melanoma, sarcoma, bladder carcinoma, liver carcinoma and biliary passages, kidney carcinoma, myeloid disorders, lymphoid disorders, Hodgkin's, hairy cells, buccal cavity and pharynx (oral), lip, tongue, mouth, pharynx, small intestine, colon-rectum, large intestine, rectum, brain and central nervous system, and leukemia.

Another aspect of the invention is an effective amount of a compound of formula II described herein for use in a method for treating cancer.

Angiogenesis is characterized by the proliferation of endothelial cells to form new blood vessels (often called neovascularization). Inhibition of mitosis of endothelial cells results in inhibition of angiogenesis. Another aspect of this invention therefore relates to inhibition of undesirable mitosis, including undesirable angiogenesis. A mammalian disease characterized by undesirable cell mitosis, as defined herein, includes, but is not limited to, excessive or abnormal stimulation of endothelial cells (e.g., atherosclerosis), solid tumors and tumor metastasis, benign tumors, for example, hemangiomas, acoustic neuromas, trachomas, and pyogenic granulomas, vascular malfunctions, abnormal wound healing, inflammatory and immune disorders, Bechet's disease, gout or gouty arthritis, abnormal angiogenesis accompanying rheumatoid arthritis, skin diseases, such as psoriasis, diabetic retinopathy and other ocular angiogenic diseases such as retinopathy of prematurity (retrolental fibroplasic), macular degeneration, corneal graft rejection, neovascular glaucoma and Osier Weber syndrome (Osler-Weber-Rendu disease).

Other undesired angiogenesis involves normal processes including ovulation and implantation of a blastula. The compositions described above can be used as a birth control agent by reducing or preventing uterine vascularization required for embryo implantation. Accordingly, the compositions described above can be used to block ovulation and implantation of a blastula or to block menstruation (induce amenorrhea).

Diseases associated with undesirable mitosis including neovascularization can be treated according to the present invention. Such diseases include, but are not limited to, ocular neovascular disease, diabetic retinopathy, retinopathy of prematurity, corneal graft rejection, neovascular glaucoma and retrolental fibroplasias, epidemic keratoconjunctivitis, Vitamin A deficiency, contact lens overwear, atopic keratitis, superior limbic keratitis, pterygium keratitis sicca, Sjögren's syndrome, acne rosacea, phylectenulosis, syphilis, *Mycobacteria* infections, lipid degeneration, chemical bums, bacterial ulcers, fungal ulcers, *Herpes simplex* infections, *Herpes zoster* infections, protozoan infections, Kaposi's sarcoma, Mooren's ulcer, Terrien's marginal degeneration, marginal keratolysis, trauma, rheumatoid arthritis, systemic lupus, polyarteritis, Wegener's sarcoidosis, Scleritis, Steven-Johnson disease, pemphigoid, radial keratotomy, and corneal graph rejection.

Other diseases associated with undesirable mitosis including neovascularization can be treated according to the present invention. Such diseases include, but are not limited to, sickle cell anemia, sarcoid, pseudoxanthoma elasticum, Paget's disease, vein occlusion, artery occlusion, carotid obstructive disease, chronic uveitis/vitritis, Lyme's disease, systemic lupus erythematosis, Eales' disease, Bechet's disease, infections causing a retinitis or choroiditis, presumed ocular histoplasmosis, Best's disease, myopia, optic pits, Stargart's disease, pars planitis, chronic retinal detachment, hyperviscosity syndromes, toxoplasmosis, and post-laser complications. Other diseases include, but are not limited to, diseases associated with rubeosis (neovascularization of the iris and the angle) and diseases caused by the abnormal proliferation of fibrovascular or fibrous tissue including all forms of proliferative vitreoretinopathy, whether or not associated with diabetes.

Another aspect of the invention relates to the treatment of inflammatory diseases including, but no limited to, excessive or abnormal stimulation of endothelial cells (*e.g.,* atherosclerosis), solid tumors and tumor metastasis, benign tumors, for example, hemangiomas, acoustic neuromas, trachomas, and pyogenic granulomas, vascular malfunctions, abnormal wound healing, inflammatory and immune disorders, Bechet's disease, gout or gouty arthritis, abnormal angiogenesis accompanying rheumatoid arthritis, skin diseases, such as psoriasis, diabetic retinopathy and other ocular angiogenic diseases such as retinopathy of prematurity (retrolental fibroplasic), macular degeneration, corneal graft rejection, neovascular glaucoma and Osler Weber syndrome (Osler-Weber-Rendu disease). Other undesired angiogenesis involves normal processes including ovulation and implantation of a blastula. Accordingly, the compositions described above can be used to block ovulation and implantation of a blastula or to block menstruation (induce amenorrhea).

Another aspect relates to inhibition of HSP90 activity in a patient, comprising administering to a patient an effective amount of a compound of formula II or a pharmaceutically acceptable salt or prodrug thereof. The invention also provides the compounds for use in treating a disease that is mediated by HSP90.

Another aspect relates to inhibiting Aurora A activity in a patient, comprising administering to a patient an effective amount of a compound of formula II or a pharmaceutically acceptable salt or prodrug thereof.

Another aspect of this invention relates to treating or preventing a GSK-3-mediated disease with a GSK-3 inhibitor, comprising administering to a patient an effective amount of a compound of formula II or a pharmaceutically acceptable salt or prodrug thereof.

One aspect relates to enhancing glycogen synthesis and/or lowering blood levels of glucose in a patient in need thereof, which method comprises administering to the patient a therapeutically effective amount of a compound of formula II or a pharmaceutical composition thereof. This is especially useful for diabetic patients. Another aspect relates to inhibiting the production of hyperphosphorylated Tau protein, which is useful in halting or slowing the progression of Alzheimer's disease. Another aspect relates to inhibiting the phosphorylation of .beta.-catenin, which is useful for treating schizophrenia.

Another aspect of the invention relates to inhibiting GSK-3 activity in a biological sample, which method comprises contacting the biological sample with a GSK-3 inhibitor of formula II.

Another aspect of this invention relates to a compound of formula II or a composition comprising said compound for use in inhibiting GSK-3 activity in a patient.

Another aspect of this invention relates to a therapeutically effective amount of a compound of formula II or a pharmaceutical composition thereof for use in treating or preventing a CDK-2-mediated disease.

Another aspect of the invention relates to inhibiting CDK-2 activity in a biological sample or a patient, which comprises administering to the patient a compound of formula II, or a composition comprising said compound.

Another aspect of this invention relates to a therapeutically effective amount of a compound of formula II or a pharmaceutical composition thereof for use in treating or preventing an ERK-2-mediated disease.

Another aspect of the invention relates to inhibiting ERK-2 activity in a biological sample or a patient, which comprises administering to the patient a compound of formula II, or a composition comprising said compound.

Another aspect of this invention relates to a therapeutically effective amount of a compound of formula II or a pharmaceutical composition thereof for use in treating or preventing an AKT-mediated disease.

Another aspect of the invention relates to inhibiting AKT activity in a biological sample or a patient, which comprises administering to the patient a compound of formula II, or a composition comprising said compound.

Another aspect of this invention relates to a therapeutically effective amount of a compound of formula II or a pharmaceutical composition thereof for use in treating or preventing a Src-mediated disease.

Another aspect of the invention relates to inhibiting Src activity in a biological sample or a patient, which comprises administering to the patient a compound of formula II, or a composition comprising said compound.

Another aspect of this invention relates to a therapeutically effective amount of a compound of formula II, or a pharmaceutical composition thereof for use in treating or preventing an Lck-mediated disease with an Lck inhibitor.

Another aspect of the invention relates to inhibiting Lck activity in a biological sample or a patient, which comprises administering to the patient a compound of formula II, or a composition comprising said compound.

Another aspect of this invention relates to a therapeutically effective amount of a compound of formula II, or a pharmaceutical composition thereof for use in treating or preventing an Abl-mediated disease with an Abl inhibitor.

Another aspect of the invention relates to inhibiting Abl activity in a biological sample or a patient, which comprises administering to the patient a compound of formula II, or a composition comprising said compound.

Another aspect of this invention relates to a therapeutically effective amount of a compound of formula II, or a pharmaceutical composition thereof for use in treating or preventing a cKit-mediated disease.

Another aspect of the invention relates to inhibiting cKit activity in a biological sample or a patient, which comprises administering to the patient a compound of formula II, or a composition comprising said compound.

Another aspect of this invention relates to a therapeutically effective amount of a compound of formula II, or a pharmaceutical composition thereof for use in treating or preventing a Flt3-mediated disease.

Another aspect of the invention relates to inhibiting Flt3 activity in a biological sample or a patient, which comprises administering to the patient a compound of formula II, or a composition comprising said compound.

Another aspect of this invention relates to a therapeutically effective amount of a compound of formula II, or a pharmaceutical composition thereof for use in treating or preventing a KDR-mediated disease.

Another aspect of the invention relates to inhibiting KDR activity in a biological sample or a patient, which comprises administering to the patient a compound of formula II, or a composition comprising said compound.

An amount effective to inhibit protein kinase, is an amount that causes measurable inhibition of the kinase activity when compared to the activity of the enzyme in the absence of an inhibitor. Any method may be used to determine inhibition, such as, for example, the Biological Testing Examples described below.

### Pharmaceutical Compositions

Mammals, and specifically humans, suffering from a respiratory disorder can be treated by the inhalation, systemic, oral, topical, or transdermal administration of a composition comprising an effective amount of the compounds described herein or a pharmaceutically acceptable salt, ester or prodrug thereof, optionally in a pharmaceutically acceptable carrier or diluent.

The compounds or compositions are typically administered by oral or inhalation administration. Alternatively, compounds can be administered subcutaneously, intravenously, intraperitoneally, intramuscularly, parenterally, orally, submucosally, by inhalation, transdermally via a slow release patch, or topically, in an effective dosage range to treat the target condition.

An effective dose can be readily determined by the use of conventional techniques and by observing results obtained under analogous circumstances. In determining the effective dose, a number of factors are considered including, but not limited to: the species of patient; its size, age, and general health; the specific disease involved; the degree of involvement or the severity of the disease; the response of the individual patient; the particular compound administered; the mode of administration; the bioavailability characteristics of the preparation administered; the dose regimen selected; and the use of concomitant medication.

In a separate embodiment, the compounds of the invention are in the form of an inhaled dosage. In this embodiment, the compounds may be in the form of an aerosol suspension, a dry powder or liquid particle form. The compounds may be prepared for delivery as a nasal spray or in an inhaler, such as a metered dose inhaler. Pressurized metered-dose inhalers ("MDI") generally deliver aerosolized particles suspended in chlorofluorocarbon propellants such as CFC-11, CFC-12, or the non-chlorofluorocarbons or alternate propellants such as the fluorocarbons, HFC-134A or HFC-227 with or without surfactants and suitable bridging agents. Dry-powder inhalers can also be used, either breath activated or delivered by air or gas pressure such as the dry-powder inhaler disclosed in the Schering Corporation International Patent Application No. PCT/US92/05225, published 7 Jan. 1993 as well as the Turbuhaler™ (available from Astra Pharmaceutical Products, Inc.) or the Rotahaler™ (available from Allen & Hanburys) which may be used, to deliver the aerosolized particles as a finely milled powder in large aggregates either alone or in combination with some pharmaceutically acceptable carrier e.g. lactose; and nebulizers.

The compounds of the invention may be also administered in specific, measured amounts in the form of an aqueous suspension by use of a pump spray bottle. The aqueous suspension compositions of the present invention may be prepared by admixing the compounds with water and other pharmaceutically acceptable excipients. The aqueous suspension compositions according to the present invention may contain, inter alia, water, auxiliaries and/or one or more of the excipients, such as: suspending agents, e.g., microcrystalline cellulose, sodium carboxymethylcellulose, hydroxpropyl-methyl cellulose; humectants, e.g. glycerin and propylene glycol; acids, bases or buffer substances for adjusting the pH, e.g., citric acid, sodium citrate, phosphoric acid, sodium phospate as well as mixtures of citrate and phosphate buffers; surfactants, e.g. Polysorbate 80; and antimicrobial preservatives, e.g., benzalkonium chloride, phenylethyl alcohol and potassium sorbate.

Typical systemic dosages for all of the herein described conditions are those ranging from 0.01 mg/kg to 1500 mg/kg of body weight per day as a single daily dose or divided daily doses. Preferred dosages for the described conditions range from 0.5-1500 mg per day. A more particularly preferred dosage for the desired conditions ranges from 5-750 mg per day. Typical dosages can also range from 0.01 to 1500, 0.02 to 1000, 0.2 to 500, 0.02 to 200, 0.05 to 100, 0.05 to 50, 0.075 to 50, 0.1 to 50, 0.5 to 50, 1 to 50, 2 to 50, 5 to 50, 10 to 50, 25 to 50, 25 to 75, 25 to 100, 100 to 150, or 150 or more mg/kg/day, as a single daily dose or divided daily doses. In one embodiment, the compounds are given in doses of between about 1 to about 5, about 5 to about 10, about 10 to about 25 or about 25 to about 50 mg/kg. Typical dosages for topical application are those ranging from 0.001 to 100% by weight of the active compound.

The compounds are conveniently administered in units of any suitable dosage form, including but not limited to one containing from about 7 to 3000 mg, from about 70 to 1400 mg, or from about 25 to 1000 mg of active ingredient per unit dosage form. For example, an oral dosage of from about 50 to 1000 mg is usually convenient, including in one or multiple dosage forms of 50, 100, 200, 250, 300, 400, 500, 600, 700, 800, 900 or 1000 mgs. Lower dosages may be preferable, for example, from about 10-100 or 1-50 mgs. Also contemplated are doses of 0.1-50 mg, 0.1-20 mgs., or 0.1-10 mgs. Furthermore, lower doses may be utilized in the case of administration by a non-oral route, as for example, by injection or inhalation.

The compound is administered for a sufficient time period to alleviate the undesired symptoms and the clinical signs associated with the condition being treated.

The active compound is included in the pharmaceutically acceptable carrier or diluent in an amount sufficient to deliver to a patient a therapeutic amount of compound *in vivo* in the absence of serious toxic effects. Pharmaceutically acceptable carriers that may be used in these pharmaceutical compositions are generally known in the art. They include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, solvents, salts or electrolytes, such as protamine sulfate, disodium hydrogen phosphate, potassium hydrogen phosphate, sodium chloride, zinc salts, silicates, colloidal silica, magnesium trisilicate, polyvinyl pyrrolidone, cellulose-based substances, polyethylene glycol, sodium carboxymethylcellulose, polyacrylates, waxes, oils, polyethylene-polyoxypropylene-block polymers, polyethylene glycol and wool fat. Pharmaceutically accepted vehicles can contain mixtures of more than one excipient in which the components and the ratios can be selected to optimize desired characteristics of the formulation including but not limited to shelf-life, stability, drug load, site of delivery, dissolution rate, self-emulsification, control of release rate and site of release, and metabolism.

Formulations can be prepared by a variety of techniques known in the art. Examples of formulation techniques can be found in literature publications and in texts such as "Water-insoluble drug formulation", edited by Rong Liu, 2000, Interpharm Press.

If administered intravenously, carriers can be physiological saline, bacteriostatic water, Cremophor EL™ (BASF, Parsippany, NJ) or phosphate buffered saline (PBS). Sterile injectable forms of the compositions of this invention may be aqueous or oleaginous suspension. These suspensions may be formulated according to techniques known in the art using suitable dispersing or wetting agents and suspending agents. The sterile injectable preparation may also be a sterile injectable solution or suspension in a non-toxic parenterally-acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose, any bland fixed oil may be employed including synthetic mono-or di-glycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a long-chain alcohol diluent or dispersant, such as carboxymethyl cellulose or similar dispersing agents which are commonly used in the formulation of pharmaceutically acceptable dosage forms including emulsions and suspensions. Other commonly used surfactants, such as Tweens, Spans and other surface-active emulsifying agents or bioavailability enhancers which are commonly used in the manufacture of pharmaceutically acceptable solid, liquid, or other dosage forms may also be used for the purposes of formulation.

The concentration of active compound in the drug composition will depend on absorption, inactivation, and excretion rates of the drug as well as other factors known to those of skill in the art. It is to be noted that dosage values will also vary with the severity of the condition to be alleviated. It is to be further understood that for any particular subject, specific dosage regimens should be adjusted over time according to the individual need and the professional judgment of the person administering or supervising the administration of the compositions, and that the dosage ranges set forth herein are exemplary only and are not intended to limit the scope or practice of the claimed composition. The active ingredient may be administered at once, or may be divided into a number of smaller doses to be administered at varying intervals of time.

One mode of administration of the active compound for systemic delivery is oral. Oral compositions will generally include an inert diluent or an edible carrier. They may be enclosed in gelatin capsules or compressed into tablets. For the purpose of oral therapeutic administration, the active compound can be incorporated with excipients and used in the form of tablets, troches, or capsules. Pharmaceutically compatible binding agents, and/or adjuvant materials can be included as part of the composition.

The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

When the dosage unit form is a capsule, it can contain, in addition to material of the above type, a liquid carrier such as a fatty oil. In addition, dosage unit forms can contain various other materials which modify the physical form of the dosage unit, for example, coatings of sugar, shellac, or other enteric agents.

The compound or its salts can be administered as a component of an elixir, suspension, syrup, wafer, chewing gum or the like. A syrup may contain, in addition to the active compounds, sucrose as a sweetening agent and certain preservatives, dyes and colorings and flavors.

In a preferred embodiment, the active compounds are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from Alza Corporation and Nova Pharmaceuticals, Inc. Liposomal suspensions (including liposomes targeted to infected cells with monoclonal antibodies to viral antigens) are also preferred as pharmaceutically acceptable carriers. These may be prepared according to methods known to those skilled in the art, for example, as described in U.S. Patent No. 4,522,811 (which is incorporated herein by reference in its entirety). For example, liposome formulations may be prepared by dissolving appropriate lipid(s) (such as stearoyl phosphatidyl ethanolamine, stearoyl phosphatidyl choline, arachadoyl phosphatidyl choline, and cholesterol) in an inorganic solvent that is then evaporated, leaving behind a thin film of dried lipid on the surface of the container. An aqueous solution of the compound is then introduced into the container. The container is then swirled by hand to free lipid material from the sides of the container and to disperse lipid aggregates, thereby forming the liposomal suspension.

Suitable vehicles or carriers for topical application can be prepared by conventional techniques, such as lotions, suspensions, ointments, creams, gels, tinctures, sprays, powders, pastes, slow-release transdermal patches, suppositories for application to rectal, vaginal, nasal or oral mucosa. In addition to the other materials listed above for systemic administration, thickening agents, emollients, and stabilizers can be used to prepare topical compositions. Examples of thickening agents include petrolatum, beeswax, xanthan gum, or polyethylene, humectants such as sorbitol, emollients such as mineral oil, lanolin and its derivatives, or squalene.

### Combination Treatment

The compound can also be mixed with other active materials which do not impair the desired action, or with materials that supplement the desired action. The active compounds can be administered in conjunction, i.e. combination or alternation, with other medications used in the treatment of respiratory disorders.

The compounds can be administered in combination or alternation with drugs typically useful for treatment or prevention of asthma, such as certain anti-inflammatory drugs and bronchodilators. Corticosteroids (inhaled and oral), mast cell stabilizers, and the leukotriene modifier drugs are typically a useful anti-inflammatory medication for people suffering from asthma. These drugs reduce swelling and mucus production in the airways. Bronchodilators typically relieve the symptoms of asthma by relaxing the muscle bands that tighten around the airways, This action rapidly opens the airways, letting more air come in and out of the lungs. Bronchodilators also help clear mucus from the lungs.

Typically used compounds include Inhaled corticosteroids, which prevent rather than relieve symptoms. Inhaled corticosteroids include: Advair (a combination medication that includes a corticosteroid (fluticasone) plus a long acting bronchodilator drug (in this case a β-2 adrenergic receptor agonist, salmeterol)), aerobid (flunisolide), azmacort (triamcinolone), flovent (fluticasone), methylprednisolone, prednisone, pulmicort or serevent diskus (salmeterol powder), theophylline, qvar, and xopenex (levalbuterol), Inhaled corticosteroids come in three forms: the metered dose inhaler (MDI), dry powder inhaler (DPI) and nebulizer solutions. Systemic steroids include: methylprednisolone (Medrol, Methylpred, Solu-Medrol), prednisone (Deltasone) and prednisolone (Prelone, Pediapred, Orapred). Mast Cell Stabilizers include Intal and Tilade, which work by preventing the release of irritating and inflammatory substances from mast cells. Leukotriene modifiers include accolate and singular and accolate (zafirlukast), singulair (montelukast) and zyflo (zileuton).

The compounds can be administered in combination with nonsteroidal antiinflammatories such as ibuprofen, indomethacin, fenoprofen, mefenamic acid, flufenamic acid, sulindac. The compound can also be administered with corticosteriods.Any of the compounds described herein for combination or alternation therapy can be administered as any prodrug that upon administration to the recipient, is capable of providing directly or indirectly, the parent compound. Nonlimiting examples are the pharmaceutically acceptable salts (alternatively referred to as "physiologically acceptable salts"), and a compound which has been alkylated or acylated at an appropriate position. The modifications can affect the biological activity of the compound, in some cases increasing the activity over the parent compound.

### Processes for the Preparation of the Compounds

Modular synthetic processes can be used for preparing macrocyclic compounds in non-prodrug forms, such as the active form or the active form with protecting groups. The methods and procedures for preparing macrocyclic compounds in non-prodrug forms are described in detail in the International Application PCT/US2007/017754 and US Serial No. 11/891,652, both filed on August 10, 2007 and entitled "Macrocyclic Compounds Useful as Inhibitors of Kinase and HSP90", the content of which is herein incorporated by reference in its entirety. Various prodrugs can be synthesized from the active macrocyclic compounds through the methods known to one skilled in the art for making prodrugs. For example, the phosphate ester of a hydroxyl group can be prepared via reacting the hydroxyl group with a phosphorylating agent, such as phosphoryl chloride analogs. Sodium salts of all phosphonates may be otained by treatment of the final compound with basic ion resin such as Dowex 550^{a}.

Schemes 1, 2, and 3 below illustrate the general synthetic strategy for the synthesis of bis-phosphate prodrug, mono-phosphate prodrug wherein the phosphate is para to the carbonyl, and mono-phosphate prodrug wherein the phosphate is ortho to the carbonyl.

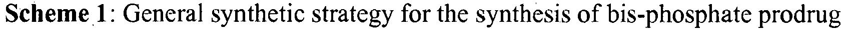

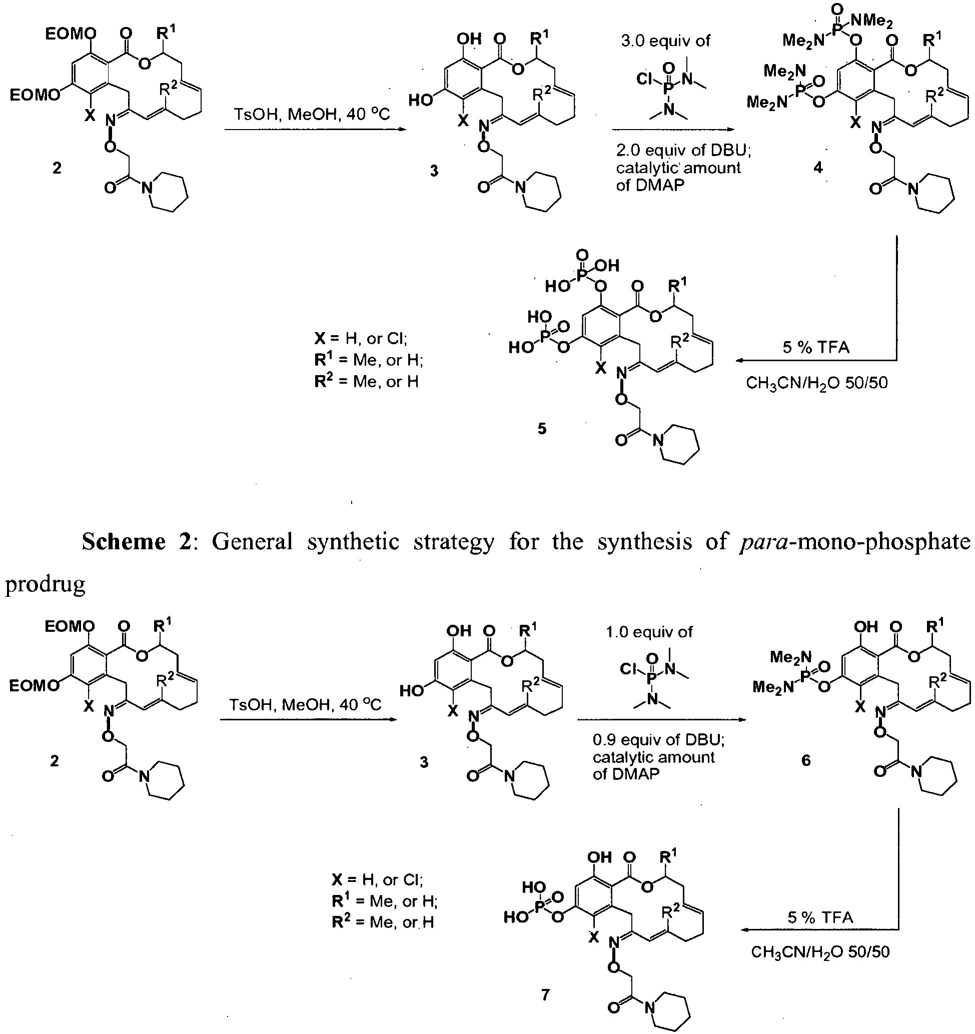

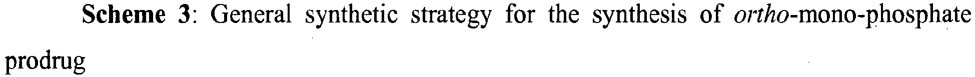

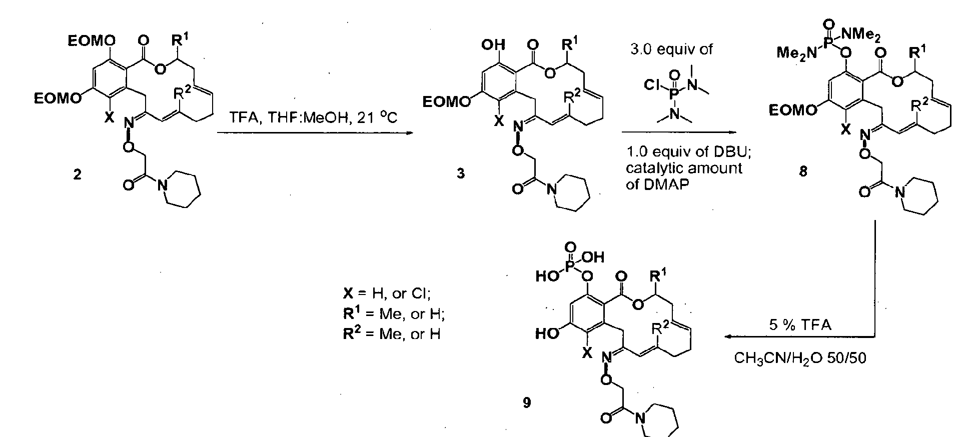

The following abbreviations are used herein.
- Ac: Acetyl (CH3C=O)
- ADP: Adenosine diphosphate
- AIBN: Azobis(isobutyronitrile)
- All: Allyl
- ATP: Adenosine triphosphate
- BER: Borohydride exchange resin
- BBN: Borabicyclononane
- Bn: Benzyl
- Bz: Benzoyl
- CAN: Ceric ammonium nitrate
- CSA: Camphorsulfonic acid
- δ: Chemical shift (NMR)
- dba: Dibenzylideneacetone
- DBU: 1,8-Diazabicyclo[5.4.0]undec-7-ene
- DDQ: 2,3-Dichloro-5,6-dicyano-1,4-benzoquinone
- DEAD: Diethyl azodicarboxylate
- DIAD: Diisopropyl azodicarboxylate
- d.e.: Diastereoisomeric excess
- DET: Diethyl tartrate
- DHP: Dihydropyran
- DIBAL or Dibal-H: Diisobutylaluminum hydride
- DIC: N,N'-diisopropylcarbodiimide
- DMAP: 4-Dimethylaminopyridine
- DMDO: Dimethyldioxirane
- DMF: Dimethylformamide
- DMPI: Dess-Martin periodinane
- DMSO: Dimethylsulfoxide
- DNA: Desoxyribo nucleic acid
- dppe: 1,2-Bis(diphenylphosphino)ethane
- EC₅₀: Plasma concentration required for obtaining 50% of maximum effect *in vivo*
- EDC: 1-Ethyl-3-[3-dimethylaminopropyl]carbodiimide hydrochloride
- EDTA: Ethylenediaminetetraacetic acid
- e.e.: Enantiomeric excess
- EOM: Ethoxymethyl (CH₃CH₂OCH₂)-
- FDA: Food and Drug Administration
- Fmoc: 9-Fluorenylmethoxycarbonyl
- GI₅₀: Concentration required for 50% inhibition of cell growth
- Grubbs' II: Grubbs' second generation catalyst: (ruthenium[1,3-bis(2,4,6-trimethylphenyl)-2-imidazolinylidene)dichloro(phenylmethylene) (tricyclohexylphosphane)

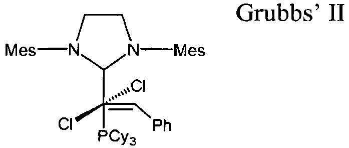

- HFIP: Hexafluoroisopropanol
- HMDS: Hexamethyldisilazide
- HMPA: Hexamethylphosphorictriamide
- HOBT: *N*-Hydroxybenzotriazole
- HPLC: High performance chromatography
- HRMS: High resolution mass spectrometry
- HSP90: Heat shock protein 90
- Hunig's Base: Diisopropylethylamine
- IC₅₀: Concentration of a drug that is required for 50% inhibition *in vitro*
- imid.: Imidazole
- Ipc₂BH: Bis-isopinocamphorylborane
- *J*: Coupling constant
- KHMDS: Potassium hexamethyldisilylamide
- L.C.: Liquid chromatography
- LDA: Lithium diisopropylamide
- LiHMDS: Lithium hexamethyldisilazide (LiN(SiMe₃)₂)
- µM: Micromolar concentration (µmol.l⁻¹)
- MAP: Mitogen-activated protein
- *m*CPBA: *meta-*Chloroperoxybenzoic acid
- MOM: Methoxymethyl (CH₃OCH₂-)
- mRNA: Messenger ribonucleic acid
- M.S.: Mass spectrum
- NaHMDS: Sodium hexamethyldisilazide
- NMR: Nuclear magnetic resonance
- NMM: *N*-Methylmorpholine
- NMO: *N*-Methylmorpholine-*N*-oxide
- NOE(SY): Nuclear overhauser effect
- PCC: Pyridinium chlorochromate
- PDC: Pyridinium dichromate
- PG: Protecting Group
- PMB: *para*-Methoxybenzyl
- PNA: Peptide nucleic acid
- Piv: Pivaloyl
- PS-: Polymer supported
- PS-TBD: (1,5,7)-Triaza-bicyclo[4.4.0]dodeca-5-ene-7-methyl polystyrene
- Pyr or Py: Pyridine
- *rac*: Racemic
- RAL: Resorcylic acid lactone
- RCM: Ring-closing metathesis
- RedAl: Sodium bis(methoxyethoxy) aluminum hydride
- R_{f}: Retention factor
- RNA: Ribonucleic acid
- RT: Room temperature
- SAE: Sharpless asymmetric epoxidation
- SAR: Structure-activity relationship
- SEM: 2=Trimethylsilylethoxymethoxy
- TBAF: Tetra-*n*-butylammonium fluoride
- TBAI: Tetra-*n*-butylammonium iodide
- TBDPS: *t*-Butyldiphenylsilyl
- TBHP: *t*-Butylhydroperoxide
- TBS: *t*-Butyidimethylsilyl
- Teoc: 2-(Trimethylsilyl)ethoxycarbonyl
- Tf: Triflate (CF₃SO₃)
- TFA: Trifluoroacetic acid
- TFAA: Trifluoroacetic acetic anhydride
- THF: Tetrahydrofuran
- THP: Tetrahydropyran
- TLC: Thin layer chromatography
- TMS: Trimethylsilyl
- Ts: Tosyl (p-CH₃C₆H₄SO₂)
- *p*-TSOH: *para*-Toluenesulfonic acid

### Examples

### EXAMPLE 1: Synthesis of para-mono-phosphoamidate compounds 10a and 10b:

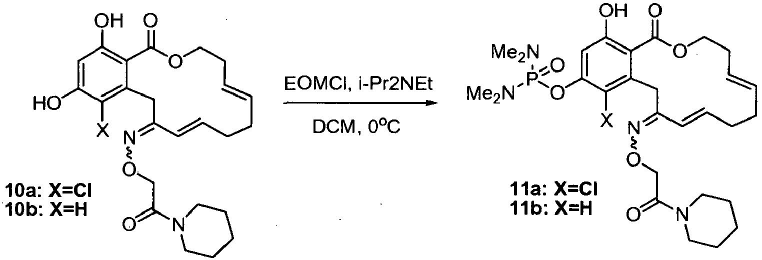

To a solution of the corresponding phenol macrocyclic compound **10a** or **10b** (1.0 equiv) in CH₂Cl₂ were added DBU (0.9 equiv), bis(dimethylamino)phosphoryl chloride (1.0 equiv) and DMAP (cat.). The reaction mixture was stirred at room temperature overnight. Then organic phase was washed with sat. NH₄Cl aq. and brine and then dried over MgSO₄. The desired mono phosphate **11a** and **11b** was purified by column chromatography (EtOAc to 5% MeOH in EtOAc) and isolated in a 50-60% yield. 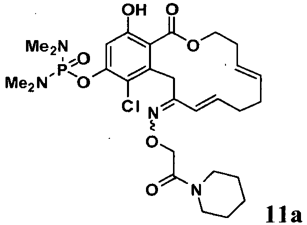 ¹H NMR (CDCl₃, 400 MHz) δ 11.53 (s, 1H), 11.16 (s, 1H), 7.11 (s, 1H), 7.06 (s, 1H), 6.51 *(d, J* = 16.1 Hz, 1H), 5.97 (dt, *J* = 15.6, 7.5 Hz, 1H), 5.42-5.35 (m, 1H), 5.32-5.28 (m, 2H), 5.13 *(d, J* = 15.6 Hz, 1H), 5.09-5.05 (m, 2H), 4.78 (s, 2H), 4.76 (s, 2H), 4.37-4.33 (m, 4H), 4.12 (s, 2H), 4.10 (s, 2H), 3.53-3.52 (m, 4H), 3.42-3.41 (m, 4H), 2.70 (s. 6H), 2.70 (s, 6H), 2.68 (s, 6H), 2.67 (s, 6H), 2.57-2.54 (m, 4H), 2.40-2.38 (m, 2H), 2.30-2.29 (m, 2H), 2.21-2.17 (m, 4H), 1.60-1.57 (m, 2H), 1.56-1.52 (m, 8H), 1.44-1.40 (m, 2H); HRMS (MALDI-TOF) m/z 633.2263 ([M + Na⁺]; C₂₈H₄₀ClN₄O₇PNa requires 633.2221). 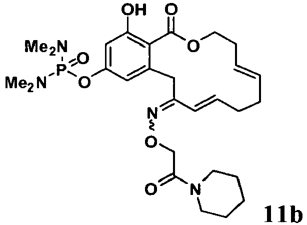 ¹H NMR (CDCl₃, 400 MHz) *δ* 11.22 (s, 1H), 11.20 (s, 1H), 6.81 (d, *J* = 2.1 Hz, 1 H), 6.79 (d, *J* = 1.7 Hz, 1H), 6.66 (d, *J* = 16.1 Hz, 1H), 6.65 (d, *J* = 2.1 Hz, 1H), 6.57 (d, *J* = 1.7 Hz, 1H), 6.16 (dt, *J =* 16.1, 7.0 Hz, 1H), 6.07 (dt, *J* = 16.1, 6.4 Hz, 1H), 5.80 (d, *J* = 16.1 Hz, 1H), 5.40-5.34 (m, 2H), 5.32-5.29 (m, 2H), 4.82 (s, 2H), 4.76 (s, 2H), 4.53-4.51 (m, 4H), 4.29 (s, 2H), 4.00 (s, 2H), 3.56 (bs, 4H), 3.40 (bs, 4H), 2.73-2.72 (m, 2H), 2.70 (s, 12H), 2.67 (s, 12H), 2.49-2.46 (m, 2H), 2.14 (bs, 4H), 2.11-2.05 (m, 4H), 1.65-1.64 (m, 4H), 1.58-1.56 (m, 8H); HRMS (MALDI-TOF) m/z 599.2652 ([M + Na⁺]; C₂₈H₄₁N₄O₇PNa requires 599.2611).

### EXAMPLE 2: Synthesis of ortho-mono-phosphoamidate compound 13:

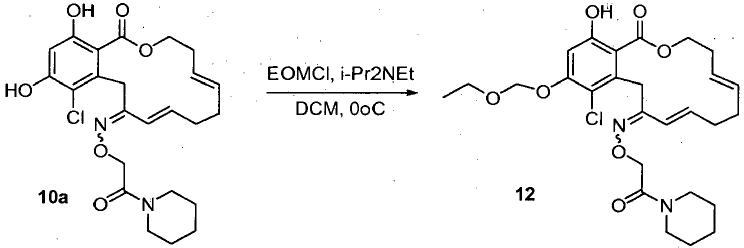

To a solution of bis-phenol **10a** (300 mg, 0.63 mmol, 1.0 equiv) in CH₂Cl₂ (5 mL) at 0 °C under nitrogen atmosphere, were added *i*-Pr₂NEt (104 µL, 0.63 mmol, 1.0 equiv) and EOMCl (58 µL, 0.63 mmol, 1.0 equiv). The reaction was slowly warmed up to room temperature, and kept stirring overnight. The reaction mixture was then washed with sat. NH₄Claq (15 mL) and extracted with CH₂Cl₂ (20 mL x 2); then the organic layers were combined and washed with brine (20 mL) and dried over anhydrous Na₂SO₄ After evaporation, the residue was purified by column chromatography (the ratio of eluent of petroleum ether : ethyl acetate, 3:2) to give rise to 165 mg of the desired monoprotected compound 12 (49%). This compound may also be obtained from the selective deprotection of the EOM group ortho to the carbonyl by treatment with TFA in MeOH:THF at room temperature.

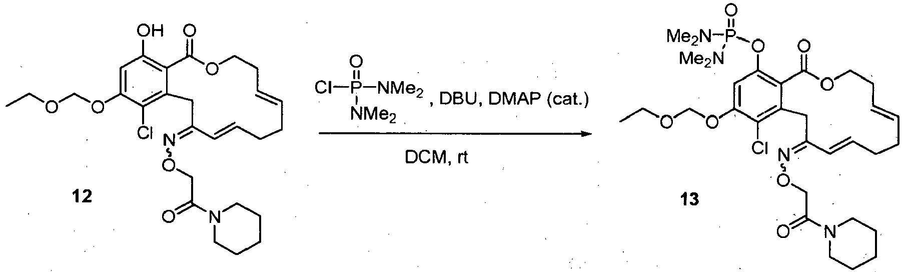

To the solution of the mono-EOM-protected macrocycle **12** (165 mg, 0.31 mmol, 1.0 equiv) in CH₂Cl₂ (5 mL) at 0°C under nitrogen atmosphere, were added DBU (93 µL, 0.62 mmol, 2.0 equiv), bis (dimethylamino)phosphoryl chloride (67 µL, 0.46 mmol, 1.5 equiv) and DMAP (3.8 mg, 0.03 mmol, 0.10 equiv). The reaction was slowly warmed up to room temperature, and kept stirring overnight. The reaction mixture was washed with sat. NH₄Claq (15 mL) and extracted with CH₂Cl₂ (15 mL x 2), the combined organic layers were then washed with brine (20 mL) and dried over anhydrous Na₂SO4. After evaporation, the residue underwent column chromatography (the ratio of eluent of petroleum ether : ethyl acetate, 1:1, then EA, EA:MeOH=10:1) to give 114 mg of the desired monoprotected compound 13 (55%).

### EXAMPLE 3: Synthesis of bis-phosphoamidate compound 14:

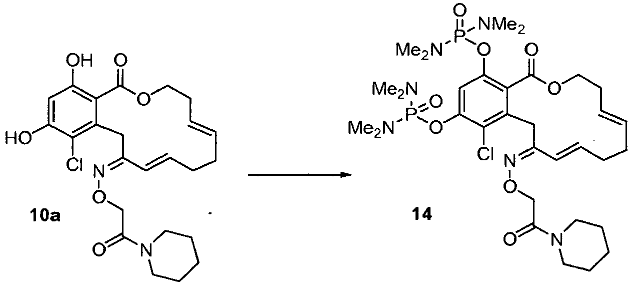

To a solution of the corresponding bis phenol macrocyclic compound **10a** (1.0 equiv) in CH₂Cl₂ were added DBU (2.0 equiv), bis(dimethylamino)phosphoryl chloride (3.0 equiv) and DMAP (cat.). The reaction mixture was stirred at room temperature overnight. The mixture was extracted with brine and dried over MgSO₄. The desired bis phosphate **14** was purified by column chromatography (EtOAc to 5% MeOH in EtOAc). 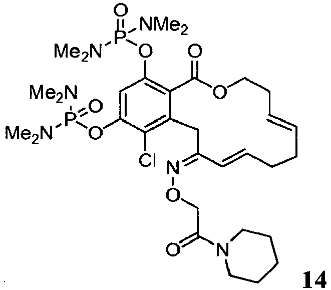 ¹H NMR (CDCl₃, 400 MHz) *δ* 6.47(d, *J* = 16.4 Hz, 1H), 6.45 (d, *J* = 1.5 Hz, 1H), 6.43 (s, 1H), 6.04 (dt, *J* = 16.4, 6.4 Hz, 1H), 5.93 (dt, *J* = 15.8, 7.0 Hz, 1H), 5.37 (d, *J* = 15.8 Hz, 1H), 5.29-5.08 (m, 4H), 4.75 (s, 2H), 4.66 (s, 2H), 4.18 (t, *J* = 5.6 Hz, 2H), 4.14 (t, *J* = 4.9 Hz, 2H), 3.89 (s, 2H), 3.78 (s, 2H), 3.53-3.50 (m, 4H), 3.44-3.40 (m, 4H), 2.73 (s, 6H), 2.72 (s, 6H), 2.71 (s, 6H), 2.69 (s, 6H), 2.67 (s, 6H), 2.66 (s, 6H), 2.65 (s, 6H), 2.63 (s, 6H), 2.34-2.27 (m, 4H), 2.07-1.99 (m, 4H), 1.60-1.57 (m, 4H), 1.53-1.50 (m, 12H); HRMS (MALDI-TOF) m/z 767.2814 ([M + Na⁺]; C₃₂H₅₁ClN₆O₈P₂Na requires 767.2830).

### EXAMPLE 4: The synthesis of phosphate compounds 1a, 1b, and 15:

Phosphate compounds **1a, 1b,** and 15 were prepared through hydrolysis of the corresponding phosphoamidate compounds **11a, 11b**, and **14** described as follows. A solution of the corresponding phosphoamidate macrocycle was treated with a 5% TFA solution in MeOH/H₂O 50/50 solution. The mixture was stirred at room temperature and followed by LCMS untill total consumption of the starting material. The solvent were removed in vacuo and the compound was purified by reverse phase (C18) chromatography. 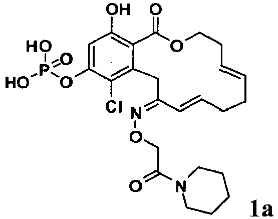 ¹H NMR (CD₃OD, 400 MHz) *δ* 7.38 (s, 1H), 7.35 (s, 1H), 6.54 (d, *J* = 16.6 Hz, 1H), 6.19 (dt, *J* = 16.1, 6.4 Hz, 1 H), 6.04 (dt, *J* = 15.6, 7.5 Hz, 1 H), 5.46-5.42 (m, 2H), 5.31-5.17 (m, 3H), 4.85 (s, 2H), 4.83 (s, 2H), 4.39-4.29 (m, 4H), 4.10 (s, 2H), 4.07 (s, 2H), 3.60-3.58 (m, 4H), 3.52-3.49 (m, 2H), 3.45-3.42 (m, 2H), 2.44-2.38 (m, 4H), 2.26-2.22 (m, 4H), 2.12-2.09 (m, 2H), 2.01-1.99 (m, 2H), 1.76-1.61 (m, 10 H), 1.55-1.54 (m, 2H), six OH signals are not visible; HRMS (MALDI-TOF) m/z 579.1217 ([M + Na⁺]; C₂₄H₃₀ClN₂O₉PNa requires 579.1276). 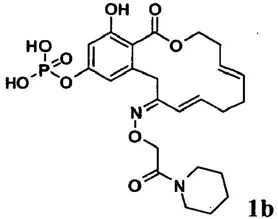 ¹H NMR (CD₃OD, 400 MHz) δ 6.81 (s, 1H), 6.80 (s, 1H), 6.73 (d, *J* = 16.0 Hz, 1H), 6.59 (s, 1H), 6.47 (s, 1H), 6.33-6.16 (m, 2H), 5.92 (d, *J* = 16.1Hz, 1H), 5.47-5.37 (m, 4H), 4.87 (s, 2H), 4.81 (s, 2H), 4.41 (t, *J* = 4.8 Hz, 4H), 3.92 (s, 2H), 3.64 (s, 2H), 3.62-3.58 (m, 4H), 3.55-3.52 (m, 4H), 2.50-2.48 (m, 4H), 2.16-2.12 (m, 8 H), 1.72-1.70 (m, 4H), 1.61-1.60 (m, 8H), six OH signals are not visible; HRMS (MALDI-TOF) m/z 545.1654 ([M + Na⁺]; C₂₄H₃₁N₂O₉PNa requires 545.1665). 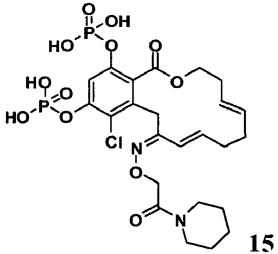 ¹H NMR (CD₃OD, 400 MHz) δ 7.76 (s, 1H); 7.72 (s, 1H); 6.54 (d, *J* = 16.0 Hz, 1H), 6.27 (dt, *J* = 16.1, 6.4 Hz, 1H), 6.05 (dot, *J* = 15.5, 7.5 Hz, 1H), 5.51-5.19 (m, 5H), 4.84 (s, 2H), 4.82 (s, 2H), 4.31 (t, *J* = 4.8 Hz, 2H), 4.25 (t, *J* = 4.3 Hz, 2H), 3.94 (s, 2H), 3.81 (s, 2H), 3.59-3.57 (m, 4H), 3.52-3.50 (m, 4H), 2.44-2.39 (m, 4H), 2.14-2.09 (m, 4H), 2.01-2.00 (m, 2H), 1.72-1.69 (m, 2H), 1.65-1.60 (m, 8H), 1.36-1.34 (m, 2H), 1.32-1.31 (m, 2H), eight OH signals are not visible; HRMS (MALDI-TOF) m/z 659.0947 ([M + Na⁺]; C₂₄H₃₁ClN₂O₁₂P₂Na requires 659.0939).

### EXAMPLE 5: The synthesis of phosphate compounds 1a, 1b, 29, 30, 31, and 32:

Additional phosphate compounds including Compounds **29, 30, 31,** and **32** were prepared according to the synthetic methods described in Schemes 4 and 5 below.

As shown in Scheme 4 below, in the course of scaling up the synthesis of the Compound **26** or **27** macrocycle via the previously developed synthetic route (Moulin, E.; Barluenga, S.; Totzke, F.; Winssinger, N., Chem. Eur. J. 2006, 12, (34), 8819-8834) based on a ring closing metathesis (RCM), we noted contamination with small amounts of the macrocycle containing the *cis* alkene produced RCM. We thus investigated an alternative strategy relying on a Mitsunobu macrolactonization with the geometry of the aforementioned alkene fixed. As shown in scheme 4, esters **16** (Barluenga, S.; Wang, C.; Fontaine, J. G.; Aouadi, K.; Beebe, K.; Tsutsumi, S.; Neckers, L.; Winssinger, N., Angew. Chem. Int. Ed. Engl. 2008, 47, (23), 4432-5) were deprotonated with LDA and reacted with Weinreb amide **24** to afford, after oxime formation, the macrocycles **18.** Global silyl deprotection followed by macrocyclization yielded Compounds **26** and **27** upon deprotection with sulfonic acid resin. While the compounds were obtained as an E/Z mixture of the oxime, the more active E isomer could be isolated by recrystalization to greater than 90% purity or as a single isomer by HPLC purification. The Weinreb amide **24** was obtained from the alkene **20** containing the required *trans* alkene via a sequence based on well established chemistry. Compounds **26** and **27** were thus obtained in 17% and 25% yield from from **16a** and **b** respectively. These syntheses could readily be scaled up to prepare over 10 g of the pochoxime A or B. 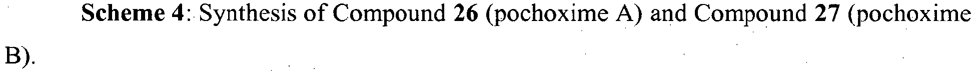 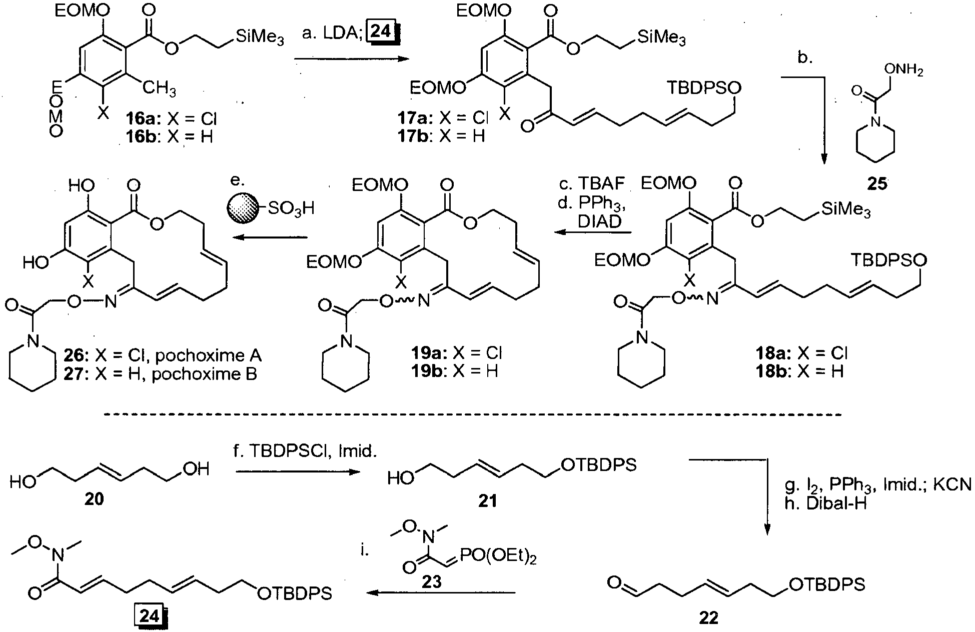 Reagents and conditions: (a) LDA (2.0 equiv), THF, -78 °C, 5 min; **24** (0.9 equiv), -78, 15 min, **17a** (65%) **17b** (54%); (b) **25** (2.5 equiv), pyridine, 40 °C, 24 h, **18a** (50%) **18b** (81%); (c) TBAF (2.5 equiv), 23 °C, 3 h; (d) Ph₃P (1.5 equiv), DIAD (1.5 equiv), toluene, 0 to 23 °C, 5 h, **19a** (60%) **19b** (61%); (e) PS-SO₃H (5.0 equiv, 3.0 mmol/g), MeOH, 23 °C, 2 h, **26** (90%) **27** (95%); (f) TBDPSCl (1.0 equiv), Imid (1.5 equiv), DMF, 23 °C, 5 h, 85%; (g) Imid (1.5 equiv), Ph₃P (1.1 equiv), iodine (1.1 equiv), 0 °C, 6 h; KCN (5.0 equiv), DMSO, 60 °C, 2 h, >90%; (h) Dibal-H (1.05 equiv), CH₂Cl₂, -78 °C, 30 min, 85%; (i) LiCl (1.2 equiv), **23** (1.2 equiv), DBU (1.0 equiv), CH₃CN, 23 °C, 45 min, 80% ; DBU = 1,8-Diazabicyclo[5.4.0]undec-7-ene; Dibal-H = diisobutylaluminium hydride Imid = imidazole PS = polystyrene, TBDPSCl = *tert*-Butyl diphenyl chlorosilane.

As shown in Scheme 5 below, owing to the greater acidity of the *para* phenol, Compunds **26, 27,** and **28** could be selectively derivatized at that position using 1.0 equivalent of bis(dimethylamino)phosphoryl chloride and 0.9 equivalent of DBU. Hydrolysis of the phosphoramide bond with wet TFA in dichloromethane followed by neutralization using anion exchange resin (Amberlite) afforded the pochoxime derivative **1a**, **1b, 29, 30, 31,** and **32** in excellent yield. To access the *para* phosphate, the *ortho* phenol was first selectively protected with an EOM group followed by the same methodology to introduce the phosphate thus yielding pochoxime A derivative **30.** The bis-phosphate derivative **31** was prepare in the same way as the *ortho*-substitued analogue but using an excess of bis(dimethylamino)phosphoryl chloride and two equivalents of DBU. Finaly, the phosphonooxymethyl derivative **32** could be accessed by the same methodology using di-*tert-*butyl chloromethylphosphonate (Ueda, Y.; Matiskella, J. D.; Golik, J.; Connolly, T. P.; Hudyma, T. W.; Venkatesh, S.; Dali, M.; Kang, S. H.; Barbour, N.; Tejwani, R.; Varia, S.; Knipe, J.; Zheng, M.; Mathew, M.; Mosure, K.; Clark, J.; Lamb, L.; Medin, I.; Gao, Q.; Huang, S.; Chen, C. P.; Bronson, J. J., Bioorg. Med. Chem. Lett. 2003, 13, (21), 3669-72) rather than bis(dimethylamino)phosphoryl chloride. In all cases, the acidic hydrolysis of the phosphoramide or *tert-*butyl deprotection resulted in an isomerization of the oxime (ca. 1:1 E:Z). 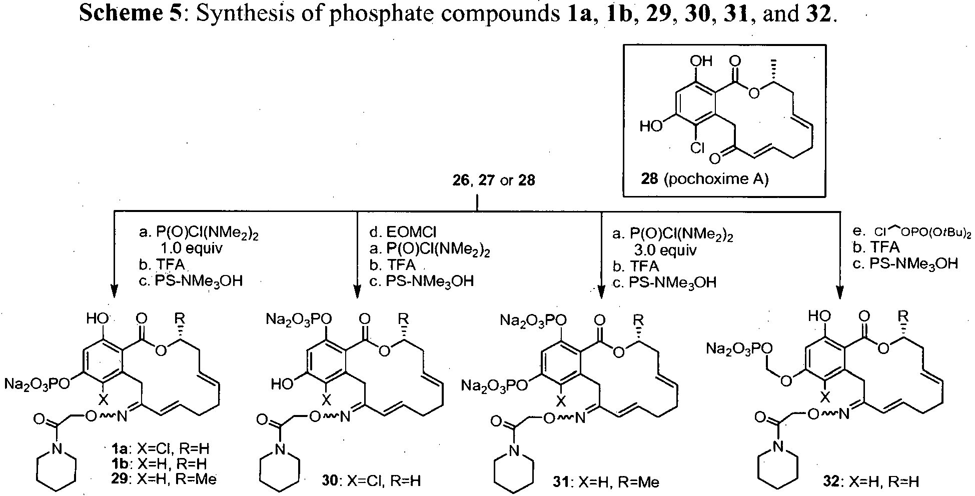 Reagents and conditions: (a) P(O)Cl(NMe₂)₂ (1-3.0 equiv), DBU (0.9 to 2.0 equiv), DMAP (0.01 equiv), CH₂Cl₂, 23 °C, 12 h, 50-60%; (b) TFA (5%), CH₃CN/H₂O 50/50; 23 °C, quantitative ; (c) Amberlite IRA68, H₂O/CH₃CN 1/1, quantitative (d) EOMCl (1.0 equiv), *i*Pr₂EtN (1.0 equiv), CH₂Cl₂, 0 to 23 °C, 12 h, 49%; (e) di-*tert*-butyl chloromethylphosphonate (1.0 equiv), Bu₄NI (1.0 equiv), CH₂Cl₂, 23 °C, 12 h, (68%); DBU = 1,8-Diazabicyclo[5.4.0]undec-7-ene; DMAP = 4-Dimethyl aminopyridine; EOMCl = ethoxymethyl chloride; TFA = trifluoroacetic acid.

### Bioassays

### General procedures for testing solubility and stability of phosphate prodrugs in vitro:

Crystalline phosphate drugs **1a** and **1b** were tested first in several different organic-and aqueous-based media, including water, PBS, ETOH, DMA, CMC, and PG. The phosphate prodrugs were found to be very soluble in water and PBS compared to the parent drugs.

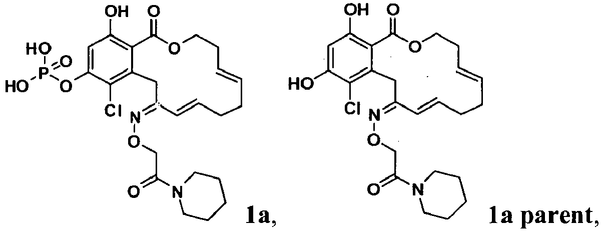

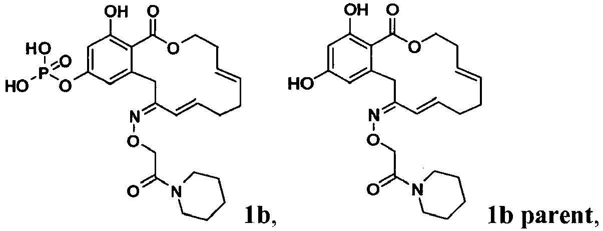

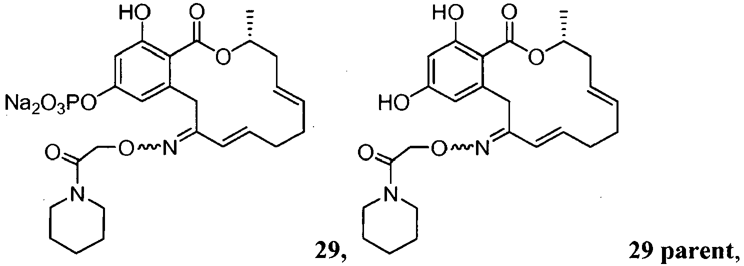

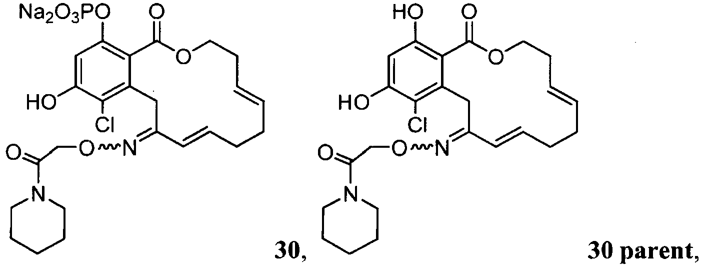

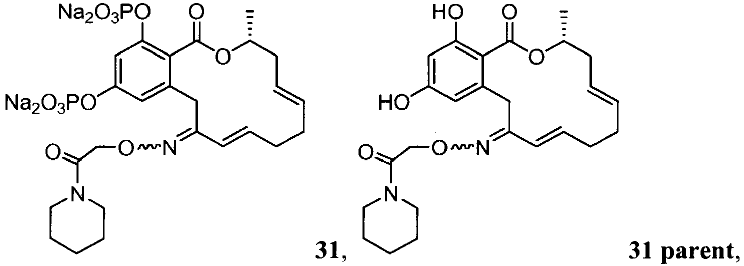

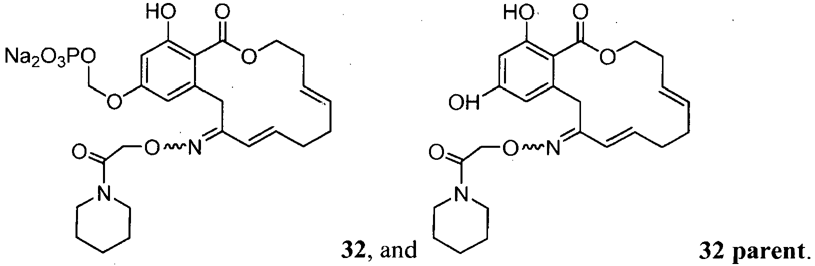

The maximum observed solubilities with small lots of crystalline drug in 4-mL glass clear tubes were:
1: at least 82.5 mg/mL in 50% DMA
2: at least 85 mg/mL in water
3: 87.5 mg/mL in 0.5% CMC / 0.1% Tween-20 / 0.45% NaCl for 1a
4: 55 mg/mL in PBS for 1a
5: 155 mg/mL in 10% ETOH / 20% PG / 70% water for 1 a
6: 50 mg/mL in 10% ETOH / 20% PG / 70% water for 1a

### Inhibition of proliferation of a panel of cancer cell lines by the parent compounds and the prodrugs

Cell proliferation assays were performed in various oncology cell lines, including breast cancer (BT474 and MDA-MB468), leukemia (K562 CML and MV4;11 AML), colon (HCT116), prostate cancer (PC-3), Tarceva- and Iressa-resistant non-small cell lung cancer (NSCLC, HCC829 and H1975), gastric cancer (N87) as well as glioblastoma (A172) cell lines. An appropriate number of cells (to reach ~70% confluence in 4 days) was plated in 96-well plates and cultured in the presence of vehicle, or increasing concentrations of 1 a parent, other active analogs **(1b** parent and **29** parent), or 17AAG for 4 days (0.001-5 □M). Cell viability at day 4 was measured by the amount of ATP present in viable cells using ATPlite assay kit according to manufacturer's protocol (Perkin Elmer, Boston, MA). IC₅₀ was calculated by fitting the data using XLfit software. Compounds **1a, 1b,** and **29** parent drugs exhibited very similar and potent anti-proliferative activity across a broad range of cancer cell lines. Interestingly, the pochoximes maintained anti-proliferative activity against MDA-MB468 which was significantly less sensitive to 17-AAG.

**Table 1 Growth inhibition and HSP90α affinity of the parent compounds Growth inhibition (IC₅₀) and HSP90 affinity of pochoxime derivative (nM)**

| Cell line | 1a parent | 1b parent | 29 parent | 17AAG |
|---|---|---|---|---|
| BT474 (breast) | 7 | 2 | 6 | 5 |
| MDA231 (breast) | 7 | 2 | 6 | NM |
| MDA-MB468 (breast) | 9 | 2 | 6 | 780 |
| N87 (gastric) | 4 | 1 | 2 | 1 |
| K562 (leukemia) | 6 | 4 | 7 | 48 |
| MV4;11 (leukemia) | 3 | 2 | 3 | 11 |
| HCT116 (colon) | 9 | 11 | NM | NM |
| HCC827 (lung) | 31 | NM | NM | 56 |
| H1975 (lung) | 25 | NM | NM | 35 |
| A172(glioblastoma) | 42 | NM | NM | NM |
| *HSP90*□ *affinity* | *21* | *15* | *18* | *32* |

To determine if a phosphate prodrug can be converted to its active parent in cells, we evaluated the potency of **1b,** a phosphate prodrug of **1b** parent, in blocking proliferation of BT474, MDA-MB468, and N87 cell lines. As shown in Table 2, **1b** displayed quite potent cellular activity although it is generally weaker than the **1b** parent (Table 1). Since the prodrug **1b** does not bind Hsp90 *in vitro,* the cellular activity observed must be due to the conversion of the prodrug to the parent compound by phosphatases present in these cells.

**Table 2 Growth inhibition and HSP90α affinity of 1b**

| Growth inhibition (IC₅₀) and HSP90 affinity of 1b (nM) | |
|---|---|
| Cell line | 1b |
| BT474(breast) | 14 |
| MDA-MB468 (breast) | 37 |
| N87 (gastric) | 19 |
| *HSP90*□ *affinity* | *> 10 000* |

### Procedures and results for in vitro conversion of prodrugs using tissue homogenates, plasma, and a mimic of gastric fluid

Since the two OH groups on the phenol ring of the parent drug are required for biological activity, the phosphate prodrugs may not be active unless hydrolysis of the prodrug occur rapidly *in vivo.* Hydrolysis of prodrug *in vivo* was estimated by incubating phosphate prodrugs *in vitro* with mouse small intestine and liver homogenates, mouse plasma, and artificial gastric fluid. Plasma was freshly collected from nude mice. Liver and intestine homogenates were prepared by adding 3 volume of saline to freshly collected mouse tissues and homogenized using glass homogenizer. Gastric fluid was prepared by adding 80 mL of 1M hydrochloric acid to 800 mL of water and then supplementing with 2.0 g of sodium chloride and 3.2 g of pepsin powder. The final volume of the artificial gastric fluid was adjusted with water to 1000 mL (pH = 1.1). Appropriate volume of prodrug stock solutions was spiked to the above vehicles to yield the final concentration at 5 µg/mL for Copmound **1a** and 80 µg/mL for Compound **1b**. 100 µL samples were collected at 0, 2, 5, 15, 30 and 60 minutes. 5 volumes methanol containing internal standard was added immediately. For samples from gastric fluid incubation, 1 volume of 0.01 M Sodium hydroxide was added before injection into LC/MS/MS. All samples were analyzed by LC/MS/MS to quantitate the concentration of **1a**, **1b** and the corresponding parent drugs. The concentration of the parent drugs of **1a** and **1b** was quantitated using standard curve prepared using each vehicle. The disintegration of **1a** and **1b** and the formation of their corresponding parents were shown in **Figures 1A** and **1B**. The results indicate that **1a** and **1b** decrease and the respective parent drugs form quickly in liver and intestine homogenates. The parent drugs accumulate to about 20% which remain stable for up to 60 minutes. Both **1a** and **1b** are stable in plasma (about 90% intact at 60 minutes) and slightly less stable in artificial gastric fluid (about 70% intact at 60 minutes).

We then evaluate other prodrug conversion *in vitro* using tissue homogenates and a mimic of gastric fluid (pepsin solution at pH 1.1). As shown in Table 3, marginal conversion occurred in the gastric fluid and plasma, while significant conversion was afforded by both the liver and intestine homogenates. Interestingly, **29** and **31** were more efficiently converted than **1a**, **1b**, **30,** and **32** with the best results for the bis-phosphate **31** reaching 43.51.6% conversion with intestine homogenate in 60 minutes. This data suggest that pro-pochoxime **31** would be the most suitable prodrug candidate.

**Table 3 Conversion (%) of phosphate prodrug to parent drug in tissue homogenates, plasma, and a mimic of gastric fluid.**

| | **1a** | **1b** | **29** | **30** | **31** | **32** |
|---|---|---|---|---|---|---|
| Liver | 18.2 | 23.2 | 29.4 | 10.5 | 30.5 | 17.9 |
| Intestine | 18.9 | 26.6 | 41.6 | 11.6 | 43.5 | 18.7 |
| Plasma | 3.4 | 2.2 | 1.4 | 0 | 0 | 4.0 |
| Gastric fluid | 0.4 | 0.4 | 0 | 0 | 0 | 0.3 |

### Procedure to evaluate in vitro partition between plasma and cells in mouse whole blood:

The whole blood was withdrawn from mice via cardio-puncture and placed in EDTA anticoagulant tubes. The test articles were spiked to whole blood at final concentration of 5µg/mL (at least 2 mL) and incubated at 37°C. 200 µL whole blood was taken at 0 (immediate after incubation), 5 min, 15 min and 30 min and then was centrifuged to separate plasma and cellular elements. Plasma was transferred to clean tubes. 50 µL of plasma and cellular elements were processed by addition of 3 volumes (150 µL) of ice-cold methanol. After centrifugation, the supernatants were subjected to LC-MS/MS analysis of test articles in both plasma and cellular elements. The concentration in plasma and cellular elements at different timepoints was listed in Table 4.

**Table 4.Test articles concentration in plasma and cellular elements**

| | 1a (ng/mL) | | 1b (ng/mL) | |
|---|---|---|---|---|
| Time(min) | Plasma | Cellular elements | Plasma | Cellular elements |
| 0.00 | 7988:91 | 1893.93 | 7119.50 | 1999.60 |
| 5.00 | 7654.94 | 1632.69 | 7833.27 | 1364.01 |
| 15.00 | 8123.27 | 1655.20 | 7672.71 | 1070.97 |
| 30.00 | 9680.38 | 1956.15 | 7409.47 | 1237.82 |

The concentration of **1a** and **1b** in plasma is larger than the nominated concentration in whole blood (5 µg/mL), which indicated that the partition of the test articles was mainly in plasma.

### Procedure to evaluate pharmacokinetics and bioavailability of prodrug in mice:

The *in vitro* studies allow us to estimate the hydrolysis of prodrug to the parent drug. However, in order to test whether the prodrug approach indeed improve the oral bioavailability, we can test the prodrugs *in vivo.* The purpose of the study is: (i) to define plasma pharmacokinetic (PK) characteristics of water soluble prodrugs in mice following oral administration, and (ii) to determine bioavailability of the prodrugs. Phosphate prodrugs are dissolved in appropriate vehicles to yield clear solutions with final concentration of 25 mg/ml for oral administration (pH ~ 7). Adult Balb/c mice (5-6 weeks of age, body weight: 17 g to 20 g) are dosed with 4, 20, 100, 500 mg/kg of prodrugs and three mice per groups are needed. Blood samples are collected from all mice 24 hours prior to administration - to establish a baseline level - and at approximately 0.5, 1, 2, 4, 8, 12 and 24 hours following *p.o.* (oral) administration of prodrug. Blood samples are centrifuged at 10,000 rpm for 3 to 5 minutes and separated immediately. Compounds in the plasma are extracted immediately with 3 volumes of cold methanol. The concentrations of the parent and the prodrug in plasma are determined. The concentrations in plasma below the limit of quantitation (LOQ = 2.5 ng/mL) are designated as zero. Pharmacokinetic parameters are calculated by model-independent methods. Area under the drug concentration curve (AUC) and area under the first moment of the drug concentration curve (AUMC) are calculated by means of the trapezoidal rule and extrapolated to infinity. Elimination half-life at b phase (t_{1/2}b) is calculated, when possible, from the slope of the terminal phase of the log plasma concentration-time points by linear regression. Oral bioavailability are calculated as F(%)=(Doseᵢᵥ×AUCₒᵣₐₗ(0→∞))/ (Doseₒᵣₐₗ×AUCᵢᵥ (0→∞))*100%. Preliminary investigations in mice showed that high concentration of the **31** parent in the liver was achieved via intravenous or oral administration of its prodrug **31** (Cmax: 3 838 ng/g by IV (4 mg/kg) and 12 469 ng/g by oral (30mg/kg)) although the concentration of the **31** parent in plasma was lower (Cmax: 197.2 ng/mL by IV (4 mg/kg) and 329.2 ng/mL by oral (30mg/kg). In addition, the oral bioavailability was calculated to be ∼ 12.3%.

## Claims

1. A compound having the structure of formula II: 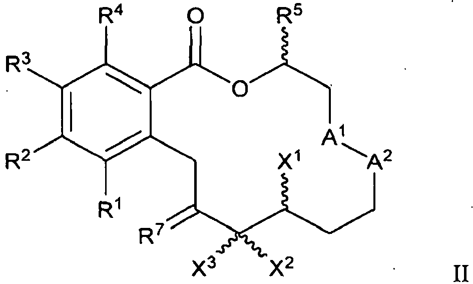 wherein,
R¹, R², R³, and R⁴ are each independently hydrogen, halogen, nitro, cyano, alkyl, alkenyl, alkynyl, arylalkyl, aryl, heteroalkyl, alkylheteroaryl, heterocyclyl, heteroaryl, OR, NR₂, SR, S(O)R, S(O)₂R, -SO₂N(R)₂, -N(R)SO₂R, -N(CO)R, -N(CO)NR₂, -N(CO)OR, -O(CO)R, -(CO)R, -(CO)OR, -(CO)NR₂, -O(CO)OR, -O(CO)NR₂, or a structural formula selected from the group consisting of 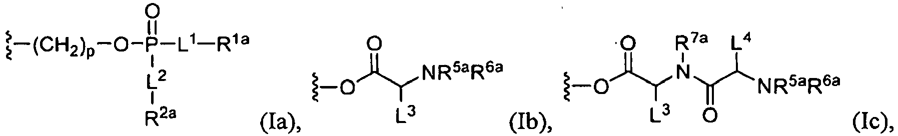 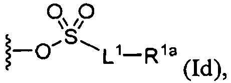 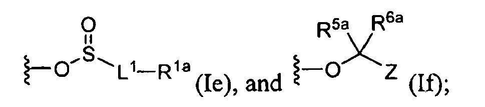 provided that at least one of R¹, R², R³, and R⁴ have a structural formula selected from the group consisting of (Ia), (Ib), (Ic), (Id), (Ie), and (If), wherein each R can be the same or different;
L¹ and L² are each independently a covalent bond, -O-, or NR^{3a}-;
p is 0, 1, or 2;
R^{1a} and R^{2a} are each independently hydrogen, alkyl, heteroalkyl, heteroaryl, heterocyclyl, alkenyl, alkynyl, arylalkyl, heteroarylalkyl, heterocyclylalkyl, -alkylene-C(O)-O-R^{4a}, or -alkylene-O-C(O)-O-R^{4a}; and
R^{3a} and R^{4a} are each independently hydrogen, alkyl, heteroalkyl, cyclylalkyl, heterocyclyl, aryl, heteroaryl, alkenyl, alkynyl, arylalkyl, heterocyclylalkyl, or heteroarylalkyl;
L³ and L⁴ are each independently hydrogen, halogen, nitro, cyano, alkyl, alkenyl, alkynyl, arylalkyl, aryl, heteroalkyl, heterocyclyl, heteroaryl, heterocyclylalkyl, heteroarylalkyl, OR, NR₂, or SR; wherein each R can be the same or different; R^{5a}, R^{6a}, and R^{7a} are each independently hydrogen, alkyl, alkenyl, alkynyl, alkylaryl, arylalkyl, aryl, heteroalkyl, alkylheteroaryl, heterocyclyl, or heteroaryl;
R⁵ is hydrogen, halogen, nitro, cyano, alkyl, alkenyl, alkynyl, arylalkyl, aryl, heteroalkyl, alkylheteroaryl, heterocyclyl, heteroaryl, OR, NR₂, SR, S(O)R, S(O)₂R,-SO₂N(R)₂, -N(R)SO₂R -N(CO)R, -N(CO)NR₂, -N(CO)OR, -O(CO)R, -(CO)R, - (CO)OR, -(CO)NR₂, -O(CO)OR, or -O(CO)NR₂; wherein each R can be the same or different;
Z has a structural formula selected from the group consisting of (Ia), (Ib), (Ic), (Id), and (Ie);
A¹ and A² together are -CH₂-CH₂-, -CH=CH-, -CH(OH)-CH(OH)-, -CH(OH)-CH(halogen)-, -CH(halogen)-CH(OH)-, 1,2-cyclopropadiyl, or 1,2-oxirane;
X¹ is hydrogen, halogen, OR, NR₂, NH-OR, SR, S(O)R, S(O)₂R, -N-O-(CH₂)ₙ-CO₂-R; or X¹ together with X² or X³ represents a covalent bond; wherein each R can be the same or different;
X² and X³ are both hydrogen, or one of X² and X³ is hydrogen and the other together. with X¹ represents a covalent bond;
R⁷ is =O, = S, =N-OR, =N-O-(CH₂)ₙCOOR, =N-O-(CH₂)ₙCONR₂, =N-NR₂, =N-N-SOR or =N-N-SO₂R; and
n is 1, 2 or 3.

2. The compound of claim 1, wherein R¹ is H, halogen or heterocyclyl.

3. The compound of claim 1, wherein R⁵ is hydrogen, alkyl, aryl, heteroaryl or arylalkyl.

4. The compound of claim 1, wherein A¹ and A² together are -CH=CH-, -CH(OH)-CH(OH)-, -CH(OH)-CH(halogen)-, or -CH(halogen)-CH(OH) or 1,2-oxirane.

5. The compound of claim 1, wherein:
R¹ is H, Cl or heterocyclyl;
R⁵ is hydrogen, alkyl, aryl or arylalkyl;
A¹ and A² together are -CH=CH- or -C(OH)-C(OH)-;
X¹ together with X² represent a bond; and
R⁷ is =O, =S, =N-OR, =N-O-(CH₂)ₙCOOR, =N-O-(CH₂)ₙCONR₂, =N-NR₂, =N-N-SOR, =N-N-SO₂R

6. The compound of claim 5, wherein:
R¹ is H or Cl;
R⁵ is hydrogen, methyl, propyl, isopropyl or phenyl; and
R⁷ is =N-OR, =N-O-(CH₂)ₙCOOR, or =N-O-(CH₂)ₙCONR₂.

7. The compound of claim 6, wherein R¹ is Cl and R⁵ is hydrogen.

8. The compound of claim 6, wherein n is 1.

9. The compound of claim 6, wherein R⁵ is hydrogen and R⁷ is =N-O-(CH₂)ₙCOOR, or =N-O-(CH₂)ₙCONR₂.

10. The compound of any of claims 1 to 9, wherein one of R² and R⁴ has structural formula (Ia), at least one of L¹ and L² is -O-, and p is 0 or 1.

11. The compound of claim 10, wherein L¹ and L² are both -O-.

12. The compound of any of claims 1 to 9, wherein one of R² and R⁴ has structural formula (I_{b}) or (If), and R^{5a} and R^{6a} are independently hydrogen or C₁-C₄ alkyl.

13. The compound of any of claims 1 to 9, wherein one of R² and R⁴ has structural formula (I_{c}), and R^{5a}, R^{6a}, and R^{7a} are independently hydrogen or C₁-C₄ alkyl.

14. The compound of any of claims 1 to 9, wherein one of R² and R⁴ has structural formula (Id) or (Ie), and L¹ is -O-.

15. The compound of claim 1 having a structural formula selected from the group consisting of 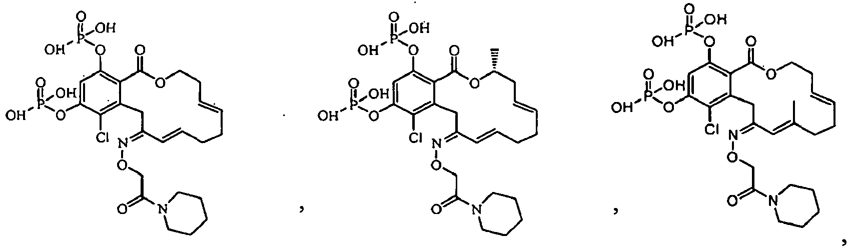 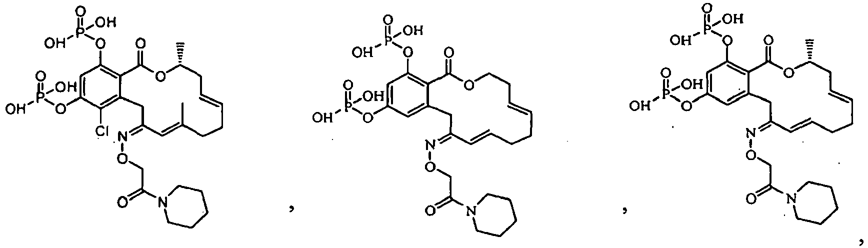 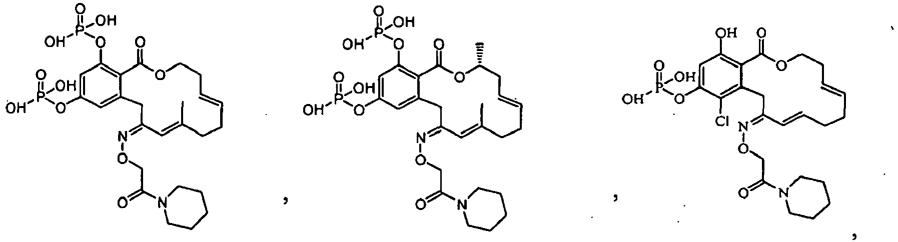 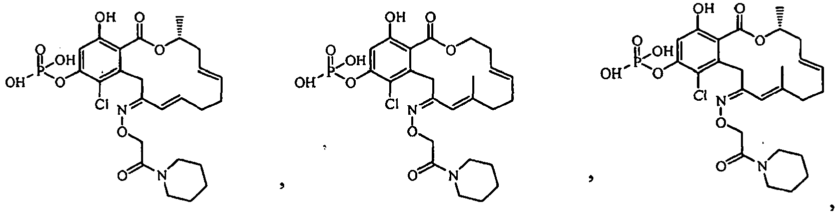 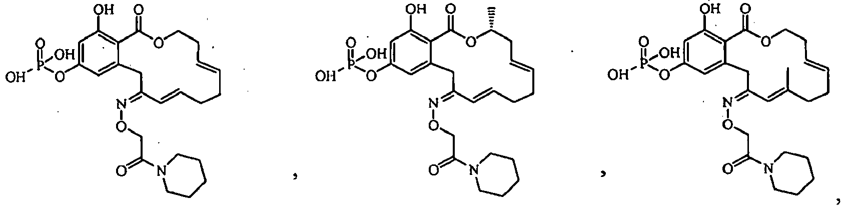 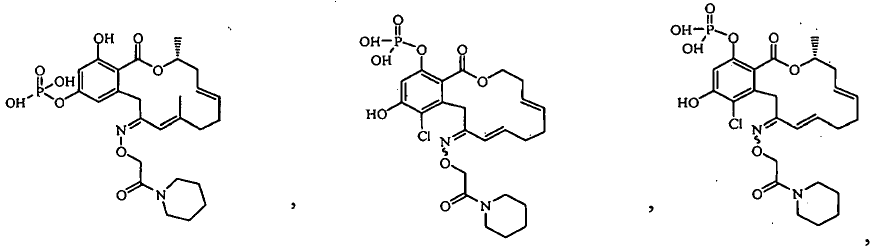 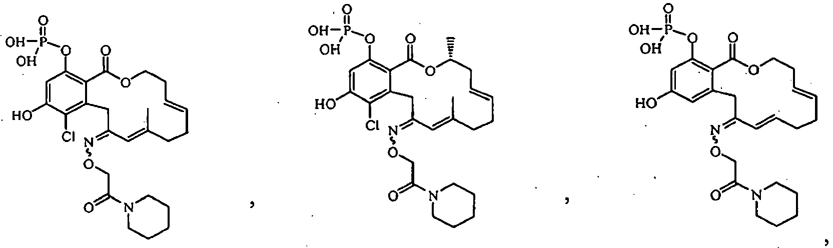 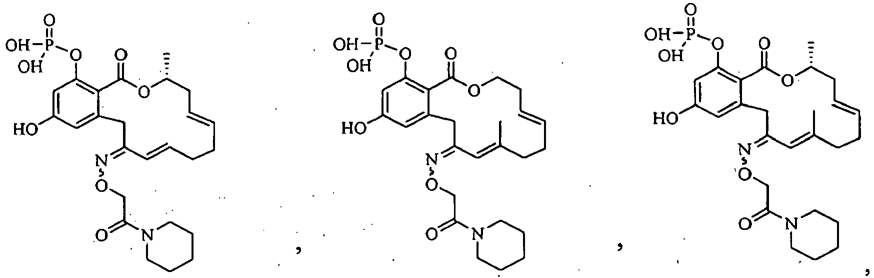 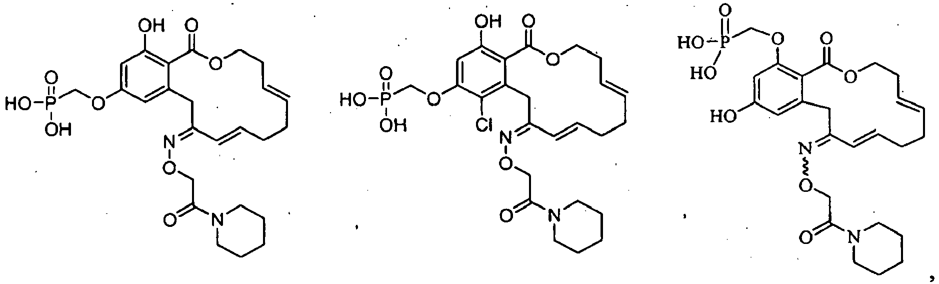 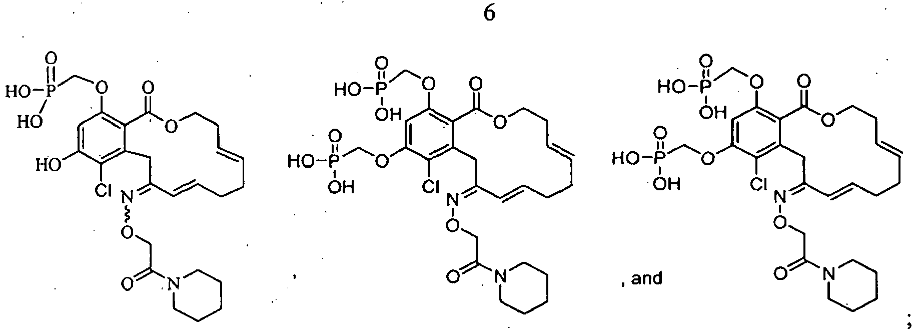 or a pharmaceutically acceptable salt or solvate thereof.

16. A pharmaceutical composition comprising a compound of any of claims 1 to 15, or a pharmaceutically acceptable salt or solvate thereof; and optionally a pharmaceutically acceptable carrier.

17. The compound of any of claims 1 to 15, or a pharmaceutically acceptable salt or solvate thereof, for use in the treatment, prevention, or amelioration of a HSP90-mediated disorder, wherein the HSP90-mediated disorder is selected from the group consisting of an autoimmune disease, an inflammatory disease, a neurological or neurodegenerative disease, cancer, a cardiovascular disease, allergy, asthma, a hormone-related disease, and tumors or symptoms resulting from neurofibromatosis.

18. Use of at least one compound of any of claims 1 to 15, or a pharmaceutically acceptable salt or solvate thereof, for the manufacture of a medicament for the treatment, prevention, or amelioration of tumors or symptoms resulting from neurofibromatosis in a subject suffering neurofibromatosis type 2 (NF2) or a condition associated with the loss of NF2 function or neurofibromatosis type 1 (NF1) or a condition associated with the loss of NF1 function.

## Patentansprüche

1. Eine Verbindung mit der Struktur der Formel II: 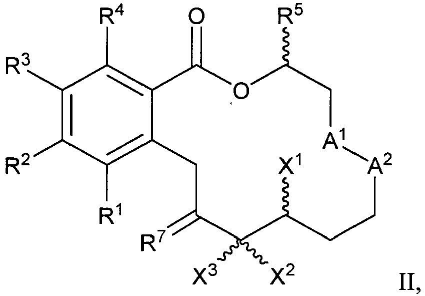 wobei
R¹, R², R³ und R⁴ jeweils unabhängig Wasserstoff, Halogen, Nitro, Cyano, Alkyl, Alkenyl, Alkinyl, Arylalkyl, Aryl, Heteroalkyl, Alkylheteroaryl, Heterocyclyl, Heteroaryl, OR, NR₂, SR, S(O)R, S(O)₂R, -SO₂N(R)₂, -N(R)SO₂R, -N(CO)R, -N(CO)NR₂, -N(CO)OR, -O(CO)R, -(CO)R, -(CO)OR, -(CO)NR₂, -O(CO)OR, -O(CO)NR₂ oder eine Strukturformel, ausgewählt aus der Gruppe, bestehend aus 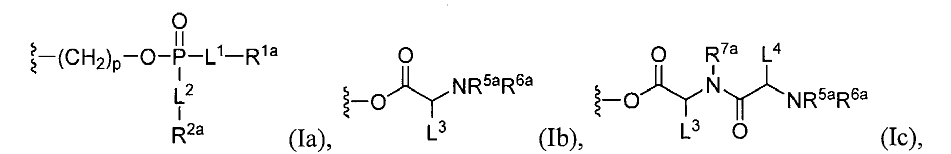 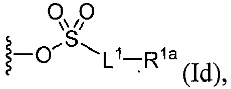 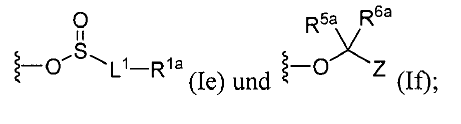 sind, mit der Maßgabe, dass mindestens einer der Reste R¹, R², R³ und R⁴ eine Strukturformel aufweist, ausgewählt aus der Gruppe bestehend aus (Ia), (Ib), (Ic), (Id), (Ie) und (If), wobei jeder Rest R gleich oder verschieden sein kann;
L¹ und L² jeweils unabhängig eine kovalente Bindung, -O- oder -NR^{3a}- sind;
p gleich 0, 1 oder 2 ist;
R^{1a} und R^{2a} jeweils unabhängig Wasserstoff, Alkyl, Heteroalkyl, Heteroaryl, Heterocyclyl, Alkenyl, Alkinyl, Arylalkyl, Heteroarylalkyl, Heterocyclylalkyl, -Alkylen-C(O)-O-R^{4a} oder -Alkylen-O-C(O)-O-R^{4a} sind; und
R^{3a} und R^{4a} jeweils unabhängig Wasserstoff, Alkyl, Heteroalkyl, Cyclylalkyl, Heterocyclyl, Aryl, Heteroaryl, Alkenyl, Alkinyl, Arylalkyl, Heterocyclylalkyl oder Heteroarylalkyl sind;
L³ und L⁴ jeweils unabhängig Wasserstoff, Halogen, Nitro, Cyano, Alkyl, Alkenyl, Alkinyl, Arylalkyl, Aryl, Heteroalkyl, Heterocyclyl, Heteroaryl, Heterocyclylalkyl, Heteroarylalkyl, OR, NR₂ oder SR sind; wobei jeder Rest R gleich oder verschieden sein kann;
R^{5a}, R^{6a} und R^{7a} jeweils unabhängig Wasserstoff, Alkyl, Alkenyl, Alkinyl, Alkylaryl, Arylalkyl, Aryl, Heteroalkyl, Alkylheteroaryl, Heterocyclyl oder Heteroaryl sind; R⁵ Wasserstoff, Halogen, Nitro, Cyano, Alkyl, Alkenyl, Alkinyl, Arylalkyl, Aryl, Heteroalkyl, Alkylheteroaryl, Heterocyclyl, Heteroaryl, OR, NR₂, SR, S(O)R, S(O)₂R, -SO₂N(R)₂, -N(R)SO₂R, -N(CO)R, -N(CO)NR₂, -N(CO)OR, -O(CO)R, -(CO)R, -(CO)OR, -(CO)NR₂, -O(CO)OR oder -O(CO)NR₂ ist, wobei jedes R gleich oder verschieden sein kann;
Z eine Strukturformel aufweist, ausgewählt aus der Gruppe bestehend aus (Ia), (Ib), (Ic), (Id) und (Ie);
A¹ und A² zusammen -CH₂-CH₂-, -CH=CH-, -CH(OH)-CH(OH)-, -CH(OH)-CH(Halogen)-, -CH(Halogen)-CH(OH)-, 1,2-Cyclopropadiyl oder 1,2-Oxiran sind;
X¹ Wasserstoff, Halogen, OR, NR₂, NH-OR, SR, S(O)R, S(O)₂R, -N-O-(CH₂)ₙ-CO₂-R ist; oder X¹ stellt zusammen mit X² oder X³ eine kovalente Bindung dar; wobei jeder Rest R gleich oder verschieden sein kann;
X² und X³ beide Wasserstoff sind oder ein Rest von X² und X³ ist Wasserstoff und der andere stellt zusammen mit X¹ eine kovalente Bindung dar;
R⁷ ist =O, =S, =N-OR, =N-O-(CH₂)ₙCOOR, =N-O-(CH₂)ₙCONR₂, =N-NR₂, =N-N-SOR oder =N-N-SO₂R und
n gleich 1, 2 oder 3 ist.

2. Verbindung nach Anspruch 1, wobei R¹ H, Halogen oder Heterocyclyl ist.

3. Verbindung nach Anspruch 1, wobei R⁵ Wasserstoff, Alkyl, Aryl, Heteroaryl oder Arylalkyl ist.

4. Verbindung nach Anspruch 1, wobei A¹ und A² zusammen -CH=CH-, -CH(OH)-CH(OH)-, -CH(OH)-CH(Halogen)- oder -CH(Halogen)-CH(OH) oder 1,2-Oxiran sind.

5. Verbindung nach Anspruch 1, wobei:
R¹ gleich H, Cl oder Heterocyclyl ist;
R⁵ Wasserstoff, Alkyl, Aryl oder Arylalkyl ist;
A¹ und A² zusammen -CH=CH- oder -C(OH)-C(OH)- sind;
X¹ zusammen mit X² eine Bindung darstellt; und
R⁷ =O, =S, =N-OR, =N-O-(CH₂)ₙCOOR, =N-O-(CH₂)ₙCONR₂, =N-NR₂, =N-N-SOR, =N-N-SO₂R ist.

6. Verbindung nach Anspruch 5, wobei:
R¹ H oder Cl ist;
R⁵ Wasserstoff, Methyl, Propyl, Isopropyl oder Phenyl ist; und
R⁷ =N-OR, =N-O-(CH₂)ₙCOOR oder =N-O-(CH₂)ₙCONR₂ ist.

7. Verbindung nach Anspruch 6, wobei R¹ Cl ist und R⁵ Wasserstoff ist.

8. Verbindung nach Anspruch 6, wobei n gleich 1 ist.

9. Verbindung nach Anspruch 6, wobei R⁵ Wasserstoff ist und R⁷ =N-O-(CH₂)ₙ-COOR oder =N-O-(CH₂)ₙ-CONR₂ ist.

10. Verbindung nach einem der Ansprüche 1 bis 9, wobei ein Rest von R² und R⁴ die Strukturformel (Ia) aufweist, mindestens eines von L¹ und L² -O- ist und p gleich 0 oder 1 ist.

11. Verbindung nach Anspruch 10, wobei L¹ und L² beide -O- sind.

12. Verbindung nach einem der Ansprüche 1 bis 9, wobei einer der Reste R² und R⁴ die Strukturformel (Ib) oder (If) aufweist und R^{5a} und R^{6a} unabhängig Wasserstoff oder C₁-C₄-Alkyl sind.

13. Verbindung nach einem der Ansprüche 1 bis 9, wobei einer der Reste R² und R⁴ die Strukturformel (Ic) aufweist und R^{5a}, R^{6a} und R^{7a} unabhängig Wasserstoff oder C₁-C₄-Alkyl sind.

14. Verbindung nach einem der Ansprüche 1 bis 9, wobei einer der Reste R² und R⁴ die Strukturformel (Id) oder (Ie) aufweist und L¹ -O- ist.

15. Verbindung nach Anspruch 1 mit einer Strukturformel, ausgewählt aus der Gruppe, bestehend aus 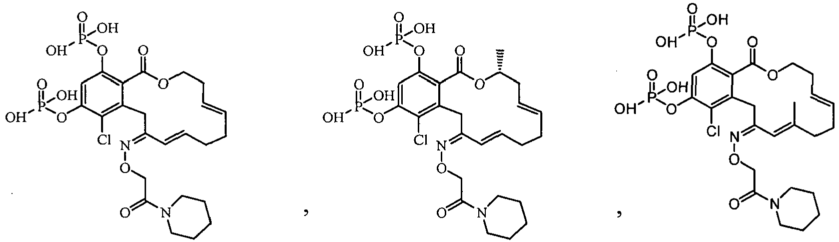 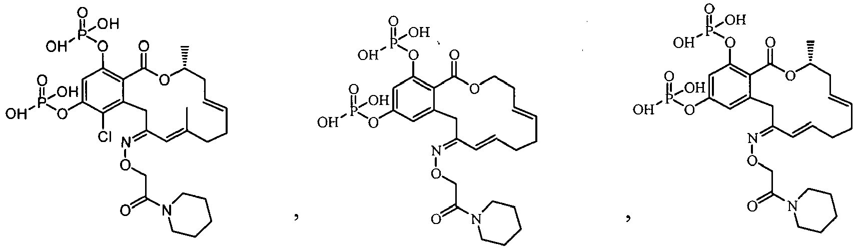 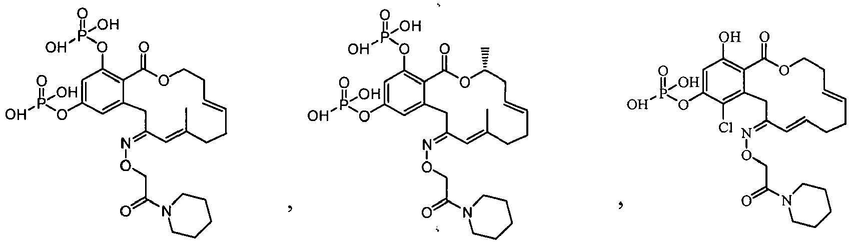 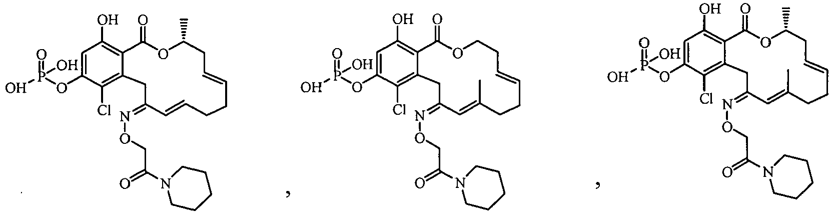 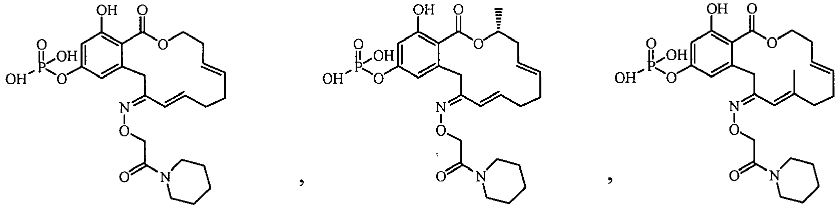 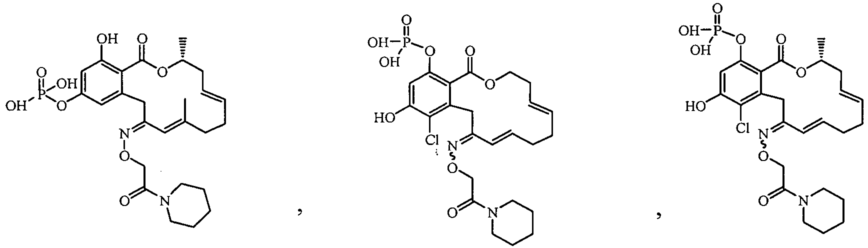 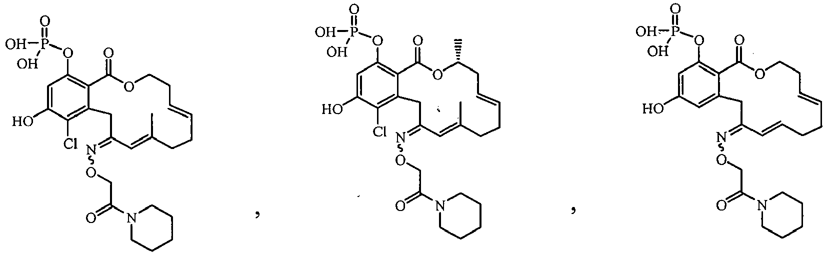 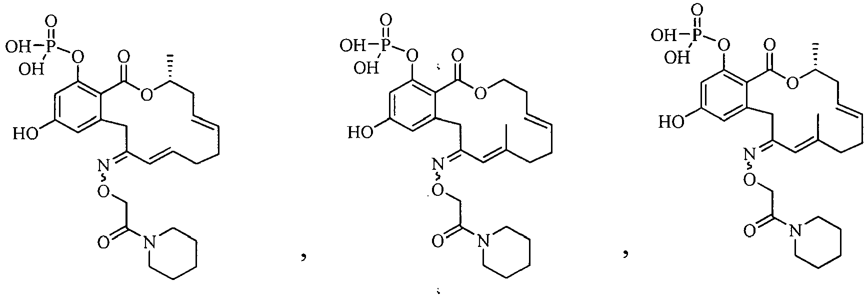 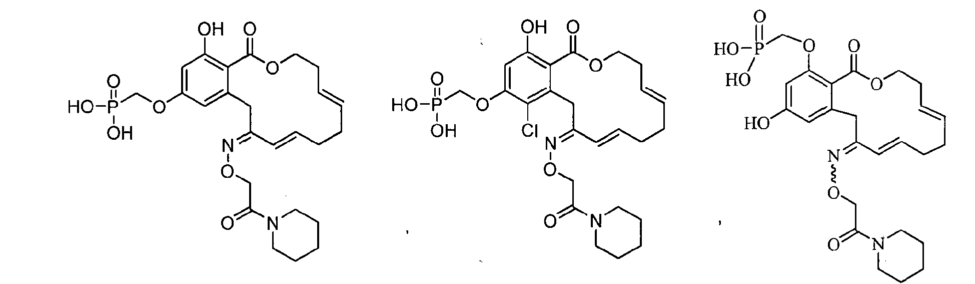 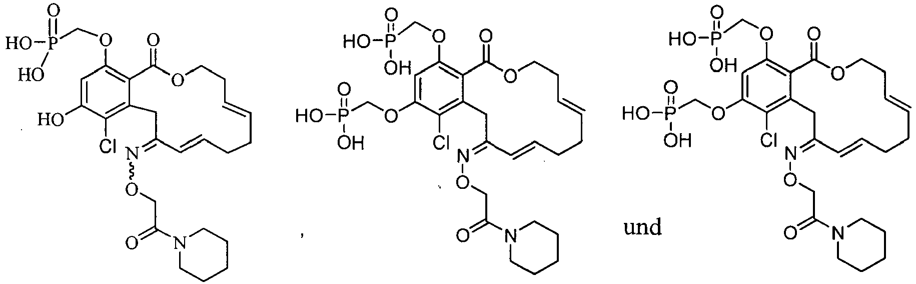 oder ein pharmazeutisch verträgliches Salz oder Solvat davon.

16. Arzneimittel, umfassend eine Verbindung nach einem der Ansprüche 1 bis 15 oder ein pharmazeutisch verträgliches Salz oder Solvat davon; und gegebenenfalls einen pharmazeutisch verträglichen Träger.

17. Verbindung nach einem der Ansprüche 1 bis 15 oder ein pharmazeutisch verträgliches Salz oder Solvat davon zur Verwendung bei der Behandlung, Vorbeugung oder Linderung einer durch HSP90-bewirkten Störung, wobei die durch HSP90-bewirkte Störung ausgewählt ist aus der Gruppe bestehend aus einer Autoimmunerkrankung, einer entzündlichen Erkrankung, einer neurologischen oder neurodegenerativen Erkrankung, Krebs, einer Herz-Kreislauf-Erkrankung, Allergie, Asthma, einer hormonbedingten Erkrankung und Tumoren oder Symptomen, die durch Neurofibromatose entstehen.

18. Verwendung mindestens einer Verbindung nach einem der Ansprüche 1 bis 15 oder ein pharmazeutisch verträgliches Salz oder Solvat davon zur Herstellung eines Medikaments zur Behandlung, Vorbeugung oder Linderung von Tumoren oder Symptomen, durch Neurofibromatose bei einer Person entstehen, die unter Neurofibromatose Typ 2 (NF2) oder einem Zustand, der mit dem Verlust der NF2 Funktion in Zusammenhang steht, oder Neurofibromatose Typ 1 (NF1) oder einem Zustand, der mit dem Verlust der NF1 Funktion in Zusammenhang steht leidet.

## Revendications

1. Composé ayant la structure de formule II : 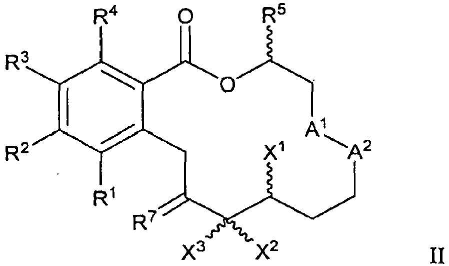 dans laquelle,
R¹, R², R³, et R⁴ représentent chacun indépendamment un atome d'hydrogène, d'halogène, un groupe nitro, cyano, alkyle, alcényle, alcynyle, arylalkyle, aryle, hétéroalkyle, alkylhétéroaryle, hétérocyclyle, hétéroaryle, OR, NR₂, SR, S(O)R, S(O)₂R, -SO₂N(R)₂, -N(R)SO₂R, -N(CO)R, -N(CO)NR₂, -N(CO)OR,-O(CO)R, -(CO)R, -(CO)OR, -(CO)NR₂, -O(CO)OR, -O(CO)NR₂, ou une formule structurale choisie dans le groupe constitué de 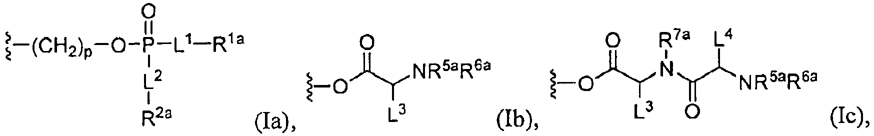 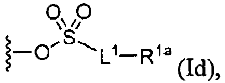 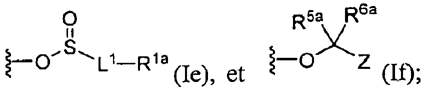 à condition qu'au moins l'un de R¹, R², R³, et R⁴ ait une formule structurale choisie dans le groupe constitué de (Ia), (Ib), (Ic), (Id), (Ie), et (If), où chaque R peut être identique ou différent ;
L¹ et L² représentent chacun indépendamment une liaison covalente, -O-, ou -NR^{3a}- ;
p vaut 0, 1, ou 2 ;
R^{1a} et R^{2a} représentent chacun indépendamment un atome d'hydrogène, un groupe alkyle, hétéroalkyle, hétéroaryle, hétérocyclyle, alcényle, alcynyle, arylalkyle, hétéroarylalkyle, hétérocyclylalkyle, -alkylène-C(O)-O-R^{4a}, ou -alkylène-OC(O)-O-R^{4a} ; et
R^{3a} et R^{4a} représentent chacun indépendamment un atome d'hydrogène, un groupe alkyle, hétéroalkyle, cyclylalkyle, hétérocyclyle, aryle, hétéroaryle, alcényle, alcynyle, arylalkyle, hétérocyclylalkyle, ou hétéroarylalkyle ;
L³ et L⁴ représentent chacun indépendamment un atome d'hydrogène, d'halogène, un groupe nitro, cyano, alkyle, alcényle, alcynyle, arylalkyle, aryle, hétéroalkyle, hétérocyclyle, hétéroaryle, hétérocyclylalkyle, hétéroarylalkyle, OR, NR₂, ou SR ; où chaque R peut être identique ou différent ;
R^{5a}, R^{6a}, et R^{7a} représentent chacun indépendamment un atome d'hydrogène, un groupe alkyle, alcényle, alcynyle, alkylaryle, arylalkyle, aryle, hétéroalkyle, alkylhétéroaryle, hétérocyclyle, où hétéroaryle ;
R⁵ représente un atome d'hydrogène, d'halogène, un groupe nitro, cyano, alkyle, alcényle, alcynyle, arylalkyle, aryle, hétéroalkyle, alkylhétéroaryle, hétérocyclyle, hétéroaryle, OR, NR₂, SR, S(O)R, S(O)₂R, -SO₂N(R)₂, -N(R)SO₂R, -N(CO)R, -N(CO)NR₂, -N(CO)OR, -O(CO)R, -(CO)R, -(CO)OR, -(CO)NR₂, -O(CO)OR, ou -O(CO)NR₂ ; où chaque R peut être identique ou différent ;
Z a une formule structurale choisie dans le groupe constitué de (Ia), (Ib), (Ic), (Id), et (Ie) ;
A¹ et A² ensemble représentent un groupe -CH₂-CH₂-, -CH=CH-, -CH(OH)-CH(OH)-, -CH(OH)-CH(halogène)-, -CH(halogène)-CH(OH)-, 1,2-cyclopropadiyle, ou 1,2-oxirane ;
X¹ représente un atome d'hydrogène, d'halogène, OR, NR₂, NH-OR, SR, S(O)R, S(O)₂R, -N-O-(CH₂)ₙ-CO₂-R; ou X¹ conjointement avec X² ou X³ représente une liaison covalente ; où chaque R peut être identique ou différent ;
X² et X³ représentent tous les deux un atome d'hydrogène, ou l'un de X² et X³ représente un atome d'hydrogène et l'autre conjointement avec X¹ représente une liaison covalente ;
R⁷ représente =O, =S, =N-OR, =N-0-(CH₂)ₙCOOR, =N-O-(CH₂)ₙCONR₂, =N-NR₂, =N-N-SOR ou =N-N-SO₂R ; et
n vaut 1, 2 ou 3.

2. Composé selon la revendication 1, dans lequel R¹ représente H, un atome d'halogène ou un groupe hétérocyclyle.

3. Composé selon la revendication 1, dans lequel R⁵ représente un atome d'hydrogène, un groupe alkyle, aryle, hétéroaryle ou arylalkyle.

4. Composé selon la revendication 1, dans lequel A¹ et A² ensemble représentent un groupe -CH=CH-, -CH(OH)-CH(OH)-, -CH(OH)-CH(halogène)-, ou -CH(halogène)-CH(OH) ou 1,2-oxirane.

5. Composé selon la revendication 1, dans lequel :
R¹ représente H, Cl ou un groupe hétérocyclyle ;
R⁵ représente un atome d'hydrogène, un groupe alkyle, aryle ou arylalkyle ;
A¹ et A² ensemble représentent -CH=CH- ou -C(OH)-C(OH)- ;
X¹ conjointement avec X² représentent une liaison ; et
R⁷ représente =O, =S, =N-OR, =N-O-(CH₂)ₙCOOR, =N-O-(CH₂)ₙCONR₂, =N-NR₂, =N-N-SOR, =N-N-SO₂R.

6. Composé selon la revendication 5, dans lequel :
R¹ représente H ou Cl ;
R⁵ représente un atome d'hydrogène, un groupe méthyle, propyle, isopropyle ou phényle ; et
R⁷ représente =N-OR, =N-O-(CH₂)ₙCOOR, ou =N-O-(CH₂)ₙCONR₂.

7. Composé selon la revendication 6, dans lequel R¹ représente Cl et R⁵ représente un atome d'hydrogène.

8. Composé selon la revendication 6, dans lequel n vaut 1.

9. Composé selon la revendication 6, dans lequel R⁵ représente un atome d'hydrogène et R⁷ représente =N-O-(CH₂)ₙCOOR, ou =N-O-(CH₂)ₙCONR₂.

10. Composé selon l'une quelconque des revendications 1 à 9, dans lequel l'un de R² et R⁴ a la formule structurale (Ia), au moins l'un de L¹ et L² représente -O-, et p vaut 0 ou 1.

11. Composé selon la revendication 10, dans lequel L¹ et L² représentent tous les deux -O-.

12. Composé selon l'une quelconque des revendications 1 à 9, dans lequel l'un de R² et R⁴ a la formule structurale (Ib) ou (If), et R^{5a} et R^{6a} représentent indépendamment un atome d'hydrogène ou un groupe alkyle en C₁ à C₄.

13. Composé selon l'une quelconque des revendications 1 à 9, dans lequel l'un de R² et R⁴ a la formule structurale (Ic), et R^{5a}, R^{6a}, et R^{7a} représentent indépendamment un atome d'hydrogène ou un groupe alkyle en C₁ à C₄.

14. Composé selon l'une quelconque des revendications 1 à 9, dans lequel l'un de R² et R⁴ a la formule structurale (Id) ou (Ie), et L¹ représente -O-.

15. Composé selon la revendication 1 ayant une formule structurale choisie dans le groupe constitué de 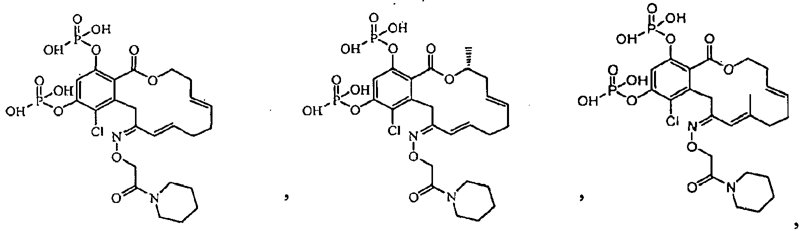 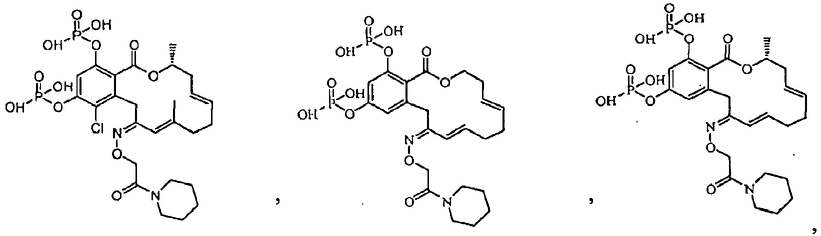 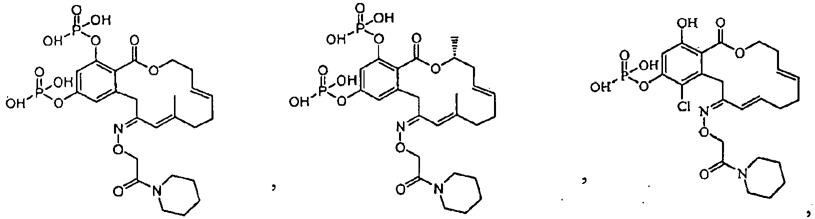 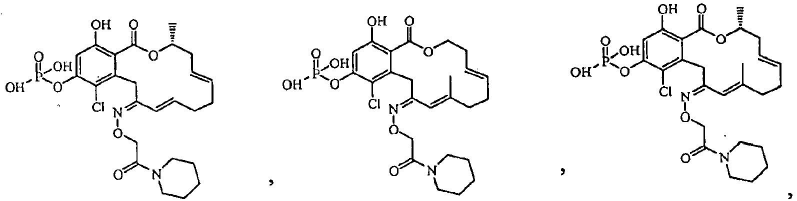 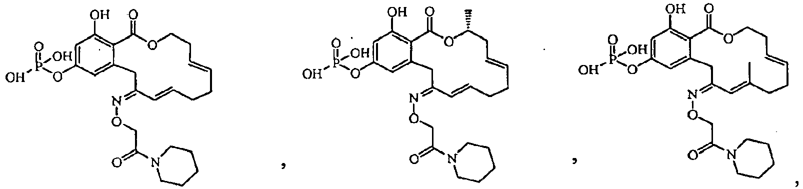 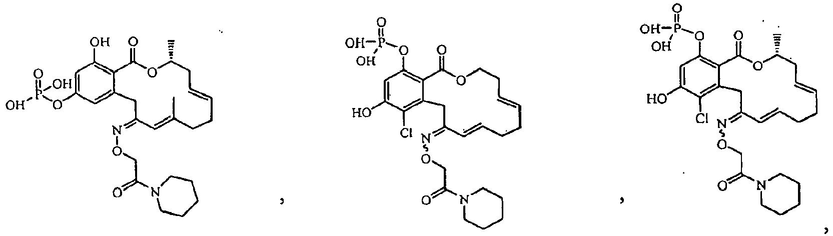 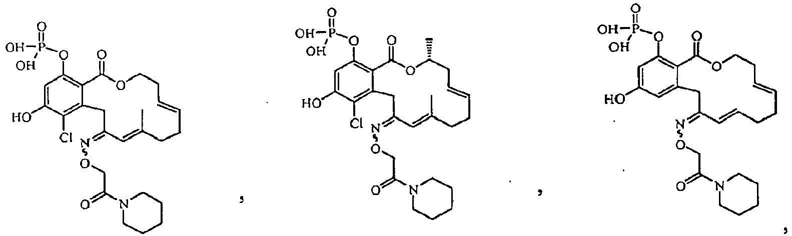 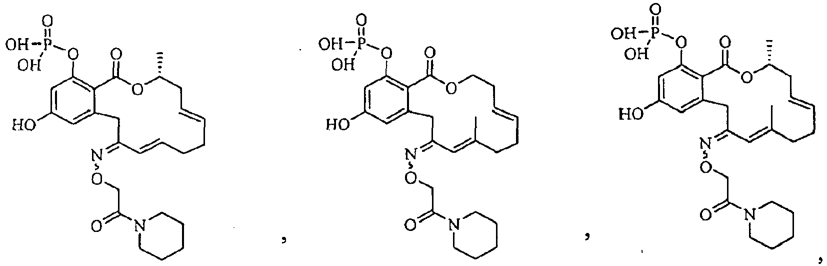 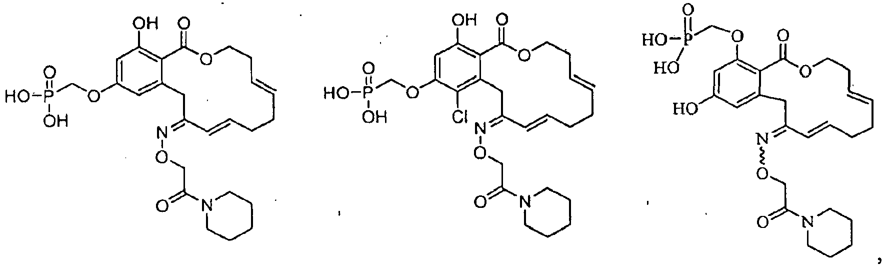 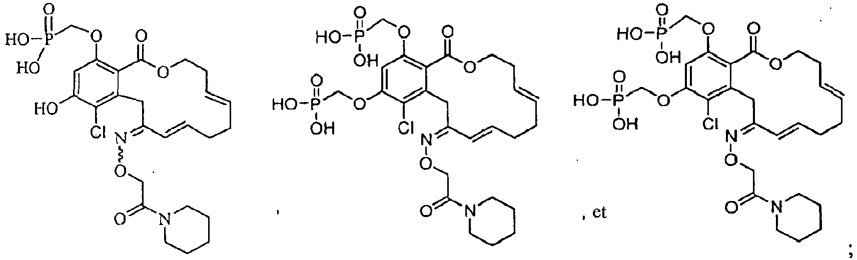 ou l'un de leurs sels pharmaceutiquement acceptables ou solvates.

16. Composition pharmaceutique comprenant un composé selon l'une quelconque des revendications 1 à 15, ou l'un de ses sels pharmaceutiquement acceptables ou solvates ; et éventuellement un support pharmaceutiquement acceptable.

17. Composé selon l'une quelconque des revendications 1 à 15, ou l'un de ses sels pharmaceutiquement acceptables ou solvates, pour une utilisation dans le traitement, la prévention, ou l'amélioration d'un trouble médié par la HSP90, où le trouble médié par la HSP90 est choisi dans le groupe constitué d'une maladie auto-immune, d'une maladie inflammatoire, d'une maladie neurologique ou neurodégénérative, du cancer, d'une maladie cardiovasculaire, de l'allergie, de l'asthme, d'une maladie associée à une hormone, et de tumeurs ou de symptômes résultant d'une neurofibromatose.

18. Utilisation d'au moins un composé selon l'une quelconque des revendications 1 à 15, ou de l'un de ses sels pharmaceutiquement acceptables ou solvates, pour la fabrication d'un médicament destiné au traitement, à la prévention, ou à l'amélioration de tumeurs ou de symptômes résultant d'une neurofibromatose chez un sujet souffrant de neurofibromatose de type 2 (NF2) ou d'une affection associée à la perte de la fonction de la NF2 ou de neurofibromatose de type 1 (NF1) ou d'une affection associée à la perte de la fonction de la NF1.
